# EUROPÄISCHE PATENTSCHRIFT

(11) **EP 1 220 943 B1**
(45) Veröffentlichungstag und Bekanntmachung des Hinweises auf die Patenterteilung: **14.07.2004**
(21) Anmeldenummer: 01913665.4
(22) Anmeldetag: 15.02.2001
(51) Int. Cl.: C12Q 1/26, G01N 33/573, C12N 5/06

(54) **Zelluläres Testsystem zur Untersuchung der metabolischen Aktivität aktiver Formen des Cytochrom P450 2D6**
Cellular test system for investgation of metabolic activity of active forms of cytochrome P450 2D6
Système pour l'investigation de l'activité metabolique des formes actives de cytochrome P450 2D6

(30) Priorität: 14.03.2000 DE 10012220
(43) Veröffentlichungstag der Anmeldung: 10.07.2002
(73) Patentinhaber: DÖHMER, Johannes, 82166 Gräfelfing (DE)
(72) Erfinder: DÖHMER, Johannes, 82166 Gräfelfing (DE); KREBSFÄNGER, Niels, 88400 Biberach a.d. Riss (DE); EICHELBAUM, Michel, 71711 Murr (DE); ZANGER, Ulrich, M., 70825 Korntal-Münchingen (DE)
(74) Vertreter: Grund, Martin, Dr.
(86) Internationale Anmeldenummer: PCT/DE2001/000597
(87) Internationale Veröffentlichungsnummer: WO 2001/068907

(56) Entgegenhaltungen:
- WO-A-93/08260
- US-A- 5 429 948
- DATABASE BIOSIS [Online] BIOSCIENCES INFORMATION SERVICE, PHILADELPHIA, PA, US; 1997 RAUSCHENBACH REIMUND ET AL: "Development of a V79 cell line expressing human cytochrome P450 2D6 and its application as a metabolic screening tool." Database accession no. PREV199799507660 XP002181678 in der Anmeldung erwähnt & ENVIRONMENTAL TOXICOLOGY AND PHARMACOLOGY, Bd. 3, Nr. 1, 1997, Seiten 31-39, ISSN: 1382-6689
- OSCARSON MIKAEL ET AL: "A combination of mutations in the CYP2D6*17 (CYP2D6Z) allele causes alterations in enzyme function." MOLECULAR PHARMACOLOGY, Bd. 52, Nr. 6, Dezember 1997 (1997-12), Seiten 1034-1040, XP001036782 ISSN: 0026-895X in der Anmeldung erwähnt
- WANG SU-LAN ET AL: "G169R mutation diminishes the metabolic activity of CYP2D6 in Chinese." DRUG METABOLISM AND DISPOSITION, Bd. 27, Nr. 3, März 1999 (1999-03), Seiten 385-388, XP001036785 ISSN: 0090-9556
- PRITCHARD MICHAEL P ET AL: "Functional co-expression of CYP2D6 and human NADPH-cytochrome P450 reductase in Escherichia coli." PHARMACOGENETICS, Bd. 8, Nr. 1, Februar 1998 (1998-02), Seiten 33-42, XP001036781 ISSN: 0960-314X in der Anmeldung erwähnt
- KEMPF ANDREAS C ET AL: "Truncated Human P450 2D6: Expression in Escherichia coli, Ni-2+-Chelate Affinity Purification, and Characterization of Solubility and Aggregation." ARCHIVES OF BIOCHEMISTRY AND BIOPHYSICS, Bd. 321, Nr. 2, 1995, Seiten 277-288, XP001039520 ISSN: 0003-9861 in der Anmeldung erwähnt
- ONDERWATER ROB C A ET AL: "The use of macroporous microcarriers for the large-scale growth of V79 cells genetically designed to express single human cytochrome P450 isoenzymes and for the characterization of the expressed cytochrome P450." PROTEIN EXPRESSION AND PURIFICATION, Bd. 8, Nr. 4, 1996, Seiten 439-446, XP002181677 ISSN: 1046-5928 in der Anmeldung erwähnt
- BAPIRO T.E. ET AL: 'The molecular and enzyme kinetic basis for the diminished activity of the cytochrome P450 2D6.17 (CYP2D6.17) variant' BIOCHEMICAL PHARMACOLOGY Bd. 64, 2002, Seiten 1387 - 1398
- YU A. ET AL: 'Expression, Purification, Biochemical Charaterization, and Comparative Function of Human Cytochrome P450 2D6.1,n 2D6.2, 2D6.10, and 2D6.17 Allelic Isoforms' THE JOURNAL OF PHARMACOLOGY AND EXPERIMENTAL THERAPEUTICS Bd. 303, Nr. 3, 2002, Seiten 1291 - 1300

## Beschreibung

Die Erfindung betrifft ein Testsystem, das humanes Cytochrom P450 2D6 exprimierende Zellinien enthält und die Verwendung dieses Testsystems zur Untersuchung pharmakologischer und toxikologischer Aspekte des hCYP2D6-Polymorphismus.

Der menschliche Körper nimmt täglich eine Vielzahl von Fremdstoffen auf: Schadstoffe aus der Umwelt, Inhaltsstoffe aus Nahrungs- und Genußmitteln sowie mitunter Arzneimittel. Alle Fremdstoffe müssen letztlich wieder ausgeschieden werden, um Schädigungen des Organismus zu vermeiden. Viele dieser Verbindungen sind allerdings schlecht wasserlöslich und deswegen nicht ohne weiteres ausscheidbar. In nahezu allen Lebewesen entwickelte sich daher evolutiv ein komplexes Enzymsystem, das Verbindungen in eine hydrophile, ausscheidbare Form umwandeln kann. Dieser Stoffwechselprozeß wird Fremdstoff-Metabolismus genannt und formal in zwei Phasen eingeteilt (Greim und Deml, 1996; Marquardt und Schäfer, 1997): In Phase I werden funktionelle Gruppen in die Verbindung eingeführt oder funktionelle Gruppen demaskiert, wobei Cytochrome P450 eine Schlüsselrolle spielen. In Phase II wird der funktionalisierte Metabolit mit gut wasserlöslichen Substanzen wie Sulfaten, Zuckern, Glutathion, Carbonsäuren oder Aminosäuren konjugiert. Die Verbindung ist nun ausreichend hydrophil und kann als nichtreaktives Endprodukt über Niere oder Darm ausgeschieden werden. Allerdings besteht die Gefahr, daß reaktive Metaboliten mit zelleigenen Strukturen wie DNA, RNA, Proteinen und Lipiden reagieren und so zytotoxische, kanzerogene oder mutagene Effekte auslösen. Dann kehrt sich die Entgiftung in eine Giftung um.

Arzneimittel werden über dasselbe Enzymsystem metabolisiert und entsorgt, was die pharmakologische Wirksamkeit in unterschiedlicher Weise beeinflussen kann: Entweder sind die Metaboliten pharmakologisch weniger wirksam als die Ausgangsverbindung oder gänzlich unwirksam, wie bei Barbituraten. In anderen Fällen sind sowohl Muttersubstanz als auch Metaboliten wirksam. Ein Beispiel hierfür ist das Hustenmittel Codein und sein Metabolit Morphin. In anderen Fällen ist erst der Metabolit wirksam, wie das Spaltprodukt von Cyclophosphamid in der Chemotherapie.

Wegen unterschiedlicher Metabolisierung kann die Wirksamkeit eines Arzneimittels individuell verschieden sein. Die Metabolisierung wird von Parametern wie Alter, Geschlecht, körperlicher Konstitution und Ernährung beeinflußt. Als weiterer entscheidender Faktor haben sich Unterschiede in den kodierenden Genen für Fremdstoff-metabolisierende Enzyme herausgestellt, z.B. Cytochrome P450. Daher werden Arzneimittel genetisch bedingt von einigen Individuen schneller, langsamer, anders oder überhaupt nicht verstoffwechselt. Variieren die Allelfrequenzen zwischen verschiedenen Populationen, führt dies zu inter-ethnischen Unterschieden in der Häufigkeitsverteilung der Phänotypen. Asiaten, Kaukasier und Schwarzafrikaner können daher sehr unterschiedlich auf ein und dasselbe Medikament reagieren. Diese pharmakologisch wichtigen inter-individuellen Unterschiede werden als pharmakogenetischer Polymorphismus bezeichnet (Meyer, 1991):
*"Ein pharmakogenetischer Polymorphismus ist eine monogene Eigenschaft, die durch das Vorhandensein innerhalb einer Population von mehr als einem Allel an demselben Locus und von mehr als einem Phänotyp hinsichtlich der Arzneimittelwirkung auf den Organismus verursacht ist. Die Häufigkeit des seltensten Alleles ist > 1 %."*

Die Häufigkeit des seltensten Allels wurde vereinbarungsgemäß auf > 1 % festgelegt, da nicht jeder Basen-Unterschied auch notwendigerweise phänotypisch oder für eine Bevölkerungsgruppe relevant ist. Es wird geschätzt, daß bis zu 20 % aller Medikamente einem pharmakogenetischen Polymorphismus unterliegen (Blech, 1999). In den USA leiden jährlich über 2 Millionen Patienten an unerwünschten Arzneimittelwirkungen, die in über 100.000 Fällen letal verlaufen. Damit gehören sie zu den sechs häufigsten Todesursachen (Lazarou *et al.,* 1998). Als Konsequenz werden "personalisierte Arzneimittel(dosierungen)" angestrebt, die exakt auf die individuelle Pharmakogenetik des Patienten abgestimmt sind. Dies wird beispielsweise bereits bei der Therapie leukämiekranker Kinder mit Azathioprin oder 6-Mercaptourin praktiziert, die andernfalls in 3 % der Fälle zu lebensbedrohlichen Nebenwirkungen führt.

Daher ist neben der Entwicklung effizienter Methoden zur Feststellung des individuellen pharmakogenetischen Profils, beispielsweise mittels DNA-Chiptechnologie, die detaillierte Kenntnis der Arzneimittel-metabolisierenden Enzyme und ihrer genetischen Polymorphismen für eine maximale Arzneimittel-Sicherheit unumgänglich.

Die wichtigste Gruppe Fremdstoff-metabolisierender Phase I-Enzyme sind die Cytochrome P450 (EC 1.14.14.1; unspezifische Monooxygenasen). Sie wurden erstmals 1958 als "zellfärbendes Pigment" der Leber unabhängig von Garfinkel (1958) und Klingenberg (1958) beschrieben. Der Name Cytochrom P450 leitet sich davon ab, daß Cytochrome im reduzierten Zustand und nach Begasung mit Kohlenmonoxid ein Differenzspektrum mit einem charakteristischen Absorptionsmaximum bei 450 nm zeigen. Anhand der Absorption ist mit dem molaren Extinktionskoeffizienten eine Quantifizierung möglich (Omura und Sato, 1964a; Omura und Sato, 1964b).

Die Cytochrom P450-Superfamilie umfaßt derzeit 481 Isoformen in 85 verschiedenen Euund 20 Prokaryontenspezies (Nelson *et al*., 1996). Die Einteilung der Cytochrom P450-Enzyme basiert *per conventionem* auf ihren Aminosäure-Sequenzhomologien. Innerhalb einer Familie sind sie zu mehr als 40 %, innerhalb einer Subfamilie mindestens zu 55 % identisch. Cytochrom P450-Gene werden in kursiver Schreibweise mit "*CYP*", cDNAs, mRNAs und Proteine mit "CYP" abgekürzt. Darauf folgt eine arabische Ziffer, welche die Familie bezeichnet, und ein lateinischer Großbuchstabe für die jeweilige Subfamilie. Einzelne Isoenzyme werden mit einer weiteren arabischen Ziffer chronologisch durchnummeriert. Dem gesamten Symbol wird ein lateinischer Kleinbuchstabe für die jeweilige Spezies vorangestellt. Damit ergibt sich beispielsweise für das humane Cytochrom P450 2D6 das Symbol *hCYP2D6* für das Gen bzw. hCYP2D6 für die cDNA, mRNA und das Protein. Alle Fremdstoffmetabolisierenden Cytochrome P450 sind über eine hydrophobe N-terminale Sequenz im endoplasmatischen Retikulum sowie der Kernmembran verankert und zytoplasmatisch orientiert (Monier *et al.,* 1988). Cytochrome P450 gehören zu den Häm-Thiolat-Enzymen und katalysieren die NADPH-abhängige Monooxygenierung ihrer Substrate zu den korrespondierenden Alkoholen oder Epoxiden sowie O- und N-Dealkylierungen. Sie bilden einen Multienzymkomplex mit der NADPH-abhängigen Cytochrom P450-Oxidoreduktase (CYPOR) und Cytochrom b₅, die den Elektronentransfer von NADPH auf das Cytochrom P450 katalysieren. Die Fähigkeit zur Komplexbildung ist unterschiedlich (Schenkman und Greim, 1993). Die Aktivität einiger Isoformen, beispielsweise von hCYP3A4, ist in besonderem Maße von der CYPOR und Cytochrom b₅ abhängig (Buters *et al.,* 1994). Bei hCYP3A4 wird durch Cytochrom b₅ zudem die Affinität zu bestimmten Substraten erhöht (Schenkman *et al.,* 1989).

Die Substratspezifität einzelner Cytochrome P450 ist in der Regel gering und häufig überlappend, so daß eine ausreichende Plastizität gewährleistet ist, um der enormen Vielfalt an zu metabolisierenden Fremdstoffen zu begegnen. Andererseits tragen die überlappenden Substratspezifitäten neben der organspezifischen Expression, der Polymorphie und der Induzierbarkeit vieler Isoenzyme erheblich zur Komplexität des Cytochrom P450-katalysierten Fremdstoff- bzw. Arzneimittel-Metabolismus bei. Die Komplexität kann weiter gesteigert werden, wenn bei gleichzeitiger Gabe mehrerer Arzneimittel durch Induktion oder Inhibition bestimmter Cytochrom P450-Isoformen der Arzneimittel-Metabolismus beeinflußt wird. Daher sind detaillierte Kenntnisse der Arzneimittel-metabolisierenden Cytochrom P450 auf genetischer, regulatorischer und enzymatischer Ebene unabdingbar, um Metabolismusbedingte unerwünschte Wirkungen zu vermeiden.

Cytochrom P450 2D6 (EC 1.14.14.1.; Debrisoquin-4-Hydroxylase) ist eine der molekularen Spezies von Cytochrom P450, die sich durch einen ausgeprägten Polymorphismus auszeichnet. CYP2D6 ist beim Menschen das einzige funktionale Isoenzym der 2D-Subfamilie. Es war das erste Cytochrom P450-Enzym, für das ein genetischer Polymorphismus beschrieben wurde. Der hCYP2D6-Polymorphismus wurde Ende der 70er Jahre unabhängig voneinander an dem Antihypertensivum Debrisoquin, (Evans *et al*., 1980; Mahgoub *et al*., 1977) und dem Antiarrhythmikum Spartein (Eichelbaum *et al*., 1979a; Eichelbaum *et al*., 1979b) entdeckt. In der Folgezeit wurde der gemeinsame polymorphe Metabolisierer-Phänotyp für beide Substrate demonstriert (Eichelbaum *et al*., 1982) und seine genetische Ursache aufgeklärt (Daly, 1995; Gonzalez *et al*., 1988a; Meyer und Zanger, 1997; Price-Evans, 1993; Steiner *et al*., 1985; Zanger *et al*., 1988). Heute sind eine Vielzahl wichtiger Substrate (vgl. Tabelle 1) und 17 Allele bekannt. Damit ist der "Debrisoquin/Spartein"-Polymorphismus der zur Zeit umfangreichste und praktisch bedeutendste pharmakogenetische Polymorphismus (Bertilsson, 1995; Brosen und Gram, 1989b; Eichelbaum und Gross, 1990; Kroemer und Eichelbaum, 1995; Nebert, 1997; Tucker, 1998).

hCYP2D6 wird konstitutiv vorwiegend in der Leber exprimiert und ist im Gegensatz zu allen anderen Arzneimittel-metabolisierenden Cytochromen P450 1A1/2, 2B6, 2C8, 2C9, 2C18, 2C19, 2E1 und 3A4/5 nicht induzierbar. Allerdings wurde während der Schwangerschaft ein leicht erhöhter Umsatz von hCYP2D6-Substraten beobachtet (Hogstedt *et al.,* 1983; Wadelius *et al.,* 1997). Der Anteil am gesamten hepatischen Cytochrom P450-Gehalt ist mit nur etwa 2 % im Vergleich zu den beiden anderen wichtigen Arzneimittel-metabolisierenden Isoformen hCYP3A4 mit ≥ 30 % und hCYP2C9 mit ≥ 20 % relativ gering (Shimada *et al.,* 1994). Die inter-individuellen Unterschiede im Expressionsniveau sind dramatisch bis hin zu völliger Defizienz (Shimada *et al.,* 1994).

Eine im Vergleich zur Leber etwa 100fach geringere Expression von hCYP2D6 wurde zudem in verschiedenen extrahepatischen Geweben nachgewiesen und eine Assoziation mit diversen Krankheiten teils widersprüchlich diskutiert: In der Lunge/Lungenkrebs (Guidice *et al*., 1997; Kivistö *et al*., 1997) im Gehirn/Parkinson (Fonne-Fister *et al.,* 1987; Nebert und McKinnon, 1994; Sabbagh *et al.,* 1999), im Gastrointestinaltrakt (Prueksaritanont *et al.,* 1995), in der Brust und in Mammatumoren (Huang et *al*., 1996; Huang *et al*., 1997), in der Blasenmukosa und in Tumorgewebe (Romkes-Sparks *et al.,* 1994) sowie in peripheren mononukleären Blutzellen (Carcillo *et al.,* 1996). Besonders interessant ist die Expression im Gehirn, da einige zentralnervös aktive Pharmaka metabolisiert werden und endogenes Tryptamin durch hCYP2D6 zum Neurotransmitter Dopamin hydroxyliert werden kann (Hiroi *et al*., 1998).

hCYP2D6 ist am Phase I-Metabolismus von etwa 30 % aller klinisch relevanten Arzneimittel unterschiedlichster Arzneimittelgruppen beteiligt (vgl. Tabelle 1; Alvan, 1991; Brosen und Gram, 1989a; Dahl und Bertilsson, 1993; Eichelbaum und Gross, 1992). Neben hCYP3A4 (55 %) und hCYP2C9 (15 %) gehört es damit trotz des niedrigeren Expressionsniveaus zu den wichtigsten Arzneimittel-metabolisierenden Cytochromen P450 (Smith *et al*., 1998). Zum Beispiel wurden die Antihypertensiva Debrisoquin und Propafenon, der β-Blocker Propanolol, das tricyclische Antidepressivum Imipramin usw. als spezifische Substrate von CYP2D6 beschrieben (Eichelbaum und Gross, 1990). hCYP2D6 wird durch Quinidin oder spezifische inhibitorische Antikörper selektiv inhibiert.

**Tabelle 1**

| **Beispiele für Arzneimittel und andere Substrate, die zumindest teilweise von hCYP2D6 metabolisiert werden**. | | | |
|---|---|---|---|
| **Arzneimittelgruppe** | **Beispiel** | **Reaktion** | **Referenz** |
| Monoaminoxidase-Inhibitoren | Amiflamin | NDem | (Alvan *et al*., 1984) |
| β-Blocker | Bufuralol | alH, arH | (Boobis *et al*., 1985) |
| Analgetika | Codein | ODem | (Mortimer *et al*., 1990) |
| Antihypertensiva | Debrisoquin | arH | (Mahgoub *et al*., 1977) |
| Anorektika | Dexfenfluramin | NDea | (Gross *et al*., 1996) |
| Neuroleptika | Haloperidol | NDea | (Tyndale *et al*., 1991b) |
| trizyklische Antidepressiva | Imipramin | arH | (Brosen *et al*., 1986) |
| α₁-Adrenozeptor Antagonisten | Indoramin | arH | (Pierce *et al*., 1987) |
| β₂-adrenerge Stimulanzien | Methoxyphenamin | arH, NDem | (Roy *et al*., 1985) |
| SSRI | Paroxetin | Dem | (Bloomer *et al*., 1992) |
| Antianginal | Perhexilin | alH | (Cooper *et al*., 1987) |
| Antidiabetika | Phenformin | arH | (Oates *et al*., 1982) |
| Antiarrhythmika | Spartein | H | (Eichelbaum *et al*., 1979b) |
| Antiöstrogene | Tamoxifen | arH | (Dehal und Kupfer, 1997) |
| Amphetamin (*life-style* Droge) | MDMA (Ecstasy) | alH | (Tucker *et al*., 1994) |
| endogener Neurotransmitter | Tryptamin | arH | (Hiroi *et al*., 1998) |
| Abkürzungen: alH: aliphatische Hydroxylierung; arH: aromatische Hydroxylierung; Dem: Demethylenierung; MDMA: Methylendioxymethamphetamin; NDea: N-Dealkylierung; NDem: N-Demethylierung; ODem: O-Demethylierung; SSRI: Selektiver Serotonin Reuptake Inhibitor | | | |

Jedes Jahr sterben nach Schätzungen der WHO weltweit 250.000 Frauen an Brustkrebs (Logan, 1975). In den USA und Westeuropa ist Brustkrebs die Ursache in 4 % aller Todesfälle bei Frauen. (American Cancer Society). Allein in Deutschland erkranken jährlich etwa 42.000 Frauen an Brustkrebs (Becker und Wahrendorf, 1981-1990). Rund 30 % der Tumore sind hormonsensitiv.

Zur Therapie Östrogen-sensitiver Tumore wird der nicht-steroidale selektive Östrogenrezeptor-Modulator Tamoxifen (Novaldex®) eingesetzt (Furr und Jordan, 1984; Jordan, 1998; Osborne, 1998). Tamoxifen bindet an den Östrogenrezeptor und blockiert so die Stimulation der Proliferation durch Bindung von Östrogen. In anderen Organen hingegen besitzt es eine erwünschte paradoxe östrogene Partialwirkung, beispielsweise wirkt es der Osteoporose entgegen (MacGregor und Jordan, 1998). Derzeit werden Studien zum präventiven Gebrauch von Tamoxifen bei Risikogruppen durchgeführt (Jordan, 1997; Nayfield, 1995).

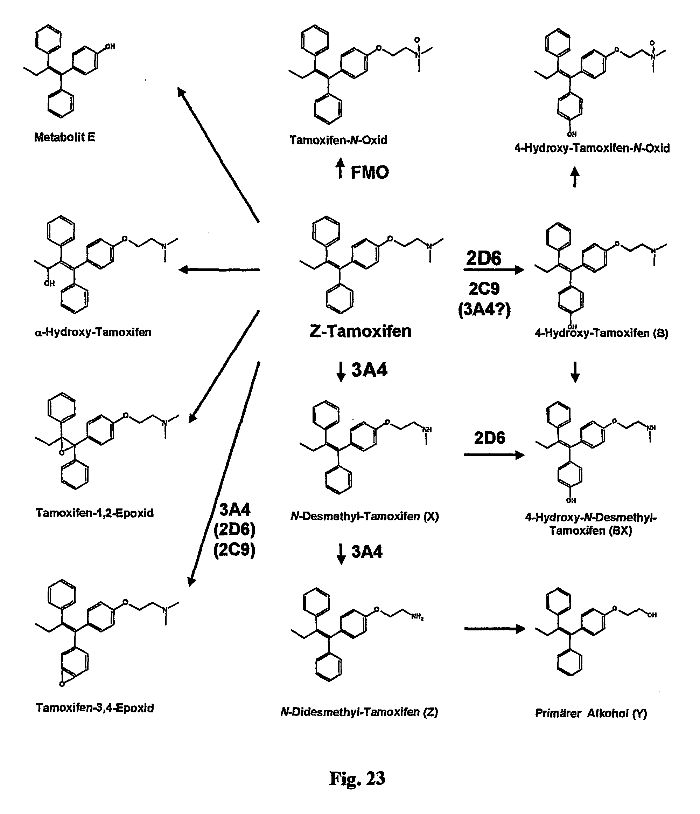

Tamoxifen zeigt hinsichtlich Pharmakologie, Metabolitenprofil und DNA-Adduktbildung ausgeprägte Speziesunterschiede (De Matteis *et al*., 1998; Glatt *et al*., 1998; Jordan und Chem, 1982; Jordan und Robinson, 1987; Lim *et al*., 1994). Die Hauptmetaboliten sind *N*-Desmethyl-Tamoxifen, Tamoxifen-*N*-Oxid und 4-Hydroxy-Tamoxifen; vgl. Figur 23. 4-Hydroxy-Tamoxifen ist etwa 100fach stärker anti-östrogen als die Muttersubstanz selbst und trägt daher trotz eines niedrigeren Plasmaspiegels vermutlich wesentlich zur pharmakologischen Wirkung bei (Borgna und Rochefort, 1981; Furr und Jordan, 1984). Diskutiert als die an der Tamoxifen-4-Hydroxylierung beteiligten Cytochrom P450-Isoformen werden hCYP2C9, hCYP2D6, hCYP2E1 und hCYP3A4 (Crewe *et al.,* 1997; Dehal und Kupfer, 1997; Styles *et al.,* 1994). Mögliche inter-individuelle Unterschiede hinsichtlich der Bildung von 4-Hydroxy-Tamoxifen müßten möglicherweise bei der Festlegung der therapeutischen Dosis von Tamoxifen berücksichtigt werden.

Zusammen mit den Pseudogenen *CYP2D7P* und *CYP2D8P* bildet *CYP2D6* einen Gencluster am *CYP2D*-Locus an Position q13.1 auf dem langen Arm von Chromosom 22 (Eichelbaum *et al*., 1987; Gonzalez *et al*., 1988b; Gough *et al*., 1993; Kimura *et al*., 1989). Es besteht wie die beiden Pseudogene aus 9 Exons und 8 Introns.

Von den 17 bekannten *hCYP2D6*-Allelen kodieren lediglich fünf für ein (eingeschränkt) funktionales Enzym, nämlich *hCYP2D6*1, hCYP2D6*2, hCYP2D6*9, hCYP2D6*10* und *hCYP2D6*17* (Daly *et al*., 1996a). Mindestens ein weiteres funktionales Allel wird für die ghanaische Bevölkerung postuliert (Droll *et al*., 1998; Masimirembwa *et al.,* 1996a). Die phänotypische Einteilung erfolgt anhand des Quotienten Testsubstrat/Metabolit im Urin pro Zeiteinheit. Je kleiner diese "*metabolic ratio (MR)*" ist, desto schneller wird das Testsubstrat, beispielsweise Debrisoquin, Dextromethorphan, Metoprolol oder Spartein, metabolisiert. Homozygote Träger nicht-funktionaler Allele sind stets defizient hinsichtlich der 2D6-Aktivität und weisen einen sogenannten "*poor metabolizer (PM)*"-Phänotyp mit großer MR auf. Homozygote Individuen für das Wildtyp-Allel *hCYP2D6*1* sind phänotypisch *"extensive metabolizer (EM)*" mit kleiner MR (Sachse *et al.,* 1997). Genamplifikation eines funktionalen Alleles führt zum sogenannten "*ultrarapid metabolizer (UM)*"-Phänotyp (Bertilsson *et al.,* 1993; Johansson *et al*., 1993; Lundqvist *et al*., 1999). Homozygote Träger des eingeschränkt funktionalen Alleles *hCYP2D6*10* besitzen gegenüber dem EM-Phänotyp eine erhöhte MR und werden daher gelegentlich als "*intermediate metabolizer (IM)*" bezeichnet (Armstrong *et al.,* 1994; Yokota *et al.,* 1993).

Variationen der Allelhäufigkeiten zwischen verschiedenen Populationen führen außerdem zu inter-ethnischen Unterschieden (Bertilsson, 1995): Bei Kaukasiern betragen die Häufigkeiten der funktionalen Allele *hCYP2D6*1* und *hCYP2D6*2* rund 35 *%, hCYP2D6*2xN*, *hCYP2D6*9* und *hCYP2D6*10* sind mit 1 - 2 % selten, *hCYP2D6*17* wurde bisher nicht nachgewiesen. Die nicht-funktionalen Allele *hCYP2D6*4* und *hCYP2D6*5* sind mit etwa 20 % bzw. 5 % verbreitet, die übrigen mit 0 - 2 %. Der Anteil an schlechten Metabolisierern liegt damit in der kaukasischen Bevölkerung bei ca. 5 - 10 % (Alvan *et al*., 1990; Evans *et al*., 1993; Griese *et al*., 1998; Sachse *et al*., 1997).

In asiatischen Populationen hingegen beträgt die Häufigkeit für das nicht-funktionale Allel *hCYP2D6*4* lediglich 0,8 %, diejenige des eingeschränkt funktionalen Allels *hCYP2D6*10* jedoch 23 - 70 %. Entsprechend sind zwar nur etwa 1 % der Asiaten schlechte Metabolisierer, jedoch ist die MR gegenüber Kaukasiern im Mittel erhöht (Bertilsson *et al*., 1992; Dahl *et al*., 1995b; Horai *et al*., 1989; Roh *et al*., 1996).

Ähnliches gilt für einige schwarzafrikanische Populationen mit Allelfrequenzen von etwa 4 % für *hCYP2D6*4*, ca. 5 % für *hCYP2D6*10* und 15 - 35 % für *hCYP2D6*17* (Evans *et al*., 1993; Masimirembwa *et al.,* 1996b).

Verschiedene *hCYP2D6*-Allele wurden in diversen Systemen heterolog exprimiert: In *E. coli* (Gillam *et al*., 1995; Kempf *et al*., 1995; Pritchard *et al*., 1998), in Hefe (Bichara *et al*., 1996; Ellis *et al*., 1992; Krynetski *et al*., 1993; Krynetski *et al*., 1995; Oscarson *et al.,* 1997), in Insektenzellen (Evert *et al.,* 1997; Paine *et al*., 1996; Patten *et al*., 1996), in CHO-Zellen (Patten *et al.,* 1996), in COS-1 Zellen (Gonzalez *et al*., 1990; Johansson *et al*., 1994; Kagimoto *et al*., 1990; Oscarson *et al*., 1997), in Hep G2-Zellen (Aoyama *et al.,* 1990; Tyndale *et al.,* 1991a), in NIH3T3-Zellen (de Groene *et al.,* 1996) und in den humanen B lymphoblastoiden Zellen AHH-1 TK+/- (Crespi *et al*., 1991; Crespi *et al*., 1995; Penman *et al*., 1993). Das Wildtyp-Allel *hCYP2D6*1* wurde zudem schon in V79 Chinesischen Hamsterzellen exprimiert (Fischer *et al.,* 1992; Rauschenbach *et al.,* 1997).

Es existiert bisher allerdings kein Testsystem, das für eine Untersuchung der pharmakologischen, toxikologischen und anderer Aspekte des hCYP2D6-Polymorphismus geeignet wäre.

Der vorliegenden Erfindung liegt die technische Aufgabe zugrunde, ein Testsystem bereitzustellen, das eine vergleichende Untersuchung der metabolischen Aktivität aktiver Formen humanen Cytochroms P450 2D6 in Bezug auf eine große Vielfalt von Stoffen ermöglicht. Bevorzugt soll dieses System als analytisches Werkzeug in der präklinischen Arzneimittel-Entwicklung und zur *in vitro*-Darstellung des humanen und Speziesvergleichenden Fremdstoff-Metabolismus geeignet sein. Es soll so einen Beitrag zum Ersatz und zur Ergänzung von Tierversuchen in der Pharmakologie und Toxikologie leisten. Insbesondere soll das System für Studien des Phase 1-Arzneimittel-Metabolismus geeignet sein und eine zuverlässige, einfache und kostengünstige Identifizierung der am Umsatz eines Arzneimittel-Kandidaten beteiligten Enzyme zu einem möglichst frühen Zeitpunkt der präklinischen Arzneimittel-Entwicklung ermöglichen.

Der vorliegenden Erfindung liegt weiterhin die technische Aufgabe zugrunde, Verfahren zur Untersuchung pharmakologischer und toxikologischer Aspekte des hCYP2D6-Polymorphismus bereitzustellen. Bevorzugt sollen diese Verfahren in der präklinischen Phase der Arzneimittel-Entwicklung Anwendung finden, sollen unter standardisierten und reproduzierbaren Bedingungen erfolgen und einen hohen prädiktiven Wert für den Menschen aufweisen. Durch diese Verfahren sollen bevorzugt Tierversuche in dieser Phase ersetzt werden.

Diese Aufgaben werden erfindungsgemäß durch den Gegenstand der Patentansprüche gelöst.

In einem ersten Aspekt betrifft die Erfindung ein Testsystem, umfassend Zelllinien, die jeweils verschiedene funktionale humane Cytochrom P450 2D6-Allele heterolog exprimieren.

Es werden die fünf *hCYP2D6*-Allele **1, *2, *9, *10* und **17*, die für funktionales Enzym kodieren, heterolog in diesem zellulären System exprimiert. Eine bevorzugte Zelle für eine Expression weist normalerweise keine Cytochrom P450-Aktivität auf. Das Expressionsniveau und die enzymkinetischen Eigenschaften des erfindungsgemäßen rekombinanten hCYP2D6-exprimierenden Systems entsprechen bevorzugt sowohl der physiologischen Situation als auch denen vergleichbarer Expressionssysteme. Ein bevorzugtes Expressionssystem sind eukaryotische Zellen, insbesondere Säugerzellen, und am besten Fibroblastenzellen. In einer Ausführungsform entstammen die Zellen dem Chinesischen Hamsters und sind bevorzugt Lungenfibroblasten des Chinesischen Hamsters oder davon abgeleitete Zellen, insbesondere V79-Zellen. In einer bevorzugten Ausführungsform wird cDNA exprimiert. Das erfindungsgemäße Testsystem ist bevorzugt zur umfassenden *in vitro* Darstellung des humanen Cytochrom P450 2D6-Polymorphismus geeignet. In einer besonders bevorzugten Ausführungsform ermöglicht das Testsystem die Eigenschaften der fünf bekannten funktionalen Formen humanen Cytochroms P450 2D6 unter standardisierten experimentellen Bedingungen zu prüfen und zu vergleichen. Bevorzugt werden erfindungsgemäß parentale bzw. Schein-transfizierte Zellinien als Negativkonktrolle eingesetzt werden.

Die Zellinie V79 wurde in den 50er Jahren aus morphologisch und neoplastisch transformierten Lungenfibroblasten eines adulten männlichen Chinesischen Hamsters etabliert. Die Entstehung der Zellinie V79 wurde nicht veröffentlicht. Aus der Beschreibung der Versuchsreihe zur Etablierung von Zellinien aus dem Lungengewebe des Chinesischen Hamsters kann durch Analogie auf die Entstehung der Zellinie V79 geschlossen werden (Ford und Yerganian, 1958). Sie hat seitdem breite Anwendung in Toxizitäts- und Mutagenitätsstudien gefunden (Bradley *et al.,* 1981; Chu und Malling, 1968; Doehmer, 1993; Sawada und Kamataki, 1998; *Swierenga et al.,* 1991) und ist nach den OECD Guidelines for the Testing of Chemicals zertifiziert.

Die in dem erfindungsgemäßen Testsystem eingesetzten V79-Zellinien können bereits etablierte V79-Zellinien sein.

Allerdings unterscheiden sich die bakannten V79-Zellinien teilweise deutlich voneinander. Besonders bevorzugt ist der Subklon V79MZ. Im Gegensatz zu anderen V79-Zellen wächst dieser Subklon an ein Substrat angeheftet ohne sich davon zu lösen. Ferner besitzt dieser Subklon im Gegensatz zum Beispiel zu V79NH-Zellen keine Acetyltransferase-Aktivität, die die mit dem erfindungsgemäßen Testsystem durchgeführten Messungen beeinflussen könnte. Daher weist das erfindungsgemäße Testsystem in V79MZ-Zellen deutliche Vorteile auf.

Bevorzugte erfindungsgemäße Cytochrom P450 2D6 exprimierende Zellinien sind daher die am 15.02.2000 bei der DSMZ-Deutsche Sammlung von Mikroorganismen und Zellkulturen GmbH unter den Zugangsnummern DSM ACC2446, DSM ACC2447, DSM ACC2448, DSM ACC2449 und DSM ACC2450 hinterlegten Zellinien V79MZh2D6*1, V79MZh2D6*2, V79MZh2D6*9, V79MZh2D6*10 und V79MZh2D6*17.

Mit der Bereitstellung des erfindungsgemäßen hCYP2C-exprimierenden Testsystems steht der humane Phase I-Arzneimittel-Metabolismus praktisch vollständig als *in vitro*-Testsystem zur Verfügung. Die erfindungsgemäßen Zellinien eignen sich zur Identifizierung der für ein bestimmtes Substrat metabolisch kompetenten Cytochrom P450-Isoformen, zur Aufklärung von Metabolitenprofilen und Substrat-Bindungsmechanismen, zur Untersuchung von Enzymkinetiken und Arzneimittel-Wechselwirkungen sowie zu Studien zur Zyto- und Genotoxizität.

Das erfindungsgemäße Testsystem in eukaryotischen Zellen ist für metabolische Studien der funktionellen Isoformen humanen Cytochroms P450 2D6 geeignet. Es vermeidet dabei die ethische Problematik bei Verwendung menschlichen oder tierischen Organmaterials. Weiterhin bietet das Testsystem den Vorteil exakt definierter und standardisierbarer Versuchsbedingungen, die mit *in vivo*-Systemen oder *nativen* Gewebeproben wegen der außerordentlichen Komplexität und der inter-individuellen Variabilität des Materials nicht erreichbar sind. So ist in komplexen Systemen die Identifizierung der metabolisch kompetenten Cytochrome P450 nur indirekt möglich, etwa durch Inhibitionsstudien durch inhibitorische Antikörper oder inhibitorisch wirkende Chemikalien nach Induktion der zu untersuchenden Isoform mittels bestimmter Chemikalien wie Dexamethason, Phenobarbital oder Dioxin. Die indirekte Identifizierung hat nur indikativen Wert, da inhibitorische Antikörper selten vollständig hemmen oder nicht spezifisch genug sind. Inhibitorisch wirkende Chemikalien sind in keinem Fall Cytochrom P450-spezifisch. Induzierende Chemikalien können irreführend sein, da eine geringfügige Stimulation anderer Cytochrome P450 eine Beteiligung des hauptsächlich induzierten Cytochroms P450 überlagern kann. Die erfindungsgemäße heterologe Expression erlaubt hingegen die direkte und eindeutige Zuordnung von Cytochrom P450-Isoformen und Metaboliten.

Das erfindungsgemäße Testsystem bietet gegenüber Studien mit aufgereinigtem Enzym den Vorteil, daß die Zellen ohne aufwendige Aufreinigungsschritte verwendet werden können. Zudem vermeidet das erfindungsgemäße System eine Veränderung der Substratspezifität der getesteten Cytochrom P450 2D6-Formen oder eine Verunreinigung der zu testenden Enzyme durch eine Aufreinigung. Das System in Säugerzellen bietet weiterhin den Vorteil, daß es z.B gegenüber Hefezellen relevante und wichtige biologische und metabolische Endpunkte und eine metabolische Kompetenz bietet, die denen eines Tieres oder des Menschen vergleichbar sind. Das erfindungsgemäße System in V79-Zellen hat insbesondere den Vorteil, daß diese Zellen eine Vielzahl toxikologischer Endpunkte bei niedrigem und stabilem Hintergrund bieten und sich daher besonders für Mutagenitäts- und Toxizitätsstudien eignen (Bradley *et al*., 1981). Insbesondere zeichnen sich V79-Zellen gegenüber allen anderen Säugerzellinien, einschließlich humanen Zellinien, durch einen außergewöhnlich stabilen, pseudodiploiden Karyotyp mit konstanter Chromosomenzahl aus, der auch nach Transfektion mit Fremd-DNA erhalten werden kann (Doepker *et al*., 1998; Simi *et al*., 1999). Dies ist im Hinblick auf zytogenetische Endpunkte entscheidend. Die Stabilität der Zellen ist weiterhin wichtig für eine zuverlässige Untersuchung gemäß der erfindungsgemäßen Verfahren. Die Verdopplungszeit von V79-Zellen ist mit weniger als 12 Std. im Vergleich zu allen anderen Zellinien die bislang kürzeste. Vor allem exprimieren V79-Zellen kein endogenes Cytochrom P450 (Kiefer und Wiebel, 1989; Onderwater *et al*., 1996) Insbesondere konnte für die Zellinie V79MZ gezeigt werden, dass kein endogenes Cytochrom P450 exprimiert wird und die Zellen sind daher für das transfizierte Cytochrom P450-Isoenzym exakt definiert. Darüber hinaus konnte für die Zellinie V79MZ gezeigt werden, dass die Zellen adhärent in Einzelschichten wachsen und in Kultur einen stabilen Phänotyp zeigen.

Weiterhin werden in V79-Zellen Häm sowie Cytochrom b₅ in ausreichender Menge endogen synthetisiert (Onderwater *et al*., 1996). Im Gegensatz dazu ist bei stark exprimierenden Systemen wie Baculovirus-infizierten Insektenzellen die endogene Häm-Synthese für die Sättigung des exprimierten Cytochroms mit prosthetischem Häm nicht ausreichend (Asseffa *et al*., 1989; Barnes *et al*., 1994; Buters *et al*., 1994; Paine *et al*., 1996). Bei dem erfindungsgemäßen System unter Verwendung von V79-Zellen ist eine Supplementierung mit oder Koexpression von Cytochrom P450-NADPH-Oxidoreduktase nur in Ausnahmefällen notwendig (Schneider *et al*., 1996). Jedoch werden erfindungsgemäß auch Testsysteme umfaßt, die Zellinien enthalten, die funktionale Formen humanen Cytochroms P450 2D6 und eine Cytochrom P450-NADPH-Oxidoreduktase und/oder Cytochrom b₅, bevorzugt menschlichen Ursprungs, coexprimieren. Das erfindungsgemäße System bietet weiterhin den Vorteil, daß die zu exprimierenden Nukleinsäuresequenzen, insbesondere cDNA-Sequenzen, nicht wie bei Expression in *E*. *coli* adaptiert werden müssen (Sengstag *et al*., 1994). Weiterhin sind geeignete intrazelluläre Membransysteme zur Cytochrom P450-Inkorporation vorhanden, so daß keine nachträgliche Rekonstitution erforderlich ist (Gillam *et al*., 1995). V79-Zellen sind außerdem im Gegensatz zum Beispiel zu Hefezellen für viele Substrate permeabel. Metabolismus-Untersuchungen können daher auch unter Ausnutzung des zelleigenen Stoffwechsels, beispielsweise zur NADPH-Regeneration, direkt in der Zellkultur durchgeführt werden.

Erfindungsgemäß bereitgestellt wird ein Kit, der das erfindungsgemäße Testsystem von humanes Cytochrom P450 2D6 exprimierenden Zellinien enthält. Der erfindungsgemäße Kit kann die erfindungsgemäßen Cytochrom P450 2D6 exprimierenden Zellinien und/oder ein Lysat und/oder eine mikrosomale Fraktion davon und/oder eine für Cytochrom P450 2D6 angereicherte Fraktion und/oder aufgreinigtes Cytochrom P450 2D6 enthalten. Als weitere Bestandteile kann der erfindungsgemäße Kit z.B. Wachstumsmedien, Kontrollsubstanzen und - zellen, Leberextrakt, -homogenat und/oder -mikrosomen und/oder Stoffwechsel-aktive Zellen, wie primären Hepatozyten-Kulturen und/oder Stoffwechsel-aktive Enzyme, wie Cytochrome P450 usw. umfassen.

Im nachfolgenden werden verschiedenen Verwendungsmöglichkeiten des erfindungsgemäßen Testsystems mit den humanes Cytochrom P450 2D6 exprimierenden Zellen beschrieben. Die erfindungsgemäßen Verfahren erfolgen entweder mit ganzen Zellen, einem Lysat oder einer mikrosomalen Fraktion davon, einer für Cytochrom P450 2D6 angereicherten Fraktion oder mit aus den erfindungsgemäßen Cytochrom P450 2D6 exprimierenden Zellen aufgereinigtem Cytochrom P450 2D6. In einer Ausführungsform sollen die erfindungsgemäßen Zellen in Kombination mit Leberextrakt, -homogenat und/oder -mikrosomen und/oder Stoffwechsel-aktiven Zellen, wie primären Hepatozyten-Kulturen und/oder Stoffwechsel-aktiven Enzymen, wie weitere Cytochrome P450, Carboxylesterasen, Exoxidhydrolasen, Flavin-enthaltende Monooxigenasen, N-Acetyltransferasen, Sulfotransferasen, Glutathion S-Transferase, Methyltransferasen, Monoamin- und Diaminoxidasen usw., Anwendung finden.

Die Zellen lassen sich auch in der Kombination mit Elektroden zur Kultivierung auf Silizium oder ähnlichem Material zur direkten Kopplung von Enzym und Datenträgern einsetzen und als metabolisch kompetente BioChips in der Pharmakologie und Toxikologie verwenden.

Das erfindungsgemäße Testsystem humanes Cytochrom P450 2D6 exprimierender Zellinien kann in der Untersuchung einer genetisch bedingten Toxizität von bestimmten Metaboliten, wie Arzneistoffen verwendet werden. Weiterhin ermöglicht das erfindungsgemäße System die Untersuchung und Bestimmung der metabolischen Aktivierung von kanzerogenen Substanzen und ermöglicht somit die Bestimmung einer kanzerogenen Wirkung bestimmter Verbindungen, insbesondere in Abhängigkeit der Form des exprimierten humanen Cytochroms P450 2D6. Somit betrifft die vorliegende Erfindung ein Verfahren zur Identifikation von mutagenen, kanzerogenen oder toxischen Wirkungen von Substanzen, wobei die erfindungsgemäßen humanes Cytochrom P450 2D6 exprimierenden Zellinien mit der zu untersuchenden Substanz kontaktiert werden. Eine mutagene Wirkung kann zum Beispiel durch die Messung von gegenüber bestimmten Zellgiften, wie Antibiotika, resistenten Zellen oder Stoffwechsel-veränderten Zellen, wie das Auftreten eines Hypoxanthin-Guanin-Phosphoribosyl-Transferase (HGPRT)-negativen Phänotyps, nachgewiesen werden. Eine toxische Wirkung der zu testenden Substanz kann beispielsweise durch ein Absinken der Überlebensfähigkeit der erfindungsgemäßen Zellinien ermittelt werden. Eine kanzerogene Wirkung kann durch Auftreten eines entarteten Teilungsphänotyps der erfindungsgemäßen Zellinien, wie unbegrenzte Teilungsfähigkeit, oder aufgrund der Fähigkeit der erfindungsgemäßen Zellinien, Tumore in Versuchstieren zu erzeugen, getestet werden. Das erfindungsgemäße Testsystem ermöglicht dabei eine hohe experimentelle Sensitivität und ermöglicht die Untersuchung der metabolischen Funktionen verschiedener polymorpher Formen des funktionalen humanen Cytochroms P450 2D6.

Die Eignung des erfindungsgemäßen Systems von Zellinien als analytisches Werkzeug in der präklinischen Arzneimittel-Entwicklung wurde erfindungsgemäß am Beispiel der pharmakologisch und toxikologisch wichtigen 4-Hydroxylierung des Brustkrebs-Therapeutikums Tamoxifen demonstriert. Die Übereinstimmung der katalytischen Eigenschaften der neuen polymorphen Zellinien mit den Ergebnissen früherer *in vitro-* und in vivo-Untersuchungen demonstriert die Eignung der neuen Zellbatterie *zur in vitro*-Darstellung des hCYP2D6-Polymorphismus. Mit den erfindungsgemäßen Zellinien sind erstmals vergleichende enzymkinetische Untersuchungen für die verschiedenen polymorphen menschlichen hCYP2D6-Formen möglich. Die erfindungsgemäßen Zellinien sind besonders zur Identifizierung der an komplexen Metabolismus-Situationen beteiligten Cytochrom P450 Isoformen und der Aufklärung ihrer Metabolitenprofile geeignet, weil sie für einzelne Isoformen exakt definiert sind und ein Arbeiten unter standardisierten, reproduzierbaren Bedingungen ermöglichen. In Übereinstimmung mit den Ergebnissen aus der hCYP2D6-spezifischen Bufuralol-Hydroxylierung konnte außerdem erfindungsgemäß gezeigt werden, daß Träger der Allele *hCYP2D6*17* und insbesondere *hCYP2D6*10* deutlich niedrigere Plasmaspiegel an 4-Hydroxy-Tamoxifen entwickeln als Träger der übrigen funktionalen Allele. Weil 4-Hydroxy-Tamoxifen mindestens 100fach stärker anti-östrogen ist als Tamoxifen selbst, können individuelle Unterschiede in der Bildung dieses aktiven Metaboliten für den therapeutischen Effekt von erheblicher klinischer Bedeutung sein.

Somit betrifft die Erfindung in einem weiteren Aspekt ein Verfahren der präklinischen Arzneimittel-Entwicklung unter Verwendung des erfindungsgemäßen Testsystems. Dabei sollen *in vitro*-Studien Hinweise auf hCYP2D6-Polymorphismus-abhängige Unterschiede des *in vivo*-Metabolismus verschiedener Verbindungen, einschließlich Arzneistoffe, wie Tamoxifen liefern. Ausgeprägte inter-individuelle Unterschiede bei der metabolischen Prozessierung, was in einer Bildung pharmakologisch weniger aktiver oder potenterer Metaboliten resultiert, wie die Bildung von 4-Hydroxy-Tamoxifen aus Tamoxifen, müßten möglicherweise bei der Festlegung der therapeutischen Dosis berücksichtigt werden.

Weiterhin ermöglicht das erfindungsgemäße Testsystem die Identifizierung von Individuen, die aufgrund der metabolischen Aktivität oder Defizienz der spezifisch exprimierten Cytochrom P450 2D6-Formen zu einer Risikogruppe zählen. Derartige Individuen zeichnen sich z.B. durch eine metabolische Konversion von Xenobiotika zu toxischen, mutagenen oder karzinogenen Formen oder eine fehlende Entgiftung von Arzneistoffen oder anderen Xenobiotika aus.

Zur Messung der 4-Hydroxylierung von Tamoxifen werden die Zellen des erfindungsgemäßen Testsystems oder z.B. ein Homogenat davon mit Tamoxifen zur Reaktion gebracht. Das Reaktionsprodukt, 4-Hydroxy-Tamoxifen, kann sodann mit bekannten Verfahren gemessen werden. Unterschiede in der Menge des Reaktionsprodukts zwischen den verschiedenen Zellinien des erfindungsgemäßen Testsystems zeigen, ob Tamoxifen durch die verschiedenen Formen humanen Cytochroms P450 2D6 metabolisiert, schlecht metabolisiert oder nicht metabolisiert wird.

Erfindungsgemäß wird ein Verfahren zum Screening von Arzneistoffen unter Verwendung des erfindungsgemäßen Testsystems von Cytochrom P450 2D6 exprimierenden Zellinien bereitgestellt. Das Verfahren ermöglicht ein Auffinden von Substanzen, die durch die verschiedenen Formen oder durch bestimmte Formen humanen Cytochroms P450 2D6 metabolisiert oder nicht metabolisiert werden. Dabei wird eine zu testende Substanz nacheinander mit den verschiedenen Zellinien des erfindungsgemäßen Testsystems kontaktiert und ein metabolisches Produkt gemessen. Das Vorhandensein eines metabolischen Produkts weist darauf hin, daß die jeweilige Form von humanem Cytochrom P450 2D6 zur Metabolisierung des Stoffes in der Lage ist. Somit ermöglicht dieses erfindungsgemäße Verfahren zum Beispiel ein Auffinden von neuen Arzneimitteln, die Derivate bereits bekannter Verbindungen sind und eine pharmakologische Wirkung beibehalten, wobei sie jedoch nicht oder weniger stark metabolisiert werden. Ferner ermöglicht dieses Verfahren ein Auffinden von Substanzen, die besser metabolisiert werden und dadurch eine schnellere Entgiftung des Körpers gewährleisten.

Zum Beispiel können verschieden modifizierte Formen von Tamoxifen, die vorzugsweise pharmakologisch aktiv sind, mit den Zellen des erfindungsgemäßen Testsystems oder z.B. einem Homogenat davon kontaktiert und zur Reaktion gebracht werden. Die Menge bestimmter Metaboliten als Reaktionsprodukte, wie eine 4-hydoxylierte Form, zeigt, ob eine modifizierte Form von Tamoxifen gut oder schlecht von den verschiedenen oder bestimmten Formen humanen Cytochroms P450 2D6 umgesetzt wird.

**Die hier verwendeten Abkürzungen besitzen die folgende Bedeutung:**
Außer den im Duden gebräuchlichen Akürzungen, den üblichen Codes für Aminosäuren und Nukleotide sowie den gängigen Kürzeln für Restriktionsenzyme, Polymerasen, usw. wurden verwendet:
- A: Absorption
- APS: Ammoniumpersulfat
- bp: Basenpaar(e)
- B SA: Rinderserumalbumin
- cDNA: komplementäre Desoxyribonukleinsäure
- CMV: Cytomegalovirus
- Cyt b₅: Cytochrom b₅
- Cyt_{c}: Cytochrom c
- CYP: Cytochrom P450-Protein, -mRNA, -cDNA
- *CYP*: Cytochrom P450-Gen
- CYPOR: NADPH-abhängige Cytochrom P450-Oxidoreduktase
- Da: Dalton
- DEPC: Diethylpyrocarbonat
- DMEM: Dulbecco's Modified Eagle's Medium
- DMSO: Dimethylsulfoxid
- DNA: Desoxyribonukleinsäure
- dNTP: Desoxynukleosidtriphosphat
- ECL: *Enhanced* Chemilumineszenz
- *E. coli*: *Escherichia coli*
- EDTA: Ethylendiamintetraacetat
- EM: *extensive metabolizer*
- FCS: Fötales Kälberserum
- FITC: Fluorescein-Isothiocyanat
- G418: Geneticin 418-Sulfat
- H: Homogenat
- HEPES: N-(2-Hydroxyethyl)-piperazin-N'-2-ethansulfonsäure
- IM: *intermediate metabolizer*
- kbp: Kilobasenpaare
- kDa: Kilodalton
- K_{M}: Michaelis-Menten-Konstante
- LB: Luria Broth
- LMW: *low molecular weight*
- LTR: *long terminal repeat*
- MPSV: Myeloproliferativer Sarkoma Virus
- MR: *metabolic ratio*
- Mᵣ: relatives Molekulargewicht
- NADP⁺: Nikotinamid-Adenin-Dinukleotid-Phosphat, oxidiert
- NADPH + H⁺: Nikotinamid-Adenin-Dinukleotid-Phosphat, reduziert
- OD: optische Dichte
- P: Pellet
- PAGE: Polyacrylamid-Gelelektrophorese
- PBS: phosphatgepufferte Salzlösung ohne Mg²⁺ und Ca²⁺
- PCR: Polymerase-Kettenreaktion
- PEG: Polyethylenglykol
- pK_{B}: Basenexponent
- PMSF: Phenylmethylsulfonylfluorid
- PM: *poor metabolizer*
- RSV: Rous-Sarcoma-Virus
- SDS: Natriumdodecylsulfat
- SV40: Simian-Virus 40
- TAM: Tamoxifen
- TEMED: N, N, N', N'-Tetramethylendiamin
- Tris: Tris-(hydroxymethyl)-aminomethan
- TRITC: Tetramethyl-Rhodamin-Isothiocyanat
- TSS: *transformation storage solution*
- U: Unit(s) (Enzymeinheit(en))
- UM: *ultrarapid metabolizer*
- upm: Umdrehungen pro Minute
- V79-Zellen: V79 Chinesische Hamster Fibroblasten
- V79MZ-Zellen: V79 Chinesische Hamster Fibroblasten, Mainzer Subklon
- V79MZCYP-Zellen: gentechnisch veränderte V79MZ-Zellen, die heterolog Cytochrom P450 exprimieren
- V79MZh2D6-Zellen: gentechnisch veränderte V79MZ-Zellen, die humanes Cytochrom P450 2D6 exprimieren
- Vₘₐₓ: maximale Reaktionsgeschwindigkeit
- % (m/v): Massenprozent
- % (v/v): Volumenprozent

### Speziesabkürzungen:

- b: Rind
- h: Mensch
- m: Maus
- r: Ratte
- f: Fisch (*Stenotonus chrysops*)

Die Erfindung wird weiter durch die folgenden Zeichnungen erläutert:

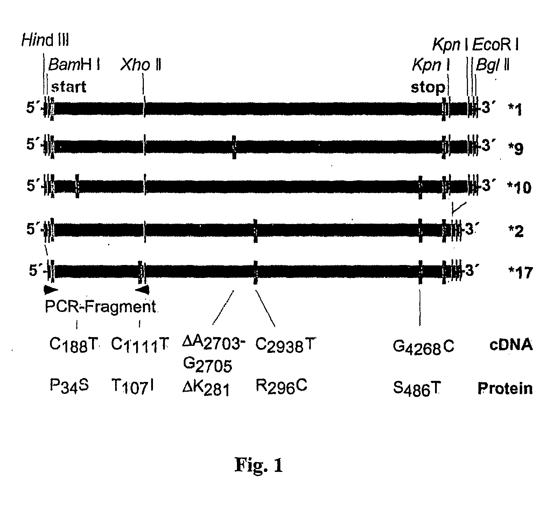

Figur 1 zeigt die Struktur der erfindungsgemäß exprimierten hCYP2D6-cDNAs. Die Wildtyp-cDNA hCYP2D6*1 kodiert für das native hCYP2D6 1 mit Val₃₇₄ (GenBank # g181349; SwissProt # P10635; Crespi *et al*., 1995; Gonzalez *et al*., 1988). Die Mutationen der cDNAs hCYP2D6*2, *9, *10 und *17 gegenüber der Wildtyp-cDNA hCYP2D6*1 sind markiert. Die Positionen sind nach Kimura *et al.* (1989) angegeben.

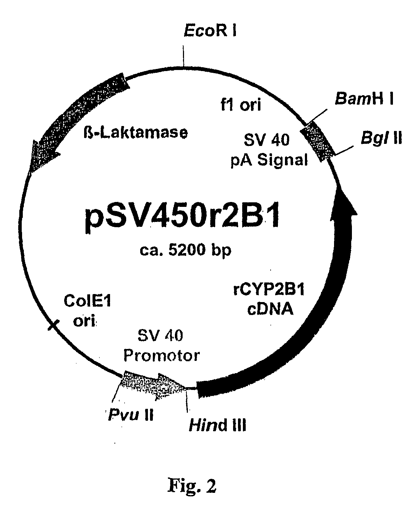

Figur 2 zeigt die Struktur des ursprünglichen Vektors pSV450r2B1 (Doehmer *et al*., 1988). Der Vektor enthält die folgenden Elemente: Fragment *Eco*R I - *Bam*H I: aus dem SV40 Affenvirus (GenBank # J02400; Fiers *et al*. 1978); Fragment *Bam*H I - *Pvu* II: Expressionskassette, die stabil in das Genom der V79-Zellen integriert werden muß; Fragment *Bam*H I - *Bgl* II: enthält das frühe SV40 Polyadenylierungs-Signal, von SV40 abgeleitet; Fragment *Bgl* II *- Hin*d III: inserierte cDNA; Fragment *Hin*d III - *Pvu* II: enthält den frühen SV40 Promotor, von SV40 abgeleitet; Fragment *Pvu* II - *Eco*R I: aus pBR322 (GenBank # J01749).

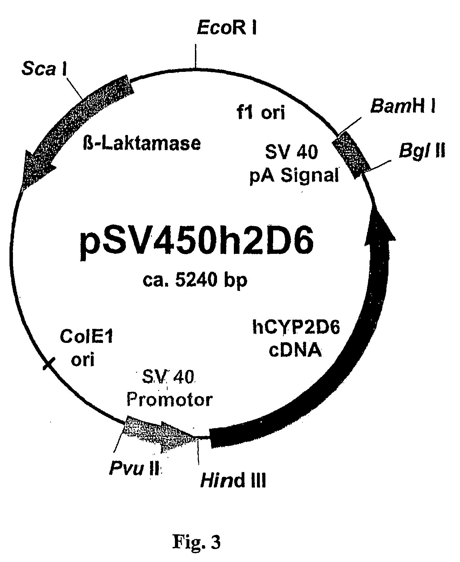

Figur 3: zeigt den Expressionsvektor pSV450h2D6 zur Expression von hCYP2D6-cDNAs; vgl. Figur 1. Zur Transfektion wurde der Expressionsvektor pSV450h2D6 mit *Sca* I linearisiert.

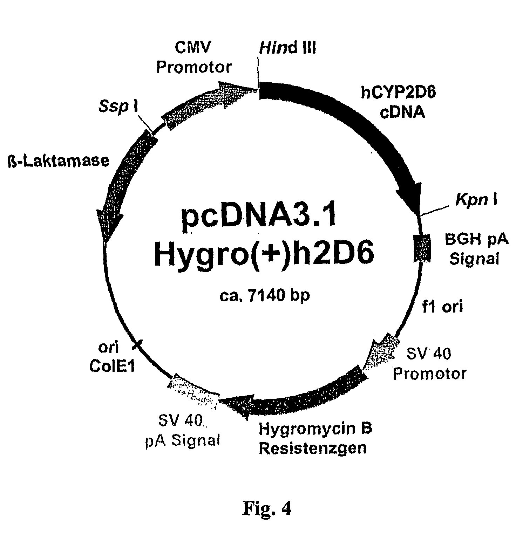

Figur 4 zeigt den Expressionsvektor pcDNA3.1Hygro(+)h2D6. Die cDNA steht unter Kontrolle des Cytomegalovirus (CMV)-Promotors. Das Hygromycin B-Resistenzgen wird über den frühen SV40 Promotor reguliert. Beide Expressionskassetten müssen stabil in das Genom der V79-Zellen integriert werden. Zur Transfektion wurde der Vektor mit *Ssp* I linearisiert.

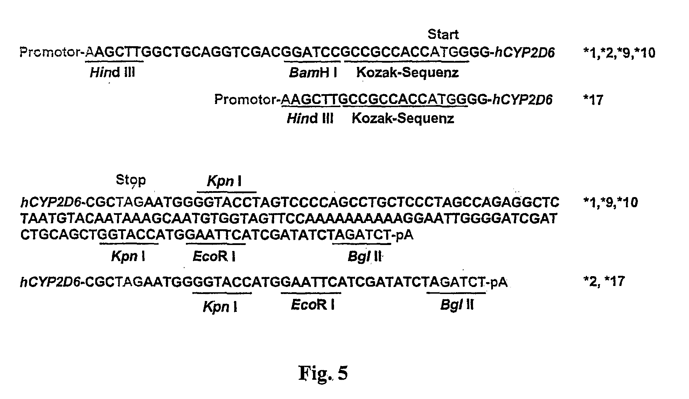

Figur 5 zeigt die Linkerregionen zwischen frühem SV40 Promotor und hCYP2D6-cDNA sowie hCYP2D6-cDNA und früher SV40 Polyadenylierungssequenz im Expressionsvektor pSV450h2D6. Die Kozak-Sequenz ist wichtig für eine hohe Translationsrate der mRNA (Kozak, 1987; Kozak, 1990).

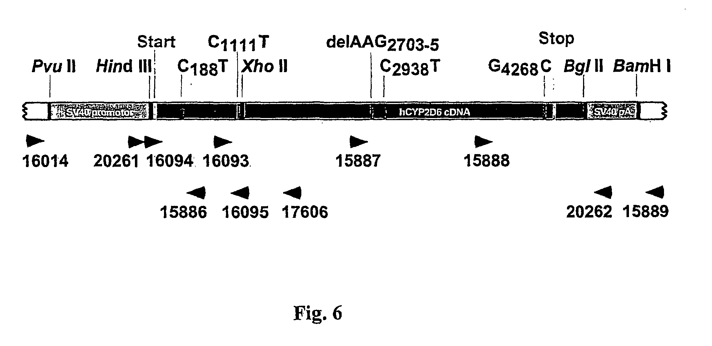

Figur 6 zeigt die relativen Positionen und Orientierungen der in der Erfindung verwendeten Primer am Plasmid pSV450h2D6. Die Primer 16298 und 16299 sind zu den Enden des Templates 16300, die Primer 19176 und 19177 zu den Enden des Templates 19183 komplementär. Die Primer 18383 und 18384 sind zu Bereichen ca. 50 Basenpaare stromaufwärts bzw. 30 Basenpaare stromabwärts des Polylinkers des Vektors pcDNA3.1Hygro(+) komplementär.

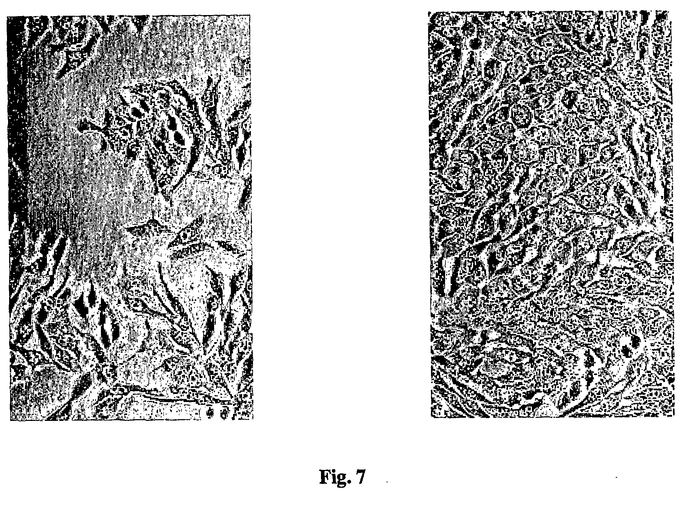

Figur 7 zeigt eine lichtmikroskopische Aufnahme parentaler V79MZ-Zellen im Phasenkontrast bei 200facher Vergrößerung bei knapp 50%iger (links) und 100%iger (rechts) Konfluenz. Die Zellen wachsen flach am Boden angeheftet und besitzen zahlreiche Nukleoli. Mitotische Zellen, idealerweise etwa 3% aller Zellen, runden sich vorübergehend ab.

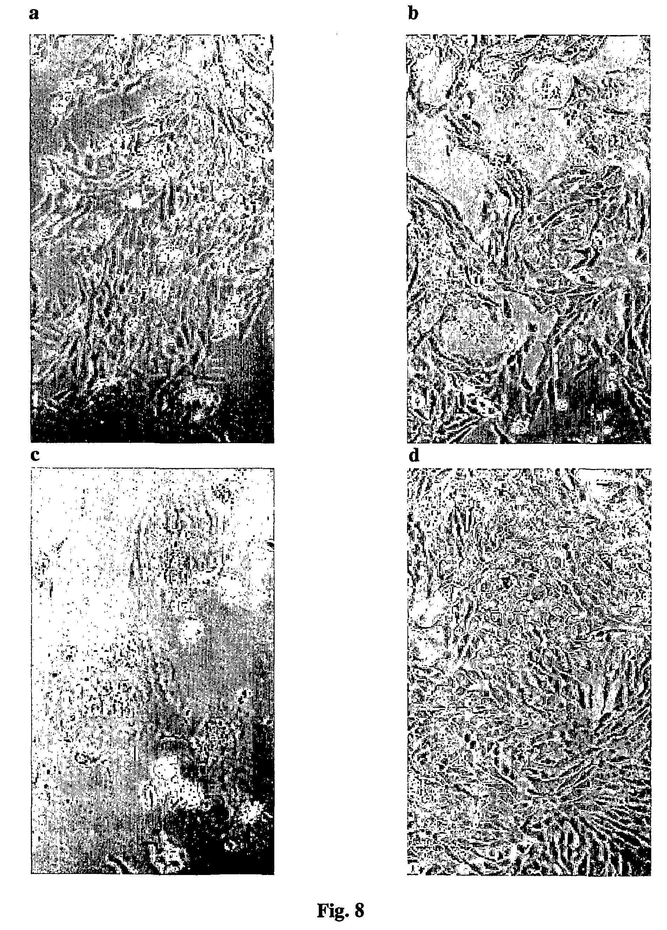

Figur 8 zeigt durch Transfektion morphologisch veränderte V79MZ-Zellen am Beispiel der Klone V79MZh2D6*9#C6 (a), V79MZf1A1#2 (b) und V79MZh2E1#13 (c) im Vergleich zu parentalen V79MZ-Zellen (d) im Phasenkontrast bei 200facher Vergrößerung. Einige Zellen waren stark vergrößert, rundlich und auffallend häufig polyploid (b und c). Während der gesamten Kultivierung war ein Anteil von etwa 2-3 % abgestorbener Zellen vorhanden (helle Flecken in a, b und c). Die Verdopplungszeit war erhöht. In einigen Fällen wurde auch bei andauernder Kultivierung eine Konfluenz von ca. 70 % nicht überschritten (b und c). Morphologisch veränderte Klone wurden verworfen.

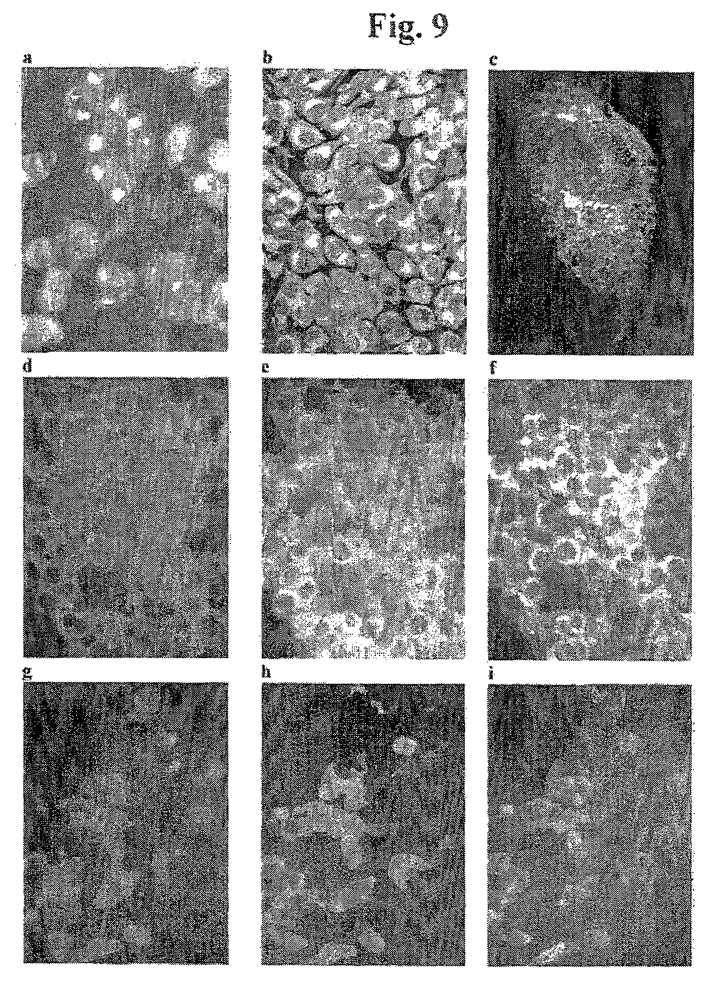

Figur 9 zeigt eine *In situ*-Immunfluoreszenz-Aufnahme.
   a) Kontrollmischung: V79MZh2D6*1-S-Zellen sind intensiv gefärbt, parentale V79MZ-Zellen nicht.
   b) Homogener Klon V79MZh2D6*2
   c) Konfokale Laser-Scanning-Mikroskopie zum Nachweis der Lokalisation des hCYP2D6 im endoplasmatischen Retikulum.
   d-f) Doppelfärbung des homogenen Klones V79MZh2D6*1-hOR: Die hCYPOR ist rot gefärbt (d), das hCYP2D6 1 grün (e). Bei Doppelbelichtung des Filmes überlagern sich die Färbungen zu gelb (f).
   g-h) Kontrollmischung zur Doppel-färbung: V79MZh2D6*1-hOR-Zellen erscheinen in der Doppelbelichtung (i) gelb, V79MZh2D6*1-Zellen rot und V79MZhOR-Zellen grün.
Figur 10 zeigt einen Acetonitril-Gradienten zur Trennung von Tamoxifen und seiner Metabolite. Als Laufmittel A wurde Wasser, 1 % Essigsäure und als Laufmittel B Acetonitril, 1 % Essigsäure verwendet.
Figur 11 zeigt einen Western-Blot der erfindungsgemäßen V79MZh2D6-Zellinien. Aufgetragen wurden jeweils 5 µl Zellhomogenat, entsprechend 5 - 15 µg Zellprotein.
Figur 12: Solubilisierung mit Emulgen 913. A. CO-Differenzspektrum der Zellinie V79MZh2D6*1 nach Solubilisierung in Emulgen 913 im Vergleich zur Negativkontrolle V79MZmockneo. Das charakteristische Maximum bei 450 nm war durch die Solubilisierung um etwa 1,8 nm blauverschoben. B. Western-Blot zum Nachweis der quantitativen Solubilisierung des Cytochroms P450. Aufgetragen wurden je 5 µg Zellprotein des solubilisierten Zellhomogenates vor Zentrifuga-tion (H), des Solubilisates nach Zentrifugation (S) und des in einem entsprechenden Volumen resuspendierten Pellets (P).
Figur 13 zeigt CO-Differenzspektren der Zellinie V79MZh1A1, aufgezeichnet 0, 5, 15, 30 und 60 min nach Sättigung der Probe mit Kohlenmonoxid. Mit der Zeit wird Cytochrom P450 durch reaktive Sauerstoffspezies zu Cytochrom P420 degradiert.
Figur 14: Einfluß von Quinidin auf die Stabilität von hCYP2D6. A. CO-Differenzspektren der Zellinie V79MZh2D6*9 nach Zellaufzucht mit und ohne Quindin im Kulturmedium. B. Western-Blot von solubilisiertem Zellhomogenat vor der Zentrifugation. Vergleichend wurden jeweils 5 µg Zellprotein von Zellen, die ohne (-Q) und mit (+Q) Quindin im Kulturmedium aufgezogen wurden, aufgetragen.
Figur 15 zeigt die Hydroxylierung von Bufuralol. A: Strukturformel von Bufuralol. (+)-Bufuralol wird am C_{1'}, (-)-Bufuralol am C₄ hydroxyliert. B: HPLC-Profil nach Inkubation mit Homogenat der Zellinie V79MZh2D6*1 (links) im Vergleich zur Negativkontrolle V79MZmockneo (rechts). Die Retentionszeiten betrugen für Hydroxy-Bufuralol ca. 8 min und für Bufuralol ca. 22 min.
Figur 16 zeigt die Inhibition der Bufuralol-Hydroxylierung durch den hCYP2D6-spezifischen Inhibitor Quinidin am Beispiel von hCYP2D6 1.
Figur 17 zeigt die Kinetik der (+)-Bufuralol-1'-Hydroxylierung, gemessen mit Homogenat der Zellinien V79MZh2D6*1, *2, *9, *10 und *17.
Figur 18 zeigt die Strukturformeln des klinisch verabreichten Z-Tamoxifens (links) und seines Konfigurations-isomeren E-Tamoxifen (rechts). Die Position der 4-Hydroxylierung am Z-Tamoxifen ist angegeben.
Figur 19 zeigt ein HPLC/MSD-Profil nach Inkubation mit Homogenat der Zellinie V79MZh2D6*1 (links) im Vergleich zur Negativkontrolle V79MZmockneo (rechts). Die Retentionszeiten betrugen für E- bzw. Z-4-Hydroxy-Tamoxifen ca. 5,5 min bzw. 6,2 min, für Tamoxifen-1,2-Epoxid ca. 8,4 min und für Tamoxifen-*N*-Oxid etwa 10,4 min. Die Peaks bei 2,1 min und 6,8 min konnten nicht eindeutig zugeordnet werden, da entsprechende Standards nicht zur Verfügung standen.
Figur 20 zeigt die Abhängigkeit der hCYP2D6-katalysierten Tamoxifen-4-Hydroxylierung von der DMSO- und Tamoxifen-Konzentration. Es sind die Mittelwerte und Standardabweichungen aus drei unabhängigen Messungen (2,5 % DMSO) bzw. die Meßwerte einer einzelnen Meßreihe (10 % DMSO) angegeben.
Figur 21 zeigt eine Kinetik der hCYP2D6-katalysierten Tamoxifen-4-Hydroxylierung, gemessen mit Homogenat der Zellinien V79MZh2D6*1, *2, *9, *10 und *17 und 2,5 % DMSO als Lösungsvermittler. Der Anfangsbereich ist bis zur Löslichkeitsgrenze von Tamoxifen bei 50 µM linear. Die Steigung der Regressionsgeraden entspricht der Clᵢₙₜ. Es sind die Mittelwerte und Standardabweichungen aus drei unabhängigen Messungen angegeben.
Figur 22 zeigt HPLC/MSD-Profile nach Inkubation mit hCYP2C9*1-hOR- und hCYP3A4-hOR-"Supersomen" sowie Homogenat der Zellinie V79MZh2D6*1 im Vergleich zur Negativkontrolle V79MZmockneo.
Figur 23 zeigt einen schematischen Ausschnitt des Metabolismus von Z-Tamoxifen in humaner Leber. Die Tamoxifen-4-Hydroxylierungs-Reaktion ist horizontal hinterlegt, die verschiedenen untersuchten Metaboliten mit Modifikationen am Stickstoff sind vertikal hinterlegt. Verändert nach (IARC, 1996).
Figur 24 zeigt ein 3D-Homologie-Modell der Bindung von Bufuralol und Tamoxifen im aktiven Zentrum von hCYP2D6 nach Lewis (1998). Dargestellt sind die beiden Substrate, der atomare Sauerstoff, der auf das Substrat übertragen wird, die Wasserstoff-Brückenbindungen, die das Substrat im aktiven Zentrum stabilisieren und die charakteristische ionische Wechselwirkung zwischen dem protonierten Stickstoff des Substrates und dem Asp₃₀₁ bzw. Glu₂₁₆ des Enzyms.

### Beispiele:

Die nachstehenden Beispiele veranschaulichen die Erfindung, sind jedoch nicht begrenzend zu verstehen.

### Beispiel 1: Vektorkonstuktion zur stabilen Expression von hCYP2D6-cDNAs in V79 Chinesischen Hamsterzellen

Zur stabilen Expression von hCYP2D6-cDNA in V79 Chinesischen Hamsterzellen wurden erfindungsgemäß drei Strategien verfolgt:
- Kotransfektion des Expressionsvektors pSV450h2D6 mit dem Neomycin-Resistenz-Plasmid pSV2neo (Clontech Laboratories, Inc., Palo Alto, CA) und Selektion mit Geneticin 418 (Geneticin 418-Sulfat; Calbiochem-Novabiochem Corp., La Jolla, CA) nach Doehmer *et al*. (1988).
- Transfektion mit dem Plasmid pcDNA3.1Hygro(+)h2D6 und Selektion mit Hygromycin B (Boehringer Mannheim GmbH, Mannheim). Durch die Vereinigung der cDNA und der Hygromycin-Resistenz auf einem Vektor sollte die zur Transfektion benötigte DNA-Menge reduziert und der Einfluß auf die chromosomale Integrität nach der Transfektion untersucht werden. Außerdem sollte durch die direkte Kopplung von cDNA und Resistenzgen der Anteil positiver Klone nach der Transfektion erhöht werden.
- Kotransfektion des Expressionsvektors pSV450h2D6 mit dem Plasmid pRc/RSVhCYPOR (Schneider *et al*., 1996) und Selektion mit Geneticin 418 nach Schneider *et al.* (1996). Der Vektor pRc/RSVhCYPOR trägt ein Neomycin-Resistenzgen und eine hCYPOR-cDNA. Die Koexpression des hCYP2D6 mit der hCYPOR sollte zeigen, ob der endogene CYPOR-Gehalt in V79MZ-Zellen für die maximale hCYP2D6-Aktivität ausreichend ist.

Die cDNAs für die Allele *hCYP2D6*1* in pBluescript SK(+) (Stratagene, Heidelberg), *hCYP2D6*2* in pVL1393 (Invitrogen Corp., Carlsbad, CA), *hCYP2D6*9* in M13mp19 (Stratagene, Heidelberg) und *hCYP2D6*10A* in M13mp19 wurden freundlicherweise von Dr. U. M. Zanger (Dr. Margarete Fischer-Bosch-Institut, Stuttgart) zur Verfügung gestellt. Die Allele sind in Figur 1 gezeigt und können auch mittels Standardverfahren erhalten werden.

### A. Konstruktion von pSV450-Polylinker-Vektoren

Zur stabilen Transfektion von V79 Chinesischen Hamsterzellen wurde der Expressionsvektor pSV450 (Doehmer *et al*., 1988) eingesetzt. Um die Klonierung von cDNAs in diesen Vektor zu vereinfachen, wurden pSV450-Vektoren mit verschiedenen Polylinkern konstruiert. In einem ersten Schritt wurden mittels PCR (Saiki *et al.,* 1988) die Polylinker für die Vektoren pSV450HB und pSV450HK gemäß PCR#1 und für den Vektor pSV450HS mittels *touch down*-PCR gemäß PCR#2 generiert.

### • PCR#1

**Ansatz:** Primer: 1,4 µl 16298 (25 mM), 1,4 µl 16299 (25 mM); Template: 1 µl 16300 (40 µM); 1 µl dNTPs (20 mM); 0,4 µl *Taq* DNA Polymerase (5 U/µl, QIAGEN, Hilden); 2,5 µl PCR-Puffer (10x); ad 25 µl mit Wasser
**PCR-Gerät:** Gene Amp PCR System 2400 (Perkin Elmer, Norwalk CT., USA)
**Temperaturprogramm:** 2 min 48 °C, 1 min 72 °C (1x); 1 min 94 °C, 1 min 55 °C, 1 min 72 °C (30x); 10 min 72 °C (1x)
**Amplifikat:** 84 bp

### • PCR#2

**Ansatz:** Primer: 1,4 µl 19176 (25 mM), 1,4 µl 19177 (25 mM); Template: 1 µl 19183 (40 µM); 1 µl dNTPs (20 mM); 0,4 µl *Taq* DNA Polymerase (5 U/µl, QIAGEN, Hilden); 2,5 µl PCR-Puffer (10x); 5 µl Q-Lösung (5x); ad 25 µl mit Wasser
**PCR-Gerät:** Gene Amp PCR System 2400 (Perkin Elmer, Norwalk CT., USA)
**Temperaturprogramm:** 1 min 94 °C (1x); 1 min 94 °C, 1 min 62 °C → 52 °C, 2 min 72 °C (10x, Annealing-Temperatur absteigend in 1 °C-Schritten); 1 min 94 °C, 1 min 52 °C, 2 min 72 °C (35x); 10 min 72 °C (1x)
**Amplifikat:** 114 bp

### Sequenzen der Primer und Templates:

16298 : 5′-TAGACAAGCTTGGATCCATG-3′

16299 : 5′-GCTATAAGCTTAGATCTCGG-3′

16300 :5′-
TAGACAAGCTTGGATCCATGGTACCGAGCTCGAGTCGACTGCAGTTAACTC
TAGATCGATGCGGCCGAGATCTAAGCTTATAGC-3′

19176 : 5′-GCATTAAGCTTAAGTCGACC-3′

19177 : 5′-CCGTATGATCACTAGTAGATC-3′

19183 : 5′-
GCATTAAGCTTAAGTCGACCGGTACCGTACGCTAGCGAATTCCGGATATCG

ATGGCGCGCCGCGGCCGCTCGAGCTCTAGACGCGTGGATCCAGATCTACTAGTG
ATCATACGG-3′

Aus dem Ursprungsvektor pSV450r2B1 (Figur 2; Doehmer *et al*., 1988) wurde die rCYP2B1-cDNA mit *Hin*d III und *Bgl* II herausgeschnitten und der jeweilige Polylinker (PCR#1, verdaut mit *Hin*d III und *Bgl* II; PCR#1, verdaut mit *Hin*d III und *Bam*H I; PCR#2, verdaut mit *Hin*d III und *Bcl* I) hineinligiert. Die neuen Vektoren wurden als pSV450HB, pSV450HK und pSV450HS bezeichnet. Die Polylinker der Vektoren pSV450HB, pSV450HK und pSV450HS haben die nachstehend gezeigten Strukturen. Alle genannten Schnittstellen sind nur einmal im Vektor vorhanden.

**pSV450HB:** *Hind* III-*Kpn* I/*Asp* 718*-Sac* I/*Ecl* 136*-Xho* I/*Ava* I*-Sal* I*-Hpa* I*-Xba* I*-Cla* I*-Xma* III*-Bgl* II
**pSV450HK:** *Hin*d III*-Bgl* II*-Xma* III*-Cla* I*-Xba* I*-Hpa* I-*Sal* I*-Xho* I/*Ava* I-*Sac* I/*Ecl* 136-*Kpn* I/*Asp* 718
**pSV450HS:** *Hin*d III*-Afl* II-*Sal* I-*Age* I-*Kpn* I/*Asp* 718-*Bsp*M II-*Eco*R V-*Cla* I-*Bss*H I*-Asc* I-*Sac* II*-Xma* III-*Not* I*-Xho* I/*Ava* I-*Sac* I/*Ecl* 136-*Xba* I*-Mlu* I*-Bsa*B I-*Bgl* II-*Spe* I

In Kombination mit der PCR, die es ermöglicht, kompatible Enden an die jeweilige cDNA anzufügen, ist damit in Zukunft die Konstruktion neuer pSV450-Expressionsvektoren ohne zeitaufwendige Zwischenklonierungen möglich. Um nach der Transfektion in V79MZ-Zellen eine optimale Translation der heterologen mRNA zu gewährleisten, sollte bei der Klonierung auf die Integrität der Kozak-Sequenz geachtet bzw. diese Sequenz optimiert werden (Kozak 1987; Kozak 1990).

### B. Konstruktion der Vektoren pSV450h2D6*1, *2, *9, *10 und *17

### pSV450h2D6*1, *9 und *10

Die cDNAs hCYP2D6*1 in pBluescript SK(+), hCYP2D6*9 in M13mp19 und hCYP2D6*10A in M13mp19 wurden mit *Bam*H I und *Eco*R I aus ihren Ursprungsvektoren herausgeschnitten und in den zuvor ebenfalls mit *Bam*H I und *Eco*R I verdauten Vektor pIC19H (ATCC, Manassas, VA) zwischenkloniert. Die neuen Vektoren wurden mit pICh2D6*1, *9 und *10 bezeichnet. Durch Restriktion dieser Vektoren mit *Hin*d III und *Bgl* II wurden cDNAs mit kompatiblen Enden zur Ligation in den Expressionsvektor pSV450 erzeugt. Die neuen Expressionsvektoren wurden pSV450h2D6*1, *9 und *10 genannt.

Nach Transformation von *E. coli* mit pSV450-cDNA-Vektoren erhaltene Klone wurden erfindungsgemäß zur Kontrolle der erfolgreichen Ligation der cDNA in den pSV450-Vektor mit einem Zahnstocher gepickt, in 10 µl sterilem Wasser resuspendiert und folgender PCR unterworfen:

### • PCR#4: Kontroll-PCR für die erfolgreiche Ligation einer cDNA in die Vektoren pSV450, pSV450HB, pSV450HK und pSV450HS

**Ansatz:** Primer: 1,4 µl 20261 (25 mM), 1,4 µl 20262 (25 mM); Template: 2 µl E. *coli*-Suspension; 1 µl dNTPs (20 mM); 0,2 µl *Taq* DNA Polymerase (5 U/µl, QIAGEN, Hilden); 2,5 µl PCR-Puffer (10x); ad 25 µl mit Wasser; 1 Tropfen Silikonöl
**PCR-Gerät:** Gene Amp PCR System 2400 (Perkin Elmer, Norwalk CT., USA)
**Temperaturprogramm:** 3 min 94 °C (1x); 0,5 min 94 °C, 1 min 56 °C, 2 min 72 °C (30x); 10 min 72 °C (1x)
**Amplifikat:** Enthält der Vektor pSV450 ein Insert zwischen den Schnittstellen für *Hin*d III und *Bgl* II, beträgt die Länge des Amplifikates 72 bp + Insert-bp. Ist kein Insert enthalten, wird ein 72 bp langes Fragment amplifiziert. Bei den Vektoren pSV450HB, pSV450HK und pSV450HS mit Polylinker ist der "72-bp-Abschnitt" entsprechend länger.

### Sequenzen der Primer:

20261 : 5′-TATTCCAGAAGTAGTGAGG-3′

20262 : 5′-ATCACCGAGCTGAGAAGC-3′

### pSV450h2D6*2

Die hCYP2D6*2-cDNA wurde mit *Hin*d III und *Kpn* I aus dem Vektor pVL1393 (Invitrogen Corp., Carlsbad, CA) herausgeschnitten und in den zuvor ebenfalls mit *Hin*d III und *Kpn* I verdauten Vektor pICh2D6*10 zwischenkloniert. Die cDNA wurde mit *Hind* III und *Bgl* II aus dem neuen Vektor pICh2D6*2 wieder herausgeschnitten und in den Expressionsvektor pSV450 (Doehmer *et al*., 1988) kloniert. Dieser Vektor wurde als pSV450h2D6*2 bezeichnet.

### pSV450h2D6*17

Allel *hCYP2D6*17* unterscheidet sich von Allel *hCYP2D6*2* allein in der Mutation C₁₁₁₁T. Diese liegt unmittelbar stromaufwärts einer Schnittstelle für *Xho* II (Figur 1). Daher konnte die cDNA für hCYP2D6 17 durch positionsgerichtete Punktmutagenese aus der hCYP2D6*2-cDNA erhalten werden: Es wurde ein 362 bp langes cDNA-Fragment mit der Mutation C₁₁₁₁T gemäß PCR#3 synthetisiert und mit *Hin*d III und *Xho* II verdaut.

### • PCR#3

**Ansatz:** Primer: 1,4 µl 16094 (25 mM), 1,4 µl 16095 (25 mM); Template: 1 µl pSV450h2D6*1 (15,5 ng/µl); 1 µl dNTPs (20 mM); 0,125 µl Red Hot DNA Polymerase (5 U/µl, Advanced Biotechnologies Ltd., Surrey, England); 2,5 µl Reaktionspuffer IV (10x); 1,5 µl Magnesiumchlorid (25 mM); ad 25 µl mit Wasser; 1 Tropfen Silikonöl
**PCR-Gerät:** Genius (Techne Ltd., Duxford Cambridge, England)
**Temperaturprogramm:** 1 min 94 °C (1x); 1 min 94 °C, 2 min 55 °C, 3 min 72 °C (30x); 10 min 72 °C (1x)
**Amplifikat:** 362 bp

### Sequenzen der Primer:

16094 : 5′-AGACGTGAAGCTTGCCGCCACCATGGGGCTA-3′

16095 : 5′-CAGGACGTAGAATGGATCTGGATGATGGGCAC-3′

Das zweite cDNA-Fragment wurde mit *Xho* II und *Bgl* II aus dem Plasmid pICh2D6*2 gewonnen. In einer 3fach-Ligation wurden beide cDNA-Fragmente zugleich in den mit *Hin*d III und *Bgl* II geschnittenen Expressionsvektor pSV450 kloniert. Der neue Vektor wurde pSV450h2D6*17 genannt.

### C. Konstruktion der Vektoren pcDNA3.1Hygro(+)h2D6*1, *2, *9,*10 und *17

Die cDNAs hCYP2D6*1, *2, *9, *10 und *17 wurden mit *Hin*d III und *Bgl* II aus dem jeweiligen Plasmid pSV450h2D6 herausgeschnitten und in den mit *Hin*d III und *Bam*H I verdauten Expressionsvektor pcDNA3.1Hygro(+) (Invitrogen Corp., Carlsbad, CA) kloniert. Die neuen Vektoren wurden pcDNA3.1Hygro(+)h2D6*1, *2, *9, *10 und *17 genannt.

Nach Transformation von *E. coli* mit pcDNA3.1Hygro(+)-cDNA-Vektoren erhaltene Klone wurden zur Kontrolle der erfolgreichen Ligation der cDNA in den pcDNA3.1Hygro(+)-Vektor mit einem Zahnstocher gepickt, in 10 µl sterilem Wasser resuspendiert und folgender PCR unterworfen:

### • PCR#5: Kontroll-PCR für die erfolgreiche Ligation einer cDNA in den Vektor pcDNA 3.1Hygro(+)

**Ansatz:** Primer: 1,4 µl 18383 (25 mM), 1,4 µl 18384 (25 mM); Template: 2 µl *E. coli*-Suspension; 1 µl dNTPs (20 mM); 0,2 µl *Taq* DNA Polymerase (5 U/µl, QIAGEN, Hilden); 2,5 µl PCR-Puffer (10x); ad 25 µl mit Wasser
**PCR-Gerät**: Gene Amp PCR System 2400 (Perkin Elmer, Norwalk CT., USA)
**Temperaturprogramm:** 3 min 94 °C (1x); 0,5 min 94 °C, 1 min 56 °C, 2 min 72 °C (30x); 10 min 72 °C (1x)
**Amplifikat:** Enthält der Vektor pcDNA3.1Hygro(+) ein Insert, beträgt die Länge des Amplifikates knapp 200 bp + Insert-bp. Ist kein Insert enthalten, wird ein 203 bp langes Fragment amplifiziert.

### Sequenzen der Primer:

18383 : 5′-CACTGCTTACTGGCTTATCG-3′

18384 : 5′-ACTAGAAGGCACAGTCGAGG-3′

### Beispiel 2: Transfektion

Parentale V79MZ-Zellen wurden erfindungsgemäß durch Kalziumphosphat-Kopräzipitation mit rekombinanten Expressionsvektoren transfiziert (Graham und Van der Eb, 1973; Parker und Stark, 1979).

V79MZ-Zellen wurden erfindungsgemäß bei 37 °C, 7 % CO₂ und 90 % Luftfeuchtigkeit in speziell beschichteten Gewebekulturgefäßen (94/16 mm- oder 145/20 mm-Gewebekulturschalen und 50 ml- oder 250 ml-Gewebekulturflaschen von der Greiner GmbH (Frickenhausen); 24er- und 96er-Gewebekulturmikrotiterplatten von Nunc Inc. (Naperville, IL)) in DMEM-Kulturmedium mit erhöhtem Glukosegehalt (4,5 g/l) kultiviert. Zusätzlich wurde das DMEM-Kulturmedium mit 1 mM Natriumpyruvat, 4 mM L-Glutamin, 10 % FCS, 100 U/ml Penicillin und 100 µg/ml Streptomycin supplementiert ("Komplettmedium"). Nach Transfektion wurden Geneticin 418- oder Hygromycin B-resistente V79MZ-Zellklone mit 0,5 mg Geneticin 418/ml-Komplettmedium bzw. 0,4 mg Hygromycin B/ml-Komplettmedium kultiviert.

Für eine Transfektion wurden zu den verwendeten DNA-Mengen jeweils 500 µl HEPES (Gibco BRL, Eggenstein)-gepufferte Salzlösung (137 mM Natriumchlorid, 6 mM Dextrose, 5 mM Kaliumchlorid, 0,7 mM Natriumhydrogenphosphat, 20 mM HEPES, pH 7,0) pipettiert. Durch Zugabe von 26 µl 2,5 M Kalziumchloridlösung und 30minütige Inkubation bei Raumtemperatur wurde die DNA an Kalziumphosphat kopräzipitiert. Das Präzipitat wurde tropfenweise zu einer Kultur von 1,5 - 2 x 10⁶ parentalen V79MZ-Zellen in einer 145/20 mm-Gewebekulturschale gegeben. Nach sorgfältigem Mischen und 4stündiger Inkubation bei 37 °C wurden die Zellen für 2 min mit 15 % (v/v) Glycerin in Komplettmedium inkubiert, um die Effizienz der DNA-Aufnahme zu steigern. Anschließend wurde das Glycerin durch Absaugen des Mediums und zweimaliges Waschen mit je 7 ml Komplettmedium entfernt. Da die Zellen zur stabilen Integration der Fremd-DNA in ihr Genom den Zellzyklus durchlaufen müssen, wurden sie zunächst für etwa 36 h in Komplettmedium ohne G418 oder Hygromycin B kultiviert. Daraufhin wurden die Zellen vorsichtig trypsiniert, in 1 mg G418/ml-Komplettmedium bzw. 0,4 mg Hygromycin B/ml-Komplettmedium aufgenommen und auf drei 96er-Gewebekulturmikrotiterplatten verteilt.

Nur Zellen, die bei der Transfektion mit den Vektoren ein Resistenzgen gegen G418 bzw. Hygromycin B aufgenommen hatten, konnten überleben (Mulligan und Berg, 1981). Nach 10 - 14 Tagen waren resistente Zellklone erkennbar. Einzelne Klone wurden direkt in der Kammer trypsiniert und die Hälfte der Zellen zur *In situ*-Immunfluoreszenz entnommen.

Die Klonalität und Stabilität wurde durch wiederholtes Subklonieren und Passagieren (ein Großteil der trypsinierten Zellen wurde verworfen oder in neue Kulturgefäße umgesetzt) der neuen Zellinien sowie durch mehrmalige *In situ*-Immunfluoreszenz und Bestimmung der enzymatischen Aktivität sichergestellt.

Die unterschiedlichen erfindungsgemäß durchgeführten Transfektionsansätze T1 - T14 und die Zellinien sind in Tabelle 2 zusammengestellt.

### • Konstruktion der Zellinien V79MZh2D6*1, *2, *9, *10 und *17:

30 µg *Sca* I-linearisierte pSV450h2D6*1-, *2-, *9-, *10- oder *17- und 1 µg EcoR I-linearisierte pSV2neo-DNA wurden transfiziert. Bei Transfektion des Resistenzvektors pSV2neo wurde der Expressionsvektor pSV450h2D6 im 30fachen Überschuß eingesetzt, um die Wahrscheinlichkeit für einen Geneticin 418-resistenten und zugleich hCYP2D6-exprimierenden Klon zu erhöhen (T1 - T5). Je Ansatz wurden 30 - 50 resistente Klone erhalten. Im Mittel exprimierte einer von 50 Klonen homogen hCYP2D6, etwa die Hälfte der Klone war in der *in situ*-Immunfluoreszenz heterogen, die restlichen exprimierten kein hCYP2D6. Die Zellinien V79MZh2D6*1, V79MZh2D6*2, V79MZh2D6*9, V79MZh2D6*10 und V79MZh2D6*17 wurden am 15.02.2000 bei der DSMZ-Deutsche Sammlung von Mikroorganismen und Zellkulturen GmbH unter den Zugangsnummem DSM ACC2446, DSM ACC2447, DSM ACC2448, DSM ACC2449 und DSM ACC2450 hinterlegt.

### • Konstruktion der Zellinien V79MZh2D6*1-H, *2-H, *9-H, *10-H und *17-H:

3 µg *Ssp* I-linearisierte pcDNA3.1Hygro(+)h2D6*1-, *2-, *9-, *10- oder *17-DNA wurden transfiziert. Die Kombination der cDNA und des Resistenzgenes auf einem einzigen Vektor ermöglichte eine Transfektion mit nur 3 µg DNA (T6 - T10). Je Ansatz wurden 10 - 30 resistente Klone erhalten. Im Mittel exprimierte einer von 10 Klonen homogen hCYP2D6, etwa zwei Drittel der Klone waren in der *in situ*-Immunfluoreszenz heterogen, die restlichen exprimierten kein hCYP2D6.
Gegenüber der Kotransfektion von pSV450h2D6 und pSV2neo konnte der Anteil homogen hCYP2D6-exprimierender V79MZ-Klone folglich 5fach gesteigert werden. Dies ist methodisch entscheidend, weil die Identifizierung homogen cDNA-exprimierender Klone der zeitlich limitierende Schritt bei der Konstruktion neuer Zellinien ist.

### • Konstruktion der Zellinie V79MZh2D6*1-hOR:

30 µg *Sca* I-linearisierte pSV450h2D6*1- und 1 µg *Sca* I-linearisierte pRc/RSV-hCYPOR-DNA wurden transfiziert.Die Koexpression von hCYP2D6 1 und hCYPOR (T11) sollte im Vergleich zu Zellinie V79MZh2D6*1 (T1) zeigen, ob der endogene CYPOR-Gehalt der V79MZ-Zellen für die maximale hCYP2D6-Aktivität ausreichend ist. Wie bei den Transfektionsansätzen T1 - T5 wurde der Expressionsvektor pSV450h2D6*1 gegenüber dem Resistenzvektor pRc/RSV-hCYPOR in 30fachem Überschuß eingesetzt. Bei diesem Ansatz wurden 34 resistente Klone erhalten, von denen einer sowohl hCYP2D6 1 als auch hCYPOR homogen exprimierte.

### • Konstruktion der scheintransfizierten Zellinien V79MZmockneo:

Folgende Tranfektionen wurden durchgeführt:
a) 30 µg *Eco*R I-linearisierte pSV2neo-DNA
b) 1 µg *Eco*R I-linearisierte pSV2neo-DNA
c) 1 µg *Eco*R I-linearisierte pSV2neo- und 30 µg *Sca* I-linearisierte pSV450HB-DNA

In Abhängigkeit von der Menge transfizierter DNA kann durch Rekombination die chromosomale Integrität der Empfängerzelle gestört und können chromosomale Aberrationen verursacht werden (Bradwell, 1989). Daher wurde im Rahmen der Konstruktion Schein-transfizierter V79MZ-Zellinien die Transfektion mit unterschiedlichen Mengen an DNA durchgeführt. Die daraus resultierenden Zellklone wurden karyotypisch charakterisiert.
Bei Transfektion mit nur 1 µg pSV2neo (T13) sowie 1 µg pSV2neo und 30 µg pSV450HB (T14) wurden etwa genauso viele Klone gezählt wie in den Ansätzen (T1 - T5). Die Zugabe von pSV450HB als "Träger-DNA" (Graham und Van der Eb, 1973; Strain und Wylie, 1984) hatte demnach keinen Einfluß auf die Anzahl an Geneticin 418-resistenten Klonen. Mit 30 µg pSV2neo-DNA wurden etwa 120 Klone erhalten. Eine 30fach höhere DNA-Menge führte also nur zu 3 - 4mal mehr Klonen.
Von 3 x 6 willkürlich ausgewählten Klonen wurde ein Klon mit einer veränderten Morphologie mikroskopisch identifiziert. Ein ähnlicher Anteil war auch bei den anderen Transfektionen beobachtet worden. Morphologisch veränderte Klone (Figur 8) wurden von weiteren Charakterisierungen ausgeschlossen und verworfen.
Die Zellinie V79MZmockneo130 wurde erfindungsgemäß neben parentalen V79-Zellen als Negativkontrolle verwendet. Im Folgenden wird sie als "V79MZmockneo" bezeichnet.

**Tabelle 2**

| **Übersicht der Transfektionsansätze und der neuen Zellinien.** | | | | | |
|---|---|---|---|---|---|
| | **Zellinie** | **Heterologe Expression** | **Res.** | **Expressions- Vektor (µg)** | **Resistenz-Vektor (µg)** |
| T1 | V79MZh2D6*1 | hCYP2D6 1 | G418 | pSV450h2D6*1 (30) | pSV2neo (1) |
| T2 | V79MZh2D6*2 | hCYP2D6 2 | G418 | pSV450h2D6*2 (30) | pSV2neo (1) |
| T3 | V79MZh2D6*9 | hCYP2D6 9 | G418 | pSV450h2D6*9 (30) | pSV2neo (1) |
| T4 | V79MZh2D6*10 | hCYP2D6 10 | G418 | pSV450h2D6*10 (30) | pSV2neo (1) |
| T5 | V79MZh2D6*17 | hCYP2D6 17 | G418 | pSV450h2D6*17 (30) | pSV2neo (1) |
| T6 | V79MZh2D6*1-H | hCYP2D6 1 | HyB | pcDNA3.1Hygro(+)h2D6*1 (3) | |
| T7 | V79MZh2D6*2-H | hCYP2D6 2 | HyB | pcDNA3.1Hygro(+)h2D6*2 (3) | |
| T8 | V79MZh2D6*9-H | hCYP2D6 9 | HyB | pcDNA3.1Hygro(+)h2D6*9 (3) | |
| T9 | V79MZh2D6*10-H | hCYP2D6 10 | HyB | pcDNA3.1Hygro(+)h2D6*10 (3) | |
| T10 | V79MZh2D6*17-H | hCYP2D6 17 | HyB | pcDNA3.1Hygro(+)h2D6*17 (3) | |
| T11 | V79MZh2D6*1-hOR | hCYP2D6 1 hCYPOR | G418 | pSV450h2D6*1 (30) | pRc/RSV -hCYPOR (1) |
| T12 | V79MZmockneo30 | | G418 | | pSV2neo (30) |
| T13 | V79MZmockneol | | G418 | | pSV2neo (1) |
| T14 | V79MZmockneo130 | | G418 | pSV450HB (30) | pSV2neo (1) |
| **Abkürzungen: Res, Resistenz; G418, Geneticin 418; HyB, Hygromycin B** | | | | | |

### Beispiel 3: Charakterisierung der neuen Zellinien

### In situ-Immunfluoreszenz

Zum Nachweis der heterologen Expression von hCYP2D6 und/oder hCYPOR wurden die nach Transfektion erhaltenen Klone mittels *In situ*-Immunfluoreszenz charakterisiert (Figur 9). Nur homogene Klone, bei denen alle Zellen gleichmäßig und intensiv gefärbt waren, wurden weiterkultiviert. Weitere Entscheidungskriterien waren eine strukturierte Färbung, die die subzelluläre Lokalisation von hCYP2D6 und hCYPOR im endoplasmatischen Retikulum zeigt (Figur 9c) und ein charakteristisch dunklerer Kernbereich.

Für eine *In situ*-Immunfluoreszenz wurden 10⁴ Zellen der zu testenden Klone auf Mikrokammer-Objektträgern (Nunc Inc., Naperville, IL) ausgesät und für 24 Std. kultiviert. Danach wurden die Kammern abgehoben und die auf dem Objektträger angehefteten Zellen mit PBS (Bio Whittaker, Verviers, Belgien) gewaschen, zur Fixierung mit eiskaltem Methanol/Aceton (1:1) für 7 min inkubiert und anschließend an der Luft getrocknet. Die derart fixierten Zellen wurden mit 150 µl primärer Antikörperlösung (polyklonales antihCYP2D6 Antiserum 637.2 aus Kaninchen, 1:200 verdünnt in Komplettmedium, freundlicherweise zur Verfügung gestellt von Dr. U. M. Zanger, Dr. Margarete Fischer-Bosch-Institut, Stuttgart) überschichtet, mit Polyethylenfolie abgedeckt und für 90 min bei Raumtemperatur inkubiert. Anschließend wurde dreimal 10 min mit PBS gewaschen, 150 µl sekundäre Antikörperlösung (FITC-gekoppelter anti-Kaninchen IgG Antikörper aus Ziege, 1,5 mg/ml, 1:125 verdünnt in Komplettmedium, Dianova, Hamburg) aufgebracht, mit Polyethylenfolie abgedeckt und für 1 Std. bei Raumtemperatur im Dunkeln inkubiert. Nach dreimaligem Waschen in PBS für je 10 min wurden 100 µl "*Antifading*"-Reagenz (100 mg p-Phenylendiammoniumdichlorid in 10 ml PBS und 80 ml Glycerin) aufgetragen und blasenfrei eingedeckelt. Die Auswertung erfolgte am Fluoreszenzmikroskop (Axioplan, Carl Zeiss, Oberkochen) mit einem Standardfiltersatz im Anregungsbereich von 450 - 490 nm. Zur Demonstration der subzellulären endoplasmatischen Lokalisation des Cytochroms P450 wurden am Laser-Scanning-Mikroskop LSM 4.10 (Carl Zeiss, Oberkochen) unter Wasserimmersion konfokale Schnitte aufgenommen. Die Fluoreszenz wurde bei 488 nm angeregt und die Emission bei 515 - 565 nm detektiert. Das exprimierte Cytochrom war entsprechend grün gefärbt.
Zum Nachweis der Koexpression von hCYP2D6 und hCYPOR durch Doppelfärbung wurde zur primären Antikörperlösung zusätzlich anti-hCYPOR Antikörper aus Ziege gegeben (Endverdünnung 1:500 in Komplettmedium). Um Kreuzreaktionen auszuschließen, wurde als sekundäre Antikörperlösung ein Gemisch aus TRITC-gekoppeltem anti-Kaninchen IgG Antikörper aus der Maus (1,5 mg/ml, 1:125 verdünnt in Komplettmedium, Pierce, Rockford, IL) und FITC-gekoppeltem anti-Ziegen IgG Antikörper ebenfalls aus der Maus (1,5 mg/ml, 1:125 verdünnt in Komplettmedium, Sigma, Deisenhofen) verwendet. Danach war bei Doppelfärbung die Oxidoreduktase grün, das Cytochrom P450 rot gefärbt.

### Auswahl repräsentativer Klone

In der *In situ*-Immunfluoreszenz wurden je Zellinie 1 - 6 hinsichtlich cDNA-Expression homogene Klone identifiziert. Für eine detaillierte Charakterisierung und spätere Anwendung wurde aus diesen ein repräsentativer Klon je Transfektionsansatz ausgewählt. Positive Auswahlkriterien waren eine gegenüber der parentalen Zellinie V79MZ unveränderte Morphologie und eine vergleichbare Verdopplungszeit. Schließlich wurden solche Klone bevorzugt, die eine durchschnittliche hCYP2D6-Aktivität besaßen. Dazu wurde von allen Klonen die spezifische (+/-)-Bufuralol-Hydroxylierung gemessen.
Es wurden Klone mit durchschnittlicher hCYP2D6-Aktivität ausgewählt, um Verzerrungen in den Allel-spezifischen Aktivitäten durch den Integrationsort der cDNA oder eine (seltene) Mehrfachintegration zu minimieren; denn im Gegensatz zur homologen Rekombination ist der Integrationsort der cDNA im V79MZ-Genom in gewissen Grenzen zufällig (Schulz *et al*., 1987). Sogenannte "Chromatineffekte" und benachbarte Sequenzabschnitte können die Transkription der integrierten cDNA beeinflussen (Butner und Lo, 1986; Jaenisch und Jahner, 1984; Wahl *et al*., 1984). Daher ist die Menge an heterolog exprimiertem hCYP2D6 und somit die enzymatische Aktivität pro mg Zellprotein vom Integrationsort der cDNA abhängig.
Folgerichtig unterschieden sich die Bufuralol-Hydroxylase-Aktivitäten verschiedener Klone einer Zellinie bis um das 3fache. Im Falle der Transfektionsansätze T2 (Zellinie V79MZh2D6*2) und T11 (Zellinie V79MZh2D6*1-hOR) war die Auswahl anhand der durchschnittlichen Aktivität nicht möglich, weil jeweils nur ein einziger Klon alle übrigen Kriterien erfüllte. Die ausgewählten Klone sind mit den neuen Zellinien identisch und nur sie wurden eingehend charakterisiert.

### Nachweis der genomisch integrierten cDNAs

Zum Nachweis der genomischen Integration der transfizierten cDNAs wurde aus den Zellinien V79MZh2D6*1, *2, *9, *10 und *17 (am 15.02.2000 bei der DSMZ-Deutsche Sammlung von Mikroorganismen und Zellkulturen GmbH unter den Zugangsnummern DSM ACC2446, DSM ACC2447, DSM ACC2448, DSM ACC2449 und DSM ACC2450 hinterlegt) die genomische DNA isoliert, gemäß PCR#6 mittels *touch down*-PCR die hCYP2D6-Expressionskassette als 2198 bzw. 2299 bp langes Amplifikat amplifiziert und gelelektrophoretisch nachgewiesen.

Zur Isolierung genomischer DNA aus V79MZ-Zellen wurden V79MZh2D6-Zellen auf 94/16 mm-Gewebekulturschalen bis zu knapp 100 %iger Konfluenz kultiviert, das Medium abgenommen und zweimal mit 5 ml PBS gewaschen. Daraufhin wurde die genomische DNA mit dem QIAamp Blood Midi Kit der Firma QIAGEN (Hilden) gemäß dem Herstellerprotokoll isoliert.

### • PCR#6

**Ansatz:** Primer: 1,4 µl 16014 (25 mM), 1,4 µl 15889 (25 mM); Template: 1 µl genomische DNA (ca. 0,2 µg/µl); 1 µl dNTPs (20 mM); 0,5 µl *Taq* DNA Polymerase (5 U/µl, QIAGEN, Hilden); 2,5 µl PCR-Puffer (10x); 5 µl Q-Lösung (5x); ad 25 µl mit Wasser
**PCR-Gerät:** Gene Amp PCR System 2400 (Perkin Elmer, Norwalk CT., USA)
**Temperaturprogramm:** 1 min 94 °C (1x); 1 min 94 °C, 1 min 62 °C → 52 °C, 3 min 72 °C (10x, Annealingtemperatur absteigend in 1 °C-Schritten); 1 min 94 °C, 1 min 52 °C, 3 min 72 °C (35x); 10 min 72 °C (1x)
**Amplifikat:** Es wird je nach inserierter hCYP2D6-cDNA ein etwa 2198 bzw. 2299 bp langes Amplifikat erhalten. Ohne Insert (Scheintransfektion) ist das Amplifikat je nach integriertem pSV450-Vektor etwa 700 bp lang.

### Sequenzen der Primer:

16014 : 5′-CAGAGGTTTTCACCGTCATC-3′

15889 : 5′-GGAATGTCCTCTCAAGTAGA-3′

Um die Identität der fünf Allele zu verifizieren, wurden die Amplifikate mit den Primern 16094 (5'-AGACGTGAAGCTTGCCGCCACCATGGGGCTA-3'), 15887 (5'-AGCTGGATGAGCTGCTAA-3') und 15888 (5'-ATCACCAACCTGTCATCGG-3') sequenziert. Damit wurde zugleich die Integrität der transfizierten DNA demonstriert, die bis zu ihrer Integration in die genomische DNA extrem anfällig für Mutationen, insbesondere Deletionen, ist (Bradwell, 1989; Calos *et al*., 1983).

### Nachweis der hCYP2D6-mRNA

Die Transkription der integrierten hCYP2D6-cDNAs wurde auf mRNA-Ebene mittels RT-PCR#1 verifiziert.

Zur Isolierung der Gesamt-RNA aus V79MZ-Zellen wurden V79MZh2D6-Zellen auf 94/16 mm-Gewebekulturschalen bis zu etwa 90 %iger Konfluenz kultiviert, das Medium abgenommen und zweimal mit 5 ml PBS gewaschen. Daraufhin wurde die Gesamt-RNA mit 1,5 ml peqGOLD TriFast™-Lösung (peqLab Biotechnologie GmbH, Erlangen) entsprechend dem Herstellerprotokoll isoliert.

### • RT-PCR#1

Es wurde mit dem Access RT-PCR System (Promega Corp., Madison, WI) gearbeitet:

**Ansatz:** Primer: 2 µl 16093 (25 mM), 2 µl 17606 (25 mM); Template: 0,5 µl isolierte Gesamt-RNA (1-2 µg/µl); 1 µl dNTPs (10 mM); AMV Reverse Transkriptase (5 U/µl); 1 µl *Tfl* DNA Polymerase (5 U/µl); 10 µl AMV/*Tfl* Reaktionspuffer (5x); 2 µl Magnesiumsulfat (25 mM); ad 50 µl mit DEPC (Sigma, Deisenhofen)-behandeltem Wasser
**PCR-Gerät:** Uno-Thermoblock (Biometra biomedizinische Analytik GmbH, Göttingen)
**Temperaturprogramm:** 50 min 48 °C (1x); 2 min 94 °C (1x); 1 min 94 °C, 1 min 60 °C, 2 min 70 °C (40x); 10 min 70 °C (1x)
**Amplifikat:** Es wird ein 410 bp Fragment der revers transkribierten hCYP2D6-mRNA amplifiziert.

### Sequenzen der Primer:

16093 : 5'-CATACTGCTTCGACCAGTTGCG-3'

17606 : 5′-GCAGGTGAGGGAGGCGATCAC-3′

### Western-Blot

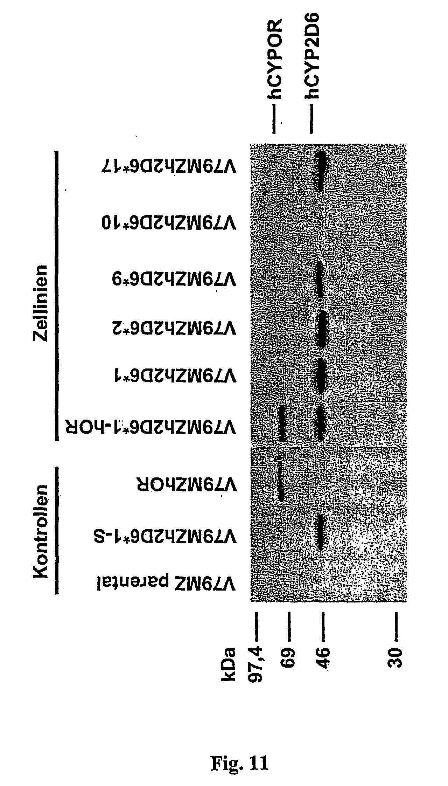

Nachdem die homogene Expression von hCYP2D6 in den erfindungsgemäßen V79MZh2D6-Zellinien über *in situ-*Immunfluoreszenz nachgewiesen worden war, wurde durch Western-Blotting das Molekulargewicht des heterolog exprimierten hCYP2D6 überprüft und ein qualitativer Hinweis auf die relativen hCYP2D6-Gehalte erhalten (Figur 11).

Hierzu wurden die in einer SDS-PAGE (Laemmli, 1970) aufgetrennten Proteine eines Zellhomogenats (vgl. Beispiel 4) 30 min lang im "*semi-dry*"-Verfahren bei 225 mA in einer *"Semi Dry* Elektroblotter"-Apparatur (Sartorius, Göttingen) aus dem Polyacrylamidgel auf eine Immobilon-P-Membran (Millipore, Dreieich) geblottet (Burnette, 1981). Für den Transfer wurden ausgehend von der Graphit-Anode zwei mit Puffer C (0,03 M Tris, 20 % Methanol, 0,04 M 6-Aminohexansäure, ad pH 10 mit Natronlauge) durchtränkte Whatman 3 MM-Papiere, das zuvor 15 min in Puffer C eingelegte Gel, die zuvor mit Methanol benetzte und daraufhin 10 min in Puffer B (0,03 M Tris, 20 % Methanol, ad pH 10 mit Natronlauge) eingelegte Membran, zwei mit Puffer B durchtränkte Whatman 3 MM Papiere und zwei mit Puffer A (0,3 M Tris, 20 % Methanol, ad pH 10 mit Natronlauge) durchtränkte Whatman 3 MM Papiere blasenfrei aufeinandergelegt und hierauf die Graphitkathode fixiert.
Zur Immunodetektion des hCYP2D6 wurde die geblottete Membran über Nacht in PBS, 7 % Magermilchpulver (Marke "Glücksklee", Nestlé Deutschland AG, Frankfurt) bei 4 °C geblockt, kurz mit PBS gewaschen und für 1 Std. in primärer Antikörperlösung (polyklonales anti-hCYP2D6 Antiserum 637.2 aus Kaninchen, 1:100 verdünnt in PBS) geschwenkt. Anschließend wurde dreimal 15 min in PBS, 0,5 % Tween20 gewaschen, 30 min in sekundärer Antikörperlösung (POD-gekoppelter anti-Kaninchen IgG aus der Ziege, 0,2 U/ml, 1:10000 verdünnt in PBS, Boehringer Mannheim, Mannheim) inkubiert und wieder dreimal 15 min in PBS, 0,5 % Tween20 gewaschen. Das hCYP2D6 wurde daraufhin über den gebundenen sekundären Antikörper mit dem ECL-Kit (*Enhanced* Chemilumineszenz, Amersham, Little Chalfont, England) nachgewiesen. Dabei wird die Lichtemission während der Peroxidase-katalysierten Oxidation von Luminol in Gegenwart von Wasserstoffperoxid mit Hyperfilm ECL (Amersham, Little Chalfont, England) detektiert.

Die experimentellen Molekulargewichte für hCYP2D6 von 56,0 ± 0,6 kDa und für hCYPOR von 80,3 ± 2,8 kDa stimmten gut mit den rechnerischen Werten von 55,8 kDa und 76,7 kDa überein. Auffallend war der relativ sehr geringe Gehalt an hCYP2D6 10; vgl. Figur 11.

### Beispiel 4: CO-Differenzspektren

Um die unterschiedlichen hCYP2D6 1-exprimierenden Zellinien V79MZh2D6*1, -hOR, -H und -S sowie die verschiedenen allelen Varianten vergleichen zu können, wurde der Cytochrom P450-Gehalt mittels CO-Differenzspektren bestimmt.

Für Herstellung von Zellhomogenat wurden V79MZ-Zellen in mindestens drei 250 ml-Gewebekulturflaschen bis zu 80 - 100 %iger Konfluenz kultiviert. Die trypsinierten Zellen wurden vereinigt, gleichmäßig auf je drei 145/20 mm-Gewebekulturschalen pro 250 ml-Gewebekulturflasche verteilt und in 20 ml Komplettmedium ohne G418 oder Hygromycin B bis zu 90 %iger Konfluenz inkubiert. Das Medium wurde verworfen und zweimal mit 5 ml eisgekühltem Puffer (100 mM Kaliumphosphat, pH 7,4) nachgespült. Die Zellen wurden in 4 ml eisgekühltem Puffer mit einem Gummischaber von der Platte abgelöst, vereinigt und durch 10minütige Zentrifugation bei 1500 x g und 4 °C pelletiert. Der Überstand wurde vollständig abgenommen, das Pellet in 1 ml Puffer pro neun 145/20 mm-Gewebekulturschalen sorgfältig resuspendiert und wie folgt aliquotiert: Zur Aufnahme von CO-Differenzspektren wurde 1 ml der Zellsuspension abgenommen (1 ml-Aliquot), der Rest 1:1 mit Puffer verdünnt, resuspendiert und zur Bestimmung des Proteingehalts, der enzymatischen Aktivität, für enzymkinetische Messungen und für Westem-Blots in 100 - 300 µl-Einheiten aufgeteilt (verdünnte Aliquots). Alle Aliquots wurden in flüssigem Stickstoff schockgefroren, um die Zellen aufzubrechen, und anschließend bis zum Gebrauch bei - 80 °C aufbewahrt.

Ein 1 ml-Aliquot Zellhomogenat wurde auf Eis aufgetaut und nach Zugabe von 20 µl 100 mM PMSF (Boehringer Mannheim GmbH, Mannheim) in Isopropanol in 1 ml Solubilisierungspuffer (100 mM Natriumhydrogenphosphat, pH 7,4, 10 % (v/v) Glycerin, 0,5 % (w/v) Emulgen 913 (Kao-Atlas, Tokyo, Japan) mit einer Pipette sorgfältig resuspendiert. Bei der Messung von hCYP2D6-Spektren wurden außerdem 10 µl 2 mM Quinidin (Hydrochlorid; Sigma, Deisenhofen) zugegeben. Das membrangebundene Cytochrom wurde durch 15minütiges leichtes Rühren auf Eis solubilisiert und unlösliches Material zur Reduzierung der Trübung durch Zentrifugation für 10 min bei 17000 x g und 4 °C pelletiert (Evert *et al.,* 1997; Tyndale *et al*., 1991a). Der Überstand wurde in einen 2 ml Glas-Glas-Homogenisator überführt, mit einigen Kristallen Natriumdithionit (Sigma, Deisenhofen) und 15 Hüben reduziert und auf zwei Quartzglasküvetten aufgeteilt. Nachdem am Aminco DW-2000 UV/VIS-Spektrophotometer (SLM Instruments Inc., Urbana, IL) das reduzierte Spektrum zwischen 400 und 500 nm aufgezeichnet worden war, wurde die Lösung in der Probenküvette mit etwa 60 Blasen Kohlenmonoxid gesättigt und sofort das CO/reduzierte Spektrum gemessen (Estabrook *et al*., 1972). Aus beiden Spektren wurde das CO/reduzierte *versus* reduzierte Spektrum (CO-Differenzspektrum) berechnet und hieraus anhand der Extinktionskoeffizienten von 91 mM⁻¹cm⁻¹ und 110 mM⁻¹cm⁻¹ (Omura und Sato, 1964b) die Cytochrom P450- und Cytochrom P420-Konzentration abgeleitet.

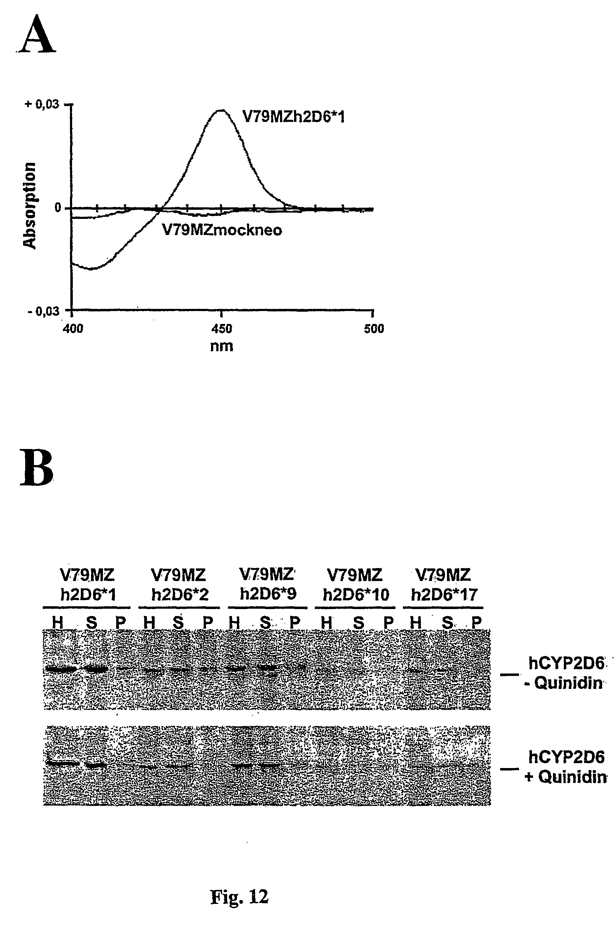

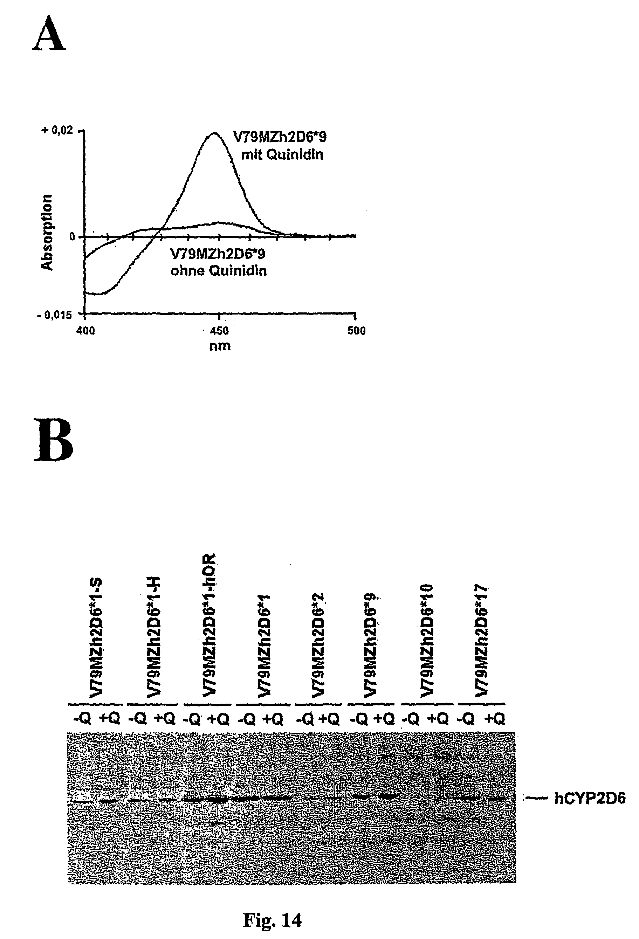

Ohne Solubiliserung und Zentrifugation war das Zellhomogenat für die Messung von CO-Spektren zu stark getrübt. Nach Zentrifugation ohne vorherige Solubilisierung war alles Cytochrom P450 im Pellet enthalten. Bei Anwendung des erfindungsgemäßen Verfahrens mit dem nicht-ionischen Detergenz Emulgen 913 wurde das Cytochrom P450 jedoch nahezu vollständig solubilisiert (Figur 12B). Nach Zentrifugation zur Entfernung der Trübung war es im Solubilisat gut meßbar. Optimal war eine Endkonzentration von 0,25 % Emulgen 913. Auch durch wiederholte Behandlung des resuspendierten Pellets mit Emulgen 913 ließ sich kein weiteres Cytochrom P450 solubilisieren. Die Solubilisierung war unabhängig von der Häm-Inkorporation, wie insbesondere die Solubilisierung von hCYP2D6 9 nach Zellaufzucht mit und ohne Quinidin zeigte (Figuren 12B und 14A).

Dahingegen wurden bei Expression in Baculovirus-infizierten Insektenzellen mit Emulgen 911 maximal etwa 50 % des hCYP2D6 1 solubilisiert (Evert *et al*., 1997; Paine *et al*., 1996). Die Variante hCYP2D6 7, die aufgrund der Mutation His₃₂₄Pro kein Häm inkorporieren kann und daher nicht-funktional ist, wurde praktisch nicht solubilisiert (Evert *et al*., 1997). Eine Abhängigkeit von exogenem Häm bestand in beiden Fällen ebenfalls nicht. Im Gegensatz dazu wurde für die Solubilisierbarkeit von Baculovirus-exprimiertem bCYPc17 eine deutliche Abhängigkeit von der Häm-Inkorporation beschrieben (Barnes *et al*., 1994). Folglich müssen außer der Assoziation mit Häm weitere Faktoren die Solubilisierbarkeit beeinflussen, beispielsweise die intrazelluläre Lokalisation des heterolog exprimierten Cytochroms P450, seine Aggregationstendenz, die Zusammensetzung der intrazellulären Membransysteme des Expressionssystems oder die Art der Bindung des Cytochroms P450 in der Membran.

### Aerobe Messung

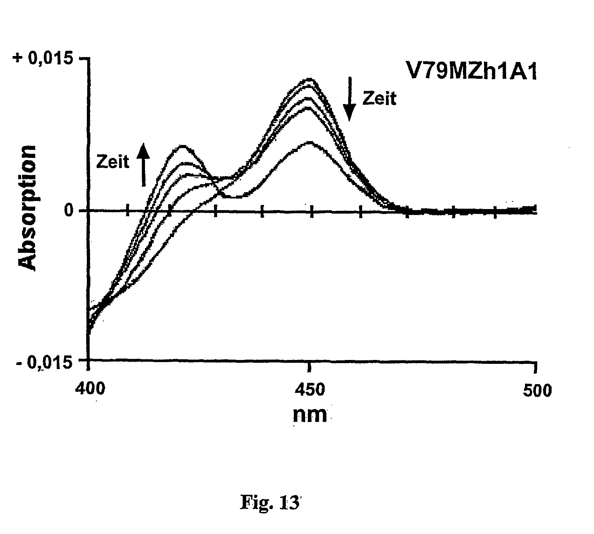

Durch die Reduktion des Solubilisates mit Natriumdithionit unter aeroben Bedingungen kann das prosthetische Häm labilisiert und zerstört werden (Omura und Sato, 1964b). Die Aufnahme eines CO-Spektrums zu verschiedenen Zeiten nach der Probenbegasung mit Kohlenmonoxid zeigte entsprechend eine deutliche Verringerung der Absorption bei 450 nm zugunsten einer Zunahme des Cytochrom P420-Peaks. Allerdings konnte zugleich gezeigt werden, daß der Fehler unter aeroben Bedingungen vernachlässigbar war, wenn die Spektren nur wenige Minuten nach Reduktion der Probe aufgenommen wurden (Figur 13).

### Einfluß von Quinidin auf die Stabilität von hCYP2D6

Enzyme lassen sich bisweilen durch ihre Substrate oder kompetitive Inhibitoren stabilisieren. Für hCYP2D6 wird eine Stabilisierung durch den kompetitiven Inhibitor Quinidin diskutiert (Gillam *et al*., 1995). Bei Aufnahme der Spektren von hCYP2D6 2, 9, 10 und 17 wurde im Gegensatz zum hCYP2D6 1-Spektrum jeweils ein mehr oder minder ausgeprägter Peak für Cytochrom P420 beobachtet. Daher wurde dem Solubilisierungspuffer Quinidin zugesetzt, um einer möglichen Labilisierung durch die Solubilisierung entgegenzuwirken. Die Spektren blieben jedoch weitgehend unverändert, insbesondere trat weiterhin ein Cytochrom P420-Peak auf. Das Cytochrom P450 mußte also schon vor der Solubilisierung und Reduktion degradiert worden sein.
Zum Nachweis wurde Quinidin bei der Zellaufzucht zur Spektrenaufnahme direkt in das Kulturmedium gegeben. Tatsächlich wurde ein stabilisierender Effekt beobachtet, der abhängig vom *hCYP2D6*-Allel sehr unterschiedlich ausgeprägt war (Tabelle 3): Das Spektrum von hCYP2D6 1 (Wildtyp) blieb unverändert. Der Cytochrom P450-Gehalt änderte sich nicht. Dies war zu erwarten, da das Enzym sich von vornherein durch hohe Stabilität auszeichnet. Bei Variante hCYP2D6 2 trat kein Cytochrom P420-Peak mehr auf. Der berechnete Cytochrom P450-Gehalt entsprach genau der Summe der zuvor bestimmten Gehalte an Cytochrom P450 und Cytochrom P420 (Tabelle 3). Ähnliches wurde bei hCYP2D6 17 beobachtet, wobei der Peak für Cytochrom P420 jedoch nicht völlig verschwand. Wie nach den Ergebnissen der Western-Analyse erwartet, war die Quantifizierung des hCYP2D6 10-CO-Differenzspektrums wegen der geringen Proteinmenge schwierig. Ein Cytochrom P450-Peak war nicht erkennbar. Der Cytochrom P420-Gehalt schien bei Inkubation mit Quinidin leicht erhöht zu sein. Eine dramatische Veränderung zeigte sich bei Variante hCYP2D6 9. Der Cytochrom P420-Peak verschwand vollständig und der Cytochrom P450-Gehalt war 4 - 6fach erhöht (Figur 14A).

Dahingegen deutete ein vergleichender Western-Blot wie bei allen anderen Varianten, wenn überhaupt, nur auf eine geringe Erhöhung des hCYP2D6 9-Apoprotein-Gehaltes hin (Figur 14B). Quinidin beeinflußt demnach die Stabilität des Apoproteins nicht entscheidend, sondern stabilisiert das prosthetische Häm im Cytochrom. Die Solubilisierbarkeit der betrachteten hCYP2D6-Varianten wurde von Quinidin bzw. der Häm-Inkorporation nicht wesentlich beeinflußt (Figur 12B).

### Cytochrom P450-Gehalte

Die bestimmten Cytochrom P450-Gehalte sind in Tabelle 3 zusammengefaßt.

**Tabelle 3**

| **Cytochrom P450-Gehalte verschiedener V79MZ-Zellinien. Angegeben sind entweder die Werte aus Einzelmessungen oder die Mittelwerte und Standardabweichungen aus mindestens drei unabhängigen Messungen.** | | | | | | |
|---|---|---|---|---|---|---|
| | **ohne Quinidin** **(pmol/mg Zellprotein)** | | | **mit Quinidin** **(pmol/mg Zellprotein)** | | |
| **Zellinie** | **P450** | **P420** | **P450 +P420** | **P450** | **P420** | **P450 +P420** |
| **V79MZmockneo** | 0 | 0 | 0 | - | - | - |
| **V79MZhOR** | 0 | 0 | 0 | - | - | - |
| **V79MZh1A1** | 11,4±1,8 | 0 | 11,4±1,8 | - | - | - |
| **V79MZr1A1** | 6,8±1,9 | 5,6±2,7 | 12,4±4,7 | - | - | - |
| **V79MZm1A1** | 7,6±1,9 | 2,6±0,8 | 10,2±2,0 | - | - | - |
| **V79MZf1A1 (scup)** | 2,5±0,8 | 3,5±1,6 | 6,1±2,4 | - | - | - |
| **V79MZh2E1** | 5,2±0,6 | 0,9±1,0 | 6,1±0,5 | - | - | - |
| **V79MZh3A4-hOR** | 8,0±1,0 | 3,0±0,9 | 11,0±1,7 | - | - | - |
| **V79MZh2D6*1-hOR** | 41,9 | 0 | **41,9** | 44,0 | 0 | 44,0 |
| **V79MZh2D6*1-S** | 17,4±1,8 | 0 | **17,4±1,8** | 14,41 | 0 | 14,41 |
| **V79MZh2D6*1-H** | 20,3 | 0 | **20,3** | 10,83 | 0 | 10,83 |
| **V79MZh2D6*1** | 24,9±3,2 | 0 | **24,9±3,2** | 30,9 | 0 | 30,9 |
| **V79MZh2D6*2** | 10,9±4,3 | 2,5±2,2 | **13,4±4,7** | 12,2 | 0 | 12,2 |
| **V79MZh2D6*9** | 5,4±2,4 | 1,0±0,9 | **6,4±1,9** | 35,7±13,3 | 0 | 35,7±13,3 |
| **V79MZh2D6*10** | 0 | 2,2±0,2 | **2,2±0,2** | 0 | 4,44 | 4,44 |
| **V79MZh2D6*17** | 4,3±0,7 | 3,8±0,7 | **8,2±1,0** | 9,0 | 1,9 | 10,9 |
| **Abkürzungen: -: nicht untersucht** | | | | | | |

In parentalen und Schein-transfizierten V79MZ-Zellen konnte kein Cytochrom P450 gemessen werden. Die relativen Gehalte an gesamtem Cytochrom P450 + P420 stimmten gut mit den relativen Bandenintensitäten in den Westem-Blots (Figuren 12B und 14B) überein. Dies ist ein Indiz dafür, daß die spektrophotometrisch bestimmten Cytochrom P450 + P420-Gehalte den Gehalten an Apoprotein entsprechen und folglich die endogene Häm-Synthese im V79MZ-Zellen ausreichend ist.
Um verschiedene Zellinien und allele Varianten zu vergleichen, kann entweder auf den Gehalt an funktionalem Holoenzym Cytochrom P450, auf den Gesamtgehalt an Cytochrom P450 + P420, den gesamten Apoproteingehalt oder auf das Expressionsniveau, also den mRNA-Gehalt, Bezug genommen werden. Je nach Bezugswert kann der Vergleich sehr verschieden ausfallen, beispielsweise aufgrund unterschiedlicher Häm-Inkorporation oder Proteinstabilitäten. Im Folgenden wird auf die Gesamtgehalte an Cytochrom P450 + P420 bezogen. Die entsprechenden Werte sind in Tabelle 3 fett gedruckt. Dabei wurde angenommen, daß die Menge an P420 wesentlich von der Präparation abhing, denn während der Anteil an P420 zwischen verschiedenen Präparationen schwankte, war der Gesamtgehalt an P420 + P450 etwa konstant.

### Vergleich mit früheren Messungen

Durch ein neues Solubilisierungsverfahren gelang es, mit 100fach weniger V79-Zellen als bisher CO-Differenzspektren aufzunehmen. Die so bestimmten Cytochrom P450-Gehalte waren etwas höher als die zuvor für V79MZCYP-Zellinien publizierten (Tabelle 4).

**Tabelle 4**

| **Vergleich der über CO-Differenzspektren bestimmten Cytochrom P450-Gehalte in V79MZCYP- Zellinien mit früheren Ergebnissen.** | | | |
|---|---|---|---|
| | **CO-Spektren mit Solubilisat pmol CYP/mg Zellprotein** | | |
| **Zellinie** | **P450** | **P420** | **Referenz** |
| **V79MZ parental** | **0** | **0** | **0 pmol/mg** (Onderwater *et al*., 1996) CO-Differenzspektrum von Mikrosomen |
| **V79MZh1A1** | **11,4 ± 1,8** | **0** | **14 pmol/mg** (Onderwater *et al*., 1996) CO-Differenzspektrum von Mikrosomen |
| **V79MZh3A4-hOR** | **8,0 ± 1,0** | **3,0 ± 0,9** | **5 pmol/mg** (Schneider *et al*., 1996) Western-Analyse von Zellhomogenat |

### Beispiel 5: Bufuralol-Hydroxylierung

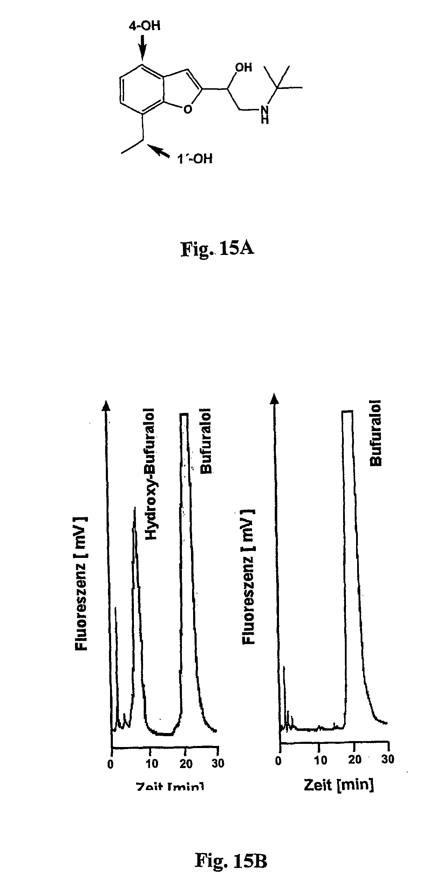

Zum Nachweis der Funktionalität des heterolog exprimierten Cytochroms P450 2D6 wurde die hCYP2D6-spezifische Hydroxylierung von Bufuralol (1'-Hydroxylierung von (+)-Bufuralol bzw. 4-Hydroxylierung von (-)-Bufuralol) bestimmt (Figur 15).

Dazu wurde ein verdünntes Aliquot Zellhomogenat auf Eis aufgetaut und durch Pipettieren sorgfältig rehomogenisiert. Die Reaktion wurde durch Zugabe von Homogenat zum Reaktionsansatz (final: 100 µl Gesamtreaktionsvolumen, 150 µg Gesamtprotein (V79MZh2D6*10: 300 µg), 200 µM Bufuralol, 2 mM NADPH (Boehringer Mannheim GmbH, Mannheim) in 0,1 M Kalium-phosphatpuffer, pH 7,4) gestartet und nach 30minütiger (V79MZh2D6*10: 90 min) Inkubation im Wasserbad bei 37 °C durch Zugabe von 12 µl 60 % (v/v) Perchlorsäure (Boehringer Mannheim GmbH, Mannheim; ca. 0,33 M) gestoppt. Die Proben wurden einige Minuten auf Eis inkubiert und das Präzipitat abzentrifugiert (10 min, 17000 x g, 4 °C). Das im Überstand enthaltene Substrat Bufuralol und Produkt Hydroxy-Bufuralol wurden mittels HPLC getrennt und fluorimetrisch detektiert (Kronbach *et al*., 1987; Kronbach, 1991). Die chromatographische Trennung erfolgte isokratisch in wäßrigorganischer mobiler Phase (30 % (v/v) Acetonitril (HPLC-Reinheitsgrad, Riedel-de Haën, Seelze), 40 % (v/v) Methanol, 30 % (v/v) Wasser, 2 mM Perchlorsäure), bei einer Flußrate von 1 ml/min und 50 °C über eine Hypersil ODS C18 *"reversed phase"-*Säule (24 cm x 4,6 mm, Partikelgröße 5 µm; Supelco, Bellefonte, PA). Dem System war eine C8-Vorsäule vorgeschaltet. Das Fluoreszenzsignal (Anregung bei 252 nm, Emission bei 352 nm) wurde am Fluorescence HPLC Monitor RF-530 (Shimadzu (Europa) GmbH, Düsseldorf) detektiert, mit dem Chromatopac C-R3A 530 (Shimadzu (Europa) GmbH, Düsseldorf) aufgezeichnet und die Peakfläche automatisch integriert.
Die Retentionszeiten betrugen für Hydroxy-Bufuralol ca. 8 min und für Bufuralol ca. 22 min. Die Quantifizierung erfolgte durch externe Standardisierung mit einer 1'-Hydroxy-Bufuralol-Standardkurve im Bereich von 0,5 - 20 µM Endkonzentration nach Inkubation mit Homogenat der Schein-transfizierten Zellinie V79MZmockneo.
Die Konzentrationen der gravimetrisch hergestellten Stammlösungen wurden spektrophotometrisch überprüft. Es wurde mit Extinktionskoeffizienten von 16,3 mM⁻¹cm⁻¹ für l'-Hydroxy-Bufuralol bei 245,5 nm (Gentest Corp., Woburn, MA) und 15,1 mM⁻¹cm⁻¹ für Bufuralol bei 248 nm (Ultrafine Chemicals, London, England) gerechnet.

Die Reaktion war bei 37 °C bis zu 150 µg Gesamtprotein/100 µl über 30 min im linearen Bereich. Um bei Variante hCYP2D6 10 ein quantifizierbares Signal messen zu können, mußten 300 µg Gesamtprotein/100 µl über 90 min inkubiert werden. Die angegebenen Aktivitäten für hCYP2D6 10 unterschätzen daher die tatsächlichen Werte; vgl. Tabelle 6.

### Inhibition der Bufuralol-Hydroxylierung

Für Inhibitionsstudien wurde dem Reaktionsansatz zur Bufuralol-Hydroxylierung Quinidin in einer Endkonzentration von 0,01 - 2 µM (2 mM Stammlösung in Methanol, dann verdünnt in 0,1 M Kaliumphosphatpuffer, pH 7,4; < 0,1 % Methanol im Assay; Kontrolle ohne Quinidin mit 0,1 % Methanol) oder inhibitorisches anti-hCYP2D6-Antiserum (LKM-Serum 2) und humanes Kontrollserum (freundlicherweise zur Verfügung gestellt von Dr. U. M. Zanger, Dr. Margarete Fischer-Bosch-Institut, Stuttgart) in Endverdünnungen von 1:100 - 1:1000 zugesetzt.

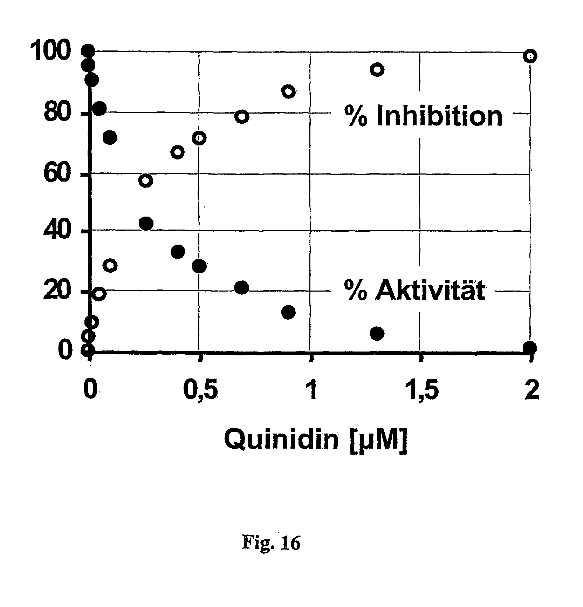

Die Bufuralol-Hydroxylierung ließ sich mit hCYP2D6-spezifischem Antiserum in einer Endverdünnung von 1:100 bei allen hCYP2D6-Varianten nahezu vollständig inhibieren. Bei einer Endkonzentration von 1 µM des hCYP2D6-spezifischen Inhibitors Quinidin war die Bufuralol-Hydroxylierung allelunabhängig zu etwa 90 % inhibiert (Figur 16).

### Beispiel 6: Kultivierungs- und Homogenisierungs-Bedingungen

Um auszuschließen, daß die endogene Häm-Synthese für den Gehalt an funktionalem Cytochrom P450 limitierend ist, wurde das Kulturmedium mit Häminchlorid (Sigma, Deisenhofen) bzw. der limitierenden Synthesevorstufe δ-Aminolävulinsäure (Hydochlorid, Sigma, Deisenhofen) und Eisen(III)zitrat (Sigma, Deisenhofen) supplementiert. Bis zur Zytotoxizität bei 10 µM Häminchlorid bzw. 10 mM δ-Aminolävulinsäure/Eisen(III)zitrat wurde keine gesteigerte Bufuralol-Hydroxylase-Aktivität pro mg Gesamtprotein im Zellhomogenat gemessen.

Die Bufuralol-Hydroxylase-Aktivität im Zellhomogenat war jedoch von der Zelldichte beim Ernten der Zellen, der Art der Homogenisierung sowie der Anzahl an Einfrier/Auftau-Zyklen abhängig. Das Verfahren zur Gewinnung des Zellhomogenates wurde daher optimiert:
Bei Zelldichten über 90 % nahm die Bufuralol-Hydroxylase-Aktivität pro mg Gesamtprotein im Zellhomogenat um etwa 10 - 20 % ab. Bei überwachsenen Kulturen mit Zelldichten deutlich über 100 % wurde nur etwa die Hälfte der maximalen Aktivität gemessen. Um Zellhomogenat für enzymatische Umsetzungen zu erhalten, mußten die Zellen aufgebrochen werden. Die höchsten Aktivitäten wurden nach Frieren in flüssigem Stickstoff bestimmt. Die Benutzung eines Glas-Glas-Homogenisators resultierte in 10 - 20 % niedrigeren Aktivitäten. Durch Zugabe von 1 mM PMSF als Proteaseinhibitor konnte der Aktivitätsverlust verhindert werden. Ultraschallbehandlung führte sowohl ohne als auch mit PMSF zum Verlust von bis zu 70 % der Aktivität. Wiederholtes Auftauen und Einfrieren des Zellhomogenates führte zu nicht reproduzierbaren Änderungen in der Bufuralol-Hydroxylase-Aktivität.

Die optimalen Bedingungen zur Gewinnung von Zellhomogenat sind bei Zelldichten von 90 % gegeben mit anschließendem Ernten der Zellen durch Pelletieren, Resuspendieren in Puffer, Aliquotieren, Schockgefrieren in flüssigem Stickstoff und Lagerung bei - 80 °C bis zum Gebrauch. Die Aliquots wurden nur einmal aufgetaut und Reste verworfen.

### Beispiel 7: Koexpression von hCYP2D6 und hCYPOR und Promotorvergleich

Für einige heterolog exprimierte Cytochrom P450-Isoformen ist die endogene CYPOR-Synthese in V79-Zellen für die maximale enzymatische Aktivität nicht ausreichend. Daher wurde beispielsweise hCYP3A4 mit hCYPOR koexprimiert (Schneider *et al.,* 1996). Ein entsprechender Ansatz wurde mit hCYP2D6 1 verfolgt (Transfektionsansatz T11); vgl. Tabelle 5.

Zum Nachweis der hCYPOR-Aktivität wurde die NADPH-abhängige Cytochrom c-Reduktase-Aktivität bestimmt (Kubota *et al*., 1977). Dazu wurde ein verdünntes Aliquot Zellhomogenat auf Eis aufgetaut und durch Pipettieren sorgfältig rehomogenisiert. Nach 2minütiger Vorinkubation des Reaktionsansatzes bei 37 °C (final: 800 µl Gesamtreaktionsvolumen, 40 - 100 µg Gesamtprotein, 225 µM Kaliumcyanid (Sigma, Deisenhofen), um die Reoxidation von reduziertem Cytochtom c durch die Cytochrom-Oxidase zu verhindern, 125 µM NADPH, 45 µM Ferri-Cytochrom c (Sigma, Deisenhofen) aus Rinderherz in 50 mM Kaliumphosphatpuffer, pH 7,8) wurde die Reaktion durch Zugabe des Cytochrom c gestartet und die Zunahme der Extinktion bei 550 nm und 37 °C am Uvikam 941 Plus UV/VIS-Spektrophotometer (Kontron Instruments Ltd., Watford, England) aufgezeichnet. Für die Berechnung der Cytochrom c-Reduktase-Aktivität aus der Anfangssteigung wurde ein Extinktionskoeffizient von 19,1 mM⁻¹cm⁻¹ verwendet (Chance, 1957).

In Übereinstimmung mit Schneider *et al*. (1996) betrug die endogene Cytochrom c-Reduktase-Aktivität von V79MZ-Zellen 9 - 14 nmol/min/mg Zellprotein. Bei zusätzlicher Expression von hCYPOR wurden deutliche höhere Cytochrom c-Reduktase-Aktivitäten von 31 - 182 nmol/mg/min gemessen.

**Tabelle 5**

| **Cytochrom c-Reduktase-Aktivitäten der CYPOR (Cyt**_{**c**}**-Red.) und Bufuralol-Hydroxylase-Aktivitäten des hCYP2D6. Die Selektivität ist der Quotient aus den (-)-Bufuralol- und (+)-Bufuralol-Aktivitäten. Es sind die Mittelwerte und Standardabweichungen aus mindestens drei unabhängigen Messungen angegeben.** | | | | | | | | | |
|---|---|---|---|---|---|---|---|---|---|
| **Zellinie** | **Promotor** | **Cyt**_{**c**}**-Red. nmol/mg/min** | **Bufuralol-Hydroxylierung pmol/mg/min** | | | **Bufuralol-Hydroxylierung pmol/pmol hCYP2D6/min** | | | **Selek- tivität** |
| | | | **(+)** | **(+/-)** | **(-)** | **(+)** | **(+/-)** | **(-)** | **(-)/(+)** |
| **V79MZmockneo** | **-** | **8,8±1,3** | **0** | **0** | **0** | **-** | **-** | **-** | **-** |
| **V79MZhOR** | **-** | **124,6±24,9** | **-** | **-** | **-** | **-** | **-** | **-** | **-** |
| **V79MZh3A4-hOR** | **-** | **31,5±8,5** | **-** | **-** | **-** | **-** | **-** | **-** | **-** |
| **V79MZh2D6*1-hOR** | **SV40** | **182,2±8,0** | **292±34** | **143±6** | **64±6** | **7,0±0,8** | **3,4±0,2** | **1,5±0,2** | **0,22±0,05** |
| **V79MZh2D6*1** | **SV40** | **13,5±4,2** | **163±32** | **108±15** | **65±14** | **6,5±2,1** | **4,3±1,2** | **2,6±0,9** | **0,40±0,16** |
| **V79MZh2D6*1-H** | **CMVp** | **14,1±1,6** | **139±27** | **76±5** | **60±13** | **6,9±1,3** | **3,8±0,3** | **2,9±0,7** | **0,43±0,18** |
| **V79MZh2D6*1-S** | **MPSV-LTR +CMVe** | **9,9±1,1** | **110±19** | **57±5** | **45±1** | **6,3±1,7** | **3,3±0,6** | **2,6±0,3** | **0,41±0,08** |
| **Abkürzungen: (+): (+)-Bufuralol; (-): (-)-Bufuralol; (+/-): razemisches Bufuralol; SV40: früher SV40 Promotor; CMVp: Cytomegalovirus-Promotor; MPSV-LTR + CMVe: Myeloproliferativer Sarkoma Virus-*long terminal repeat* und Cytomegalovirus-*Enhancer*** | | | | | | | | | |

Bei Koexpression von hCYP2D6 1 mit hCYPOR wurden wesentlich höhere Bufuralol-Hydroxylase-Aktivitäten gemessen als bei heterologer Expression von hCYP2D6 1 allein (Tabelle 5). Nach Normierung auf die hCYP2D6-Gehalte (vgl. Tabelle 4) ergaben sich jedoch unabhängig von der hCYPOR-Koexpression für alle hCYP2D6 1-exprimierenden Zellinien nahezu identische Umsatzraten. Im Gegensatz zu hCYP3A4 ist die hCYP2D6-Aktivität folglich nicht durch die endogene CYPOR-Aktivität limitiert. Die Aktivitätsunterschiede zwischen den Zellinien V79MZh2D6*1-hOR und V79MZh2D6*1 sind folglich auf eine unterschiedliche Integration der cDNA-Expressionkassette in das V79MZ-Genom zurückzuführen. Der Vergleich mit den Aktivitäten der Zellinien V79MZh2D6*1-H und V79MZh2D6*1-S zeigte zudem, daß der "Integrationseffekt" erheblich größeren Einfluß auf Aktivitätsunterschiede zwischen verschiedenen Zellinien bzw. den homogenen Klonen eines Transfektionsansatzes hat als die Wahl des Promotors. Insbesondere sind im V79-System die Expressionsraten mit SV40- und CMV-Promotor vergleichbar, während in COS-1 Zellen mit dem CMV-Promotor bis zu 10fach höhere Expressionsraten erreicht werden (Clark und Waterman, 1991). Ähnliche Ergebnisse wurden von Schneider *et al*. (1996) gefunden.
Die gute Übereinstimmung der Umsatzraten demonstrierte außerdem die Zuverlässigkeit sowohl des Bufuralol-Hydroxylase-Aktivitätstestes als auch der Quantifizierung des Cytochrom P450-Gehaltes mittels CO-Differenzspektren von solubilisiertem Zellhomogenat.

### Beispiel 8: Vergleich der Zellinien V79MZh2D6*1, *2, *9, *10 und *17

### Bufuralol-Hydroxylase-Aktivität

Die polymorphen Zellinien V79MZh2D6*1, *2, *9, *10 und *17 wurden hinsichtlich der hCYP2D6-spezifischen Bufuralol-Hydroxylierung verglichen. Während die Selektivität gegenüber (+)-Bufuralol für alle Zellhomogenate praktisch identisch war, waren die Aktivitäten gegenüber dem Wildtyp-Zellhomogenat V79MZh2D6*1 vermindert (Tabelle 6). Insbesondere besaß V79MZh2D6*10-Zellhomogenat lediglich etwa 2 % der Wildtyp-Zellhomogenat-Aktivität. Nach Normierung auf die hCYP2D6-Gehalte (vgl. Tabelle 4) ergaben sich für hCYP2D6 1 und hCYP2D6 2 vergleichbare, für hCYP2D6 9 etwa doppelt und für hCYP2D6 17 etwa halb so große Umsatzraten.

**Tabelle 6**

| **Bufuralol-Hydroxylase-Aktivitäten der polymorphen Zellinien bei 200 µM Bufuralol. Die Selektivität ist der Quotient aus den (-)-Bufuralol- und (+)-Bufuralol-Aktivitäten. Es sind die Mittelwerte und Standardabweichungen aus mindestens drei unabhängigen Messungen angegeben.** | | | | | | | |
|---|---|---|---|---|---|---|---|
| **Zellinie** | **Bufuralol-Hydroxylase-Aktivität pmol/mg/min** | | | **Bufuralol-Hydroxylase-Umsatzrate pmol/pmol hCYP2D6/min** | | | **Selektivität** |
| | (+) | (+/-) | (-) | (+) | (+/-) | (-) | (-)/(+) |
| **V79MZmockneo** | **0** | **0** | **0** | - | - | - | - |
| **V79MZh2D6*1** | **162,7±31,6** | **108,0±15,3** | **65,0±13,5** | **6,53±2,11** | **4,34±1,17** | **2,61±0,88** | **0,40±0,16** |
| **V79MZh2D6*2** | **95,2±28,6** | **59,7±13,9** | **43,8±14,7** | **7,11±4,63** | **4,46±2,60** | **3,27±2,24** | **0,46±0,29** |
| **V79MZh2D6*9** | **93,9±11,5** | **61,8±4,7** | **45,9±3,1** | **14,67±6,2** | **9,66±3,60** | **7,17±2,61** | **0,49±0,16** |
| **V79MZh2D6*10** | **3,3±0,4** | **2,3±0,2** | **1,4±0,3** | **1,48±0,29** | **1,03±0,17** | **0,63±0,18** | **0,42±0,14** |
| **V79MZh2D6*17** | **29,3±1,9** | **21,6±2,4** | **14,4±3,3** | **3,57±0,66** | **2,65±0,61** | **1,77±0,62** | **0,49±0,14** |
| **Abkürzungen: (+): (+)-Bufuralol; (-): (-)-Bufuralol; (+/-): razemisches Bufuralol** | | | | | | | |

### Kinetik der (+)-Bufuralol-1'-Hydroxylierung

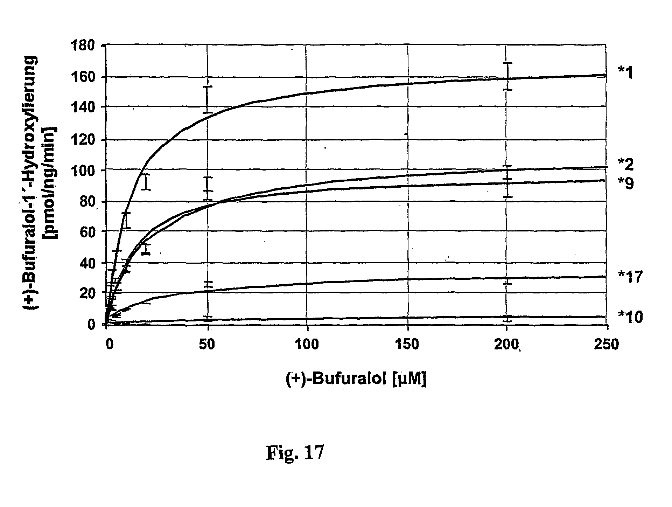

Die Allel-spezifischen kinetischen Parameter der (+)-Bufuralol-1'-Hydroxylierung wurden unter Annahme einer monophasischen Michaelis-Menten-Kinetik ohne Inhibitor (V = Vₘₐₓ * [S] / (K_{M} + [S]) ermittelt (Tabelle 7). Die nicht-lineare Kurvenanpassung an die Meßpunkte V erfolgte durch Minimierung der Summe der Abstandsquadrate und Wichtung mit dem Faktor 1/V (Figur 17). Dabei wurde mit den Mittelwerten und Standardabweichungen aus drei unabhängigen Messungen gearbeitet.

**Tabelle 7**

| **Kinetische Parameter der (+)-Bufuralol-1'-Hydroxylierung für hCYP2D6 1, 2, 9, 10 und 17. Es sind die Mittelwerte und Standardabweichungen aus drei unabhängigen Messungen angegeben.** | | | | | |
|---|---|---|---|---|---|
| **Zellinie** | **Bezug auf mg Gesamtprotein** | | | **Bezug auf den Cytochrom P450-Gehalt** | |
| | **V**_{**max**} **pmol/mg/min** | **K**_{**M**} **µM** | **Cl**_{**int**} **ml/mg/min** | **Umsatzrate pmol/pmol P450/min** | **Cl**_{**int**} **ml/pmol P450/min** |
| **V79MZh2D6*1** | **170,8±15,5** | **13,8±1,6** | **12400±2580** | **6,9±1,5** | **500±170** |
| **V79MZh2D6*2** | **111,5±2,1** | **22,6±0,6** | **4940±220** | **8,3±3,1** | **370±150** |
| **V79MZh2D6*9** | **100,0±15,0** | **15,0±2,9** | **6670±1970** | **15,6±7,0** | **1040±670** |
| **V79MZh2D6*10** | **4,3±0,1** | **53,3±10,3** | **80±17** | **1,9±0,2** | **40±10** |
| **V79MZh2D6*17** | **34,3±2,8** | **28,2±1,3** | **1220±160** | **4,2±0,9** | **150±40** |
| **Abkürzungen: V**_{**max**}**: maximale Reaktionsgeschwindigkeit bei Substratsättigung des Enzyms; K**_{**M**}**: Michaelis-Menten-Konstante; Cl**_{**int**}**: intrinsische Clearance (Cl**_{**int**} **= V**_{**max**}**/K**_{**M**}**)** | | | | | |

### Beispiel 9: Vergleich mit anderen Expressionssystemen

### hCYP2D6 2 (Substitutionen Arg₂₉₆Cys und Ser₄₈₆Thr)

Mit V79MZh2D6*2-Homogenat wurden im Vergleich zum Wildtyp-Enzym hCYP2D6 1 ein etwas erhöhter K_{M} und eine vergleichbare Umsatzrate bestimmt. Das CO-Differenzspektrum zeigte im Gegensatz zum Wildtyp-Enzym geringe Mengen an Cytochrom P420.
In völliger Übereinstimmung mit dem V79-Expressionssystem betrug die absolute Bufuralol-Hydroxylase-Aktivität von hCYP2D6 2 gegenüber dem Wildtyp bei cDNA-Expression in COS-1 Zellen bei vergleichbarer Umsatzrate nur etwa 60 % (Oscarson *et al*., 1997). Es blieb unklar, ob es sich um einen Zufall handelt oder eine Allel-abhängige Ursache existiert. Bei Expression von hCYP2D6 1 und hCYP2D6 2 in Hefe (Oscarson *et al*., 1997) war die (+)-Bufuralol-Umsatzrate bei Substratsättigung vergleichbar und der Holoprotein-Gehalt von hCYP2D6 2 leicht verringert. Als Ursache wird eine verminderte Proteinstabilität oder auch Translationsrate diskutiert. Auf eine gegenüber dem Wildtyp-Enzym geringere Proteinstabilität weist auch der Cytochrom P420-Peak im CO-Differenzspektrum von V79MZh2D6*2 hin.

### hCYP2D6 9 (Deletion Lys₂₈₁)

Mit V79MZh2D6*9-Homogenat wurden im Vergleich zum Wildtyp-Enzym hCYP2D6 1 ein identischer K_{M} und eine 2fach höhere Umsatzrate bestimmt.
Bei Expression von hCYP2D6 9 mittels rekombinantem Vacciniavirus in HepG2-Zellen wurde bei Untersuchung der (+)-Bufuralol-Hydroxylierung gegenüber dem Wildtyp-Enzym hCYP2D6 1 ein 2,4fach höherer K_{M} und eine etwa 4fach höhere Umsatzrate gemessen (Tyndale *et al*., 1991a). Die intrinsische Clearance für hCYP2D6 9 war also in beiden *In vitro*-Expressionssystemen gegenüber dem Wildtyp verdoppelt.

### hCYP2D6 10 (Substitutionen Pro₃₄Ser und Ser₄₈₆Thr)

Mit V79MZh2D6*10-Homogenat wurden im Vergleich zum Wildtyp-Enzym hCYP2D6 1 ein 4fach höherer K_{M} und eine 3,5fach geringere Umsatzrate bestimmt. Die intrinsische Clearance betrug etwa ein Zwölftel derjenigen von hCYP2D6 1. Allerdings wurde auf den Cytochrom P420-Gehalt normiert, da bei 450 nm kein quantifizierbarer Peak zu erkennen war. Die Western-Blots zeigten darüberhinaus, daß die Apoproteinmenge gegenüber allen anderen Varianten drastisch verringert war. Daraus folgt, daß die Umsatzrate bezogen auf funktionales Holoenzym möglicherweise dem Wildtyp entspricht, während die Aktivität im Homogenat nur etwa 2,5 % derjenigen von V79MZh2D6*1-Homogenat besitzt. Die Bestimmung der K_{M}-Konstante ist wegen der geringen Aktivität des V79MZh2D6*10-Homogenates ebenfalls unsicher.
*In vitro*-Expressions-Experimente in COS-1 Zellen zeigten, daß die Substitution Ser₄₈₆Thr allein die Menge an exprimiertem hCYP2D6 10 im Vergleich zum Wildtyp leicht steigert, während die Substitution Pro₃₄Ser zu drastisch verminderten Proteinmengen und 40fach verminderter Aktivität führt. Bei Kombination beider Substitutionen in hCYP2D6 10 überwiegt der Aktivitäts-mindernde Effekt der Substitution Pro₃₄Ser bei weitem (Johansson *et al*., 1994; Kagimoto *et al*., 1990). Dies entspricht exakt den Befunden der Westem-Analyse und dem Aktivitäts-Unterschied zwischen den V79MZh2D6*10 und V79MZh2D6*1-Homogenaten. Während *in vitro* also 40fache Aktivitäts-Unterschiede zwischen hCYP2D6 1 und hCYP2D6 10 gefunden werden, betragen sie *in vivo* nur etwa ein Zehntel (Droll *et al*., 1998).

### hCYP2D6 17 (Substitutionen Thr₁₀₇Ile, Arg₂₉₆Cys und Ser₄₈₆Thr)

Mit V79MZh2D6*17-Homogenat wurden im Vergleich zum Wildtyp-Enzym hCYP2D6 1 ein 2fach erhöhter K_{M} und eine 2fach niedrigere Umsatzrate bestimmt. Das CO-Differenzspektrum zeigte im Gegensatz zum Wildtyp-Enzym einen Anteil von etwa 50 % an Cytochrom P420.
In Übereinstimmung mit dem V79-Expressionssystem betrug die absolute Bufuralol-Hydroxylase-Aktivität von hCYP2D6 17 gegenüber dem Wildtyp bei cDNA-Expression in COS-1 Zellen nur etwa 20 % (Oscarson *et al*., 1997): Wie bei den Substitutionen Arg₂₉₆Cys und Ser₄₈₆Thr wurde auch mit der Substitution Thr₁₀₇Ile alleine kein wesentlicher Effekt auf die Umsatzrate der Bufuralol-Hydroxylierung beobachtet. Allerdings führte die Substitution Thr₁₀₇Ile ähnlich wie die Substitutioin Ser₄₈₆Thr zu erhöhten Proteinmengen, während die Kombination aller drei Substitutionen in hCYP2D6 17 verringerte Proteinmengen bei cDNA-Expression in COS-1 Zellen bewirkte. Als Ursache wird eine verminderte Enzym-Stabilität oder Translationsrate diskutiert. Auf eine gegenüber dem Wildtyp-Enzym geringere Proteinstabilität weist auch der hohe Anteil an Cytochrom P420 im CO-Differenzspektrum von V79MZh2D6* 17 hin.
Bei cDNA-Expression in Hefe war die Umsatzrate der Bufuralol-Hydroxylierung bei Substratsättigung für alle Mutationen und Kombinationen ähnlich (Oscarson *et al*., 1997). Die alleinige Substitution des hydrophilen Thr₁₀₇ gegen hydrophobes Ile in einer konservierten Region der β'-Helix, die zudem Teil der ersten Substrat-Erkennungs-Region ist, führte jedoch zu einer Erhöhung des K_{M} der Codein-O-Demethylierung (Oscarson *et al*., 1997). Der K_{M} für die Bufuralol-Hydroxylierung war hingegen unverändert. Erst die Komination der Substitutionen Thr₁₀₇Ile und Arg₂₉₆Cys bewirkte hier einen 5fach erhöhten K_{M}. Damit ist hCYP2D6 17 die einzige bekannte hCYP2D6-Variante, bei der eine Kombination verschiedener Substitutionen für eine geänderte Affinität des Enzyms zum Substrat verantwortlich ist.

### Beispiel 10: Tamoxifen-4-Hydroxylierung durch hCYP2D6

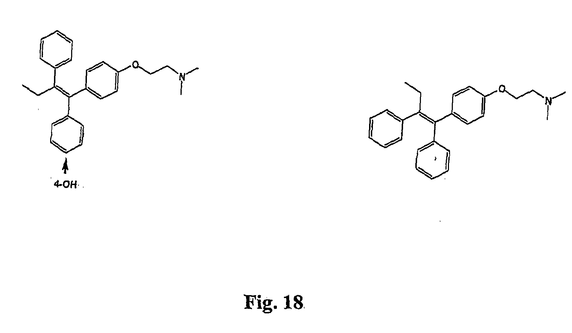

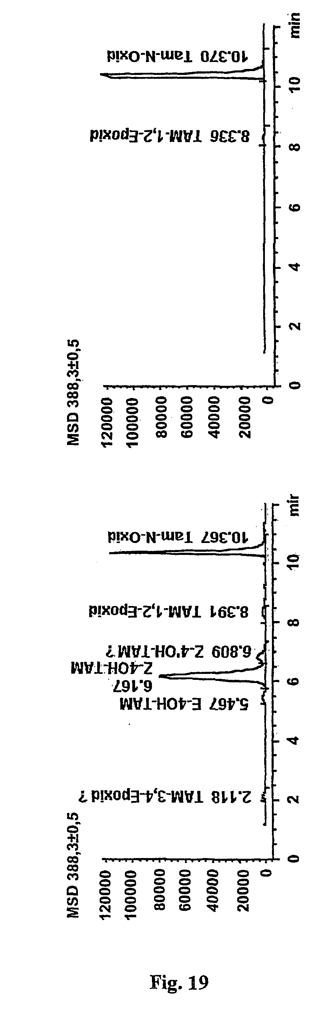

Die erfindungsgemäßen Zellinien wurden eingesetzt, um einen möglichen Einfluß des hCYP2D6-Polymorphismus auf die pharmakologisch wichtige 4-Hydroxylierung von Tamoxifen zu untersuchen (Figur 18). Substrat und Metaboliten wurden mittels HPLC/MSD getrennt und detektiert (Figur 19).

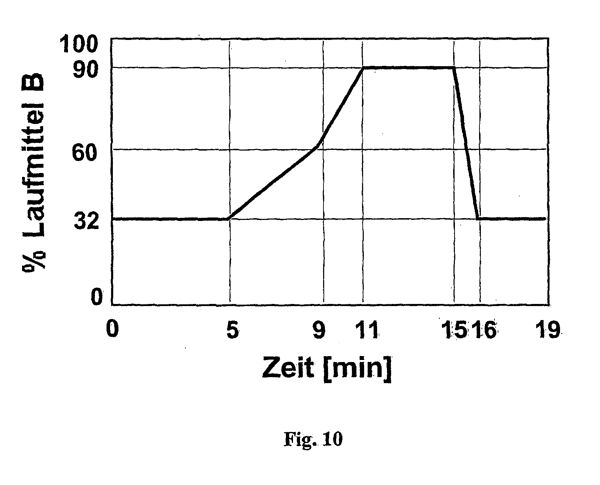

Ein verdünntes Aliquot Zellhomogenat wurde auf Eis aufgetaut und durch Pipettieren sorgfältig rehomogenisiert. Die Reaktion wurde durch Zugabe von Homogenat zum Reaktionsansatz (final: 100 µl Gesamtreaktionsvolumen, 150 µg Gesamtprotein, 1 - 150 µM Tamoxifen (2,5 µl Stammlösung in DMSO), 2 mM NADPH in 0,1 M Kaliumphosphatpuffer, pH 7,4) gestartet und nach 30minütiger (V79MZh2D6*10: 60 min) Inkubation im Wasserbad bei 37 °C durch Zugabe von 50 µl Acetonitril, 2 % (v/v) Essigsäure gestoppt. Die Proben wurden einige Minuten auf Eis inkubiert und das Präzipitat abzentrifugiert (10 min, 17000 x g, 4 °C). Das im Überstand enthaltene Substrat Tamoxifen und die Reaktionsprodukte, darunter 4-Hydroxy-Tamoxifen wurden mittels HPLC/ESI-MSD getrennt und detektiert. Die chromatographische Trennung erfolgte im Acetonitril-Gradienten (Figur 10), bei einer Flußrate von 0,5 ml/min (12. bis 19. Minute 0,8 ml/min) und 30 °C über eine C8 (2) *"reversed phase"*-Säule (Luna, 150 mm x 2 mm, Partikelgröße 5 µm; Phenomenex , Hösbach). Dem System war eine C8-Vorsäule (XDB-C8, *narrow-bore column,* 2,1 mm x 12,5 mm; Zorbax HPLC Columns, Hewlett Packard, Waldbronn) vorgeschaltet.
Die Detektion von Tamoxifen und seinen Metaboliten erfolgte am HP Series 1100 MSD (Hewlett Packard, Waldbronn) im *single ion monitoring modus* bei m/z 344,2 (*N* -Didesmethyl-Tamoxifen), m/z 358,3 (*N*-Desmethyl-Tamoxifen), m/z 360,2 (monooxygenierte Metaboliten von *N*-Didesmethyl-Tamoxifen), m/z 372,3 (Tamoxifen), m/z 374,3 (monooxygenierte Metaboliten von *N*-Desmethyl-Tamoxifen), m/z 388,3 (monooxygenierte Metaboliten von Tamoxifen) und m/z 404,3 (monooxygenierte Metaboliten von Tamoxifen-*N*-Oxid).
Die Retentionszeiten betrugen für Z-Tamoxifen-3,4-Epoxid 2,1 min, für E- und Z-4-Hydroxy-Tamoxifen ca. 5,5 und 6,2 min, für E- und Z-4-Hydroxy-Tamoxifen-*N*-Oxid etwa 7,2 und 7,9 min, für Z-Tamoxifen-1,2-Epoxid etwa 8,4 min, für Z-*N*-Didesmethyl-Tamoxifen 9,9 min, für Z-*N*-Desmethyl-Tamoxifen 10,0 min, für Z-Tamoxifen-*N*-Oxid 10,4 min und für E- und Z-Tamoxifen ca. 9,7 und 10,1 min. Die Peaks für E- und Z-4-Hydroxy-Tamoxifen, Z-Tamoxifen-*N*-oxid, Z-Tamoxifen-1,2-Epoxid, Z-*N*-Desmethyl-Tamoxifen und Z-*N*-Didesmethyl-Tamoxifen wurden mit den entsprechenden Reinsubstanzen verifiziert und extern standardisiert. Die Kalibrierung erfolgte im Bereich von 0,039 - 20 µM Endkonzentration nach Inkubation mit Homogenat der Schein-transfizierten Zellinie V79MZmockneo.
Zur Identifizierung der an der Tamoxifen-4-Hydroxylierung beteiligten Cytochrom P450-Isoformen wurden Inkubationen mit Homogenaten der Zellinien V79MZh2E1 und V79MZh3A4-hOR sowie mit Mikrosomen hCYP3A4- und hCYPOR- bzw. hCYP2C9*1-und hCYPOR-koexprimierender Insektenzellen, sogenannten "Supersomen" (Gentest, Woburn, MA, Produkt-Nr. P207 und P218), durchgeführt. Gegenüber den Inkubationen mit V79MZh2D6-Homogenat wurden folgende Parameter variiert: 150 µg V79MZh2E1-Homogenat wurden bis zu 45 min lang inkubiert, bis zu 500 µg V79MZh3A4-hOR-Homogenat und bis zu 25 µl hCYP3A4-hOR-"Supersomen", entsprechend 50 pmol hCYP3A4, wurden mit 100 µM Magnesiumchlorid und 10 µM EDTA bis zu 60 min lang inkubiert und 12,5 µl hCYP2C9*1-hOR-"Supersomen", entsprechend 25 pmol hCYP2C9*1, wurden mit 100 µM Magnesiumchlorid und 10 µM EDTA 30 min lang inkubiert

Die Reaktion war bei 37 °C bis zu 150 µg Gesamtprotein/100 µl über 30 min im linearen Bereich. Um bei Variante hCYP2D6 10 ein quantifizierbares Signal messen zu können, mußte der Reaktionsansatz 60 min lang inkubiert werden. Die angegebenen Aktivitäten unterschätzen daher die tatsächlichen Werte geringfügig.

### Einfluß von DMSO als Lösungsvermittler für Tamoxifen

Da Tamoxifen schlecht wasserlöslich ist, wurde DMSO als Lösungsvermittler zugegeben. Mit Acetonitril und Methanol wurden keine besseren Löslichkeiten erzielt. Außerdem waren sie wegen anderer unerwünschter Eigenschaften weniger gut geeignet als DMSO.

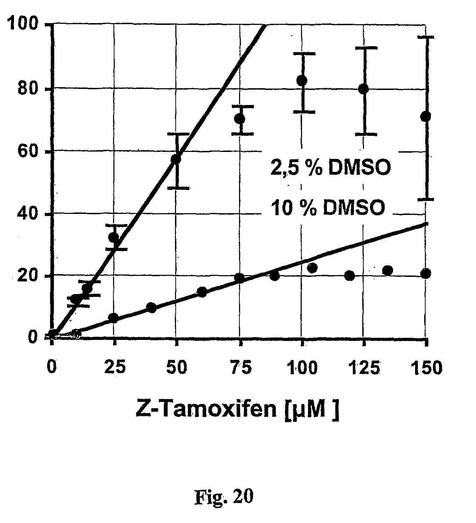

Die Tamoxifen-4-Hydroxylase-Aktivität wurde bis zu einer Konzentration von 2,5 % DMSO nur geringfügig beeinflußt. Bei einer Konzentration von 10 % DMSO betrug die Tamoxifen-4-Hydroxylase-Aktivität nur etwa 20 % derjenigen bei 2,5 % (Figur 20). Andererseits war der Anfangsbereich der Kinetik bei 10 % DMSO bis zu ca. 75 µM Tamoxifen linear, bei 2,5 % DMSO nur bis zu 50 µM. Dieses Verhalten entsprach genau den maximalen Löslichkeiten von Tamoxifen bei 10 % bzw. 2,5 % DMSO. Folglich hatte das Abknicken der Kinetik keine enzymkinetische Ursache, sondern war allein von der Löslichkeitsgrenze des Tamoxifen abhängig. Um bei allen hCYP2D6-Varianten ein quantifizierbares Signal messen zu können, wurden alle weiteren Messungen mit 2,5 % DMSO durchgeführt.

Eine nur geringe Abnahme der hCYP2D6-Aktivität bis zu 2,5 % DMSO wurde auch für die Dextromethorphan-O-Demethylierung publiziert (Chauret *et al.,* 1998; Hickman *et al*., 1998). Für die (+/-)-Bufuralol-Hydroxylase-Aktivität wurde dagegen eine drastische Abnahme schon bei niedrigen DMSO-Konzentrationen gefunden (Busby *et al*., 1999). Möglicherweise ist der inhibitorische Effekt der Lösungsmittel vom Substrat abhängig.

### Kinetik der Tamoxifen-4-Hydroxylierung

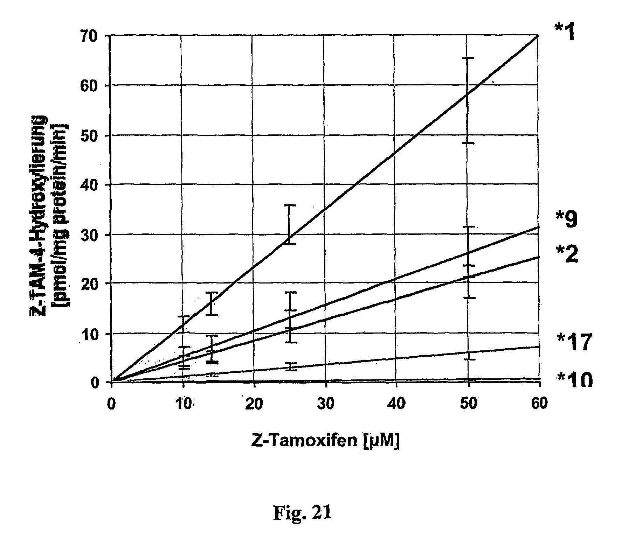

Weil das Ende der Linearität in der Kinetik der hCYP2D6-katalysierten Tamoxifen-4-Hydroxylierung keine enzymkinetische Ursache hatte, sondern allein von der Löslichkeitsgrenze des Tamoxifen abhängig war, konnten Vₘₐₓ und K_{M} nicht unabhängig voneinander bestimmt werden. Daher wurde unter Annahme einer monophasischen Michaelis-Menten-Kinetik ohne Inhibitor (V = Vₘₐₓ * [S] / (K_{M} + [S])) aus der linearen Anfangssteigung ([S] → 0) die intrinsische Clearance (Clᵢₙₜ = Vₘₐₓ / K_{M}) berechnet (Tabelle 8). Die Geradenanpassung an die Meßpunkte V erfolgte durch lineare Regression (Figur 21). Eine analoge Kinetik wurde für die Tamoxifen-4-Hydroxylierung durch hCYP2C9 1 aufgenommen (nicht dargestellt).

**Tabelle 8**

| **Kinetische Parameter der Tamoxifen-4-Hydroxylierung für hCYP3A4, hCYP2C9 1 und hCYP2D6 1, 2, 9, 10 und 17. Die Angaben für hCYP3A4 und hCYP2C9 basieren auf einer einzigen Meßreihe. Alle anderen Werte sind Mittelwerte und Standardabweichungen aus drei unabhängigen Meßreihen.** | | | | |
|---|---|---|---|---|
| **Zellinie** | **Bezug auf mg Gesamtprotein** | | **Bezug auf den CYP450-Gehalt** | |
| | **V**_{**50µM**} **pmol/mg/min** | **Cl**_{**int**} **ml/mg/min** | **Umsatzrate**_{**50µM**} **pmol/pmol/min** | **Cl**_{**int**} **ml/pmol/min** |
| **V79MZmockneo** | **0** | **0** | **0** | **0** |
| **h3A4 (Supers.)** | **ca. 1,95** | **-** | **ca. 0,10** | **-** |
| **h2C9 1 (Supers.)** | **24,08** | **482** | **0,96** | **19,3** |
| **V79MZh2D6*1** | **58,3±8,2** | **1166±165** | **2,34±0,63** | **46,8±12,6** |
| **V79MZh2D6*2** | **21,2±3,3** | **424±66** | **1,58±0,80** | **31,6±16,0** |
| **V79MZh2D6*9** | **26,2±6,5** | **524±131** | **4,09±2,23** | **81,9±44,8** |
| **V79MZh2D6*10** | **0,41±0,06** | **8,2±1,2** | **0,18±0,04** | **3,7±0,8** |
| **V79MZh2D6*17** | **6,0±1,3** | **119±27** | **0,73±0,25** | **14,6±5,0** |
| **Abkürzungen: V**_{**50µM**}**: Reaktionsgeschwindigkeit bei 50 µM Tamoxifen; Cl**_{**int**}**: intrinsische Clearance; Supers.: "Supersomen"** | | | | |

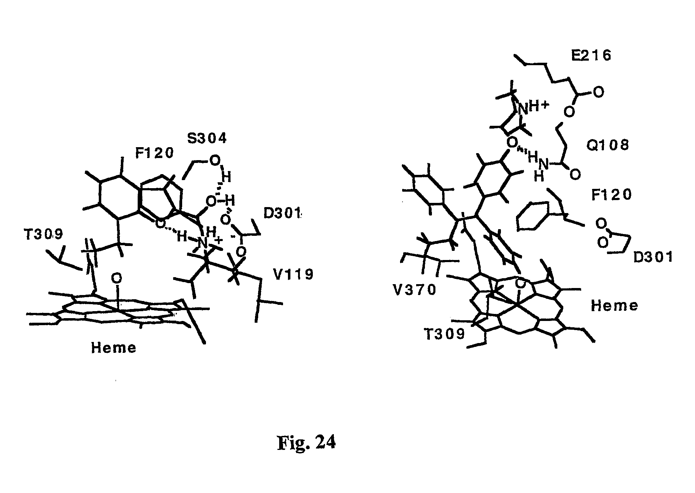

Bislang existieren keine Vergleichsdaten für die Kinetik der hCYP2D6-katalysierten Tamoxifen-4-Hydroxylierung. Mit humanen Lebermikrosomen langsamer Metabolisierer wurden bei einer Substratkonzentration von 1 µM Tamoxifen-4-Hydroxylierungs-Aktivitäten von 0,7 - 0,8 pmol/min/mg Protein und bei schnellen Metabolisierern von 1,1 - 3 pmol/min/mg Protein bestimmt (Crewe *et al*., 1997). Die Tamoxifen-Konzentration von 1 µM liegt im Bereich der Plasmakonzentrationen bei therapeutischer Dosierung (Buckley und Goa, 1989). Bei einer Substratkonzentration von 18 µM lagen die Aktivitäten für langsame Metabolisierer zwischen 6 und 8 pmol/min/mg Protein und für schnelle Metabolisierer zwischen 12 und 25 pmol/min/mg Protein (Crewe *et al*., 1997). Die Aktivitäten für V79MZh2D6*1-Homogenat lagen mit 1,2 bzw. 21 pmol/min/mg Zellprotein in denselben Bereichen. Hier fand sich wie bei der Bufuralol-Hydroxylase-Aktivität (vgl. Tabelle 11) eine Übereinstimmung der Aktivitäten zwischen V79MZh2D6*1-Zellhomogenat und humanen Lebermikrosomen. Allerdings war die Substrat-Abhängigkeit der Tamoxifen-4-Hydroxylase-Aktivität des rekombinanten hCYP2D6 stärker ausgeprägt als bei den humanen Lebermikrosomen. Außerdem wurden bei den Lebermikrosomen langsamer Metabolisierer Aktivitäten gemessen, die im Bereich derer von V79MZh2D6*2- und *9-Homogenat und über denen von V79MZh2D6*10- und *17-Homogenat lagen. Daher müssen neben hCYP2D6 weitere Isoformen mit höherer Affinität zu Tamoxifen, vermutlich hCYP2C9, an der Tamoxifen-4-Hydroxylierung *in vivo* beteiligt sein, so daß der Einfluß des hCYP2D6-Polymorphismus bei den niedrigen therapeutischen Tamoxifen-Konzentrationen *in vivo* möglicherweise überlagert wird. Dennoch zeigt der Vergleich zwischen langsamen und schnellen Metabolisierern, daß sehr wohl eine Abhängigkeit der Tamoxifen-4-Hydroxylierungsrate vom hCYP2D6-Phänotyp besteht.
Die relativen intrinsischen Clearance der hCYP2D6-katalysierten Tamoxifen- und Bufuralol-Hydroxylierung stimmen exakt überein (Tabelle 9). Die absoluten Werte sind für die (+)-Bufuralol-1'-Hydroxylierung etwa 10fach größer als für die Tamoxifen-4-Hydroxylierung. Dieser Unterschied kann durch die sehr unterschiedliche Struktur und Bindung der beiden Substrate im aktiven Zentrum von hCYP2D6 erklärt werden (vgl. Figur 24).

**Tabelle 9**

| **Vergleich der intrinsischen Clearance der Tamoxifen- und Bufuralol-Hydroxylierung mit den neuen polymorphen Zellinien V79MZh2D6*1, *2, *9, *10 und *17.** | | | | | | |
|---|---|---|---|---|---|---|
| | **intrinsische Ciearance** | **Zellinie V79MZh2D6** | | | | |
| | | ***1** | ***2** | ***9** | ***10** | ***17** |
| | **[ml/min/mg protein]** | **1166±165** | **424±66** | **524±131** | **8.2±1,2** | **119±27** |
| **Tamoxifen-4-Hydroxylierung** | | | | | | |
| | **[ml/min/pmol P450]** | **47±13** | **32±16** | **82±45** | **4±1** | **15±5** |
| | **[ml/min/mg protein]** | **12400±2580** | **4940±220** | **6670±1970** | **80±17** | **1220±160** |
| **(+)-Bufuralol-1'-Hydroxylierung** | | | | | | |
| | **[ml/min/pmol P450]** | **500±170** | **370±150** | **1040±670** | **40±10** | **150±40** |

### An der Tamoxifen-4-Hydroxylierung beteiligte Isoformen

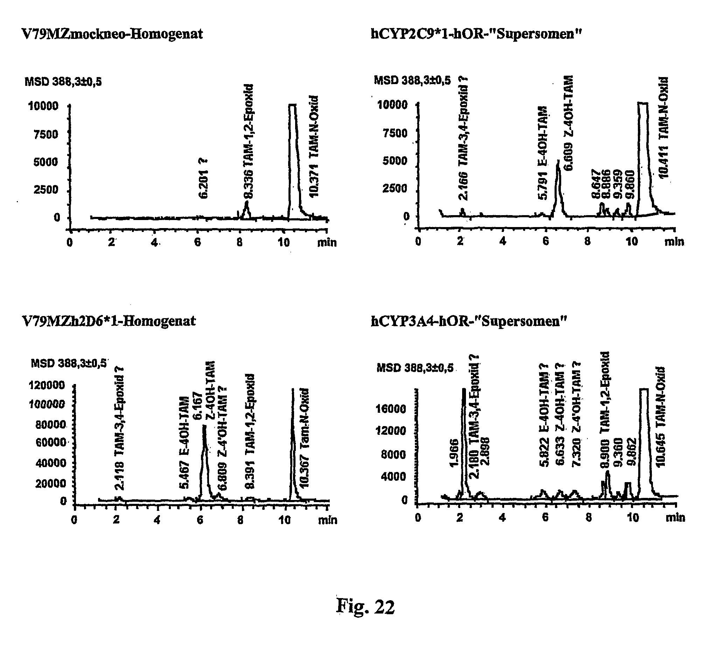

Neben hCYP2D6 wird eine Beteiligung der Isoformen hCYP3A4, hCYP2C9 und hCYP2E1 an der Tamoxifen-4-Hydroxylierung diskutiert (Crewe *et al*., 1997; Dehal und Kupfer, 1997; Styles *et al*., 1994). Um die widersprüchlichen Ergebnisse zu überprüfen und einen Hinweis auf den hCYP2D6-katalysierten Anteil der gesamten Tamoxifen-4-Hydroxylierung zu erhalten, wurden Inkubationen mit Homogenaten der Zellinien V79MZh2E1 und V79MZh3A4-hOR sowie mit hCYP3A4-hOR- und hCYP2C9*1-hOR-"Supersomen" durchgeführt (Figur 22). Die Bedingungen für diese Inkubationen wurden jedoch nicht optimiert. Die angegebenen Zahlenwerte sind daher lediglich als Richtwerte zu betrachten (Tabelle 8).

Die Tamoxifen-4-Hydroxylierung wurde von den Isoformen hCYP2D6, hCYP2C9 und hCYP3A4 katalysiert. Detektiert wurden Metaboliten mit den Massen 388,3 (monooxygenierte Metaboliten, Figur 22) und 358,3 (desmethylierte Metaboliten, nicht dargestellt). Hauptmetabolit war bei Inkubation mit hCYP2D6 und hCYP2C9 4-Hydroxy-Tamoxifen und bei Inkubation mit hCYP3A4 erwartungsgemäß *N*-Desmethyl-Tamoxifen. Die Tamoxifen-4-Hydroxylierung durch hCYP3A4 hingegen war vernachlässigbar. Dabei waren die Peakflächen von E- und Z-4-Hydroxy-Tamoxifen im Gegensatz zu den übrigen Isoformen etwa identisch und der Peak bei 2,2 min (Tamoxifen-3,4-Epoxid ?) auffallend groß.
Bei Inkubation der Zellinie V79MZh2E1 mit Homogenat wurden keine Metaboliten detektiert, die nicht auch nach Inkubation mit Homogenat der Schein-transfizierten Zellinie V79MZmockneo auftraten, nämlich Tamoxifen-*N*-oxid und *N*-Desmethyl-Tamoxifen. Beide Metaboliten waren als Verunreinigungen im Tamoxifen enthalten, allerdings in geringerer Menge als nach einer Inkubation.
Tamoxifen-*N*-oxid wurde in allen Ansätzen nachgewiesen, unabhängig von hCYP2D6 oder anderen Enzymen und auch mit Hitze-inaktiviertem Homogenat Schein-transfizierter Zellen. Die Menge war praktisch unabhängig vom Gesamtproteingehalt und der Tamoxifenkonzentration und variierte zwischen verschiedenen Meßreihen bis um den Faktor 20. Die Tamoxifen-*N*-Oxid-Eichgerade war bei Aufarbeitung der Eichlösungen in Negativkontrollansätzen mit Tamoxifen gegenüber den nicht aufgearbeiteten Eichlösungen parallel verschoben. Als Ursache dieser Beobachtungen wurde die rein chemische Oxidation des gelösten Tamoxifens, möglicherweise durch den Luftsauerstoff, angenommen. Die Dauer zwischen Inkubation und Messung der Proben würde damit die unterschiedlichen Mengen in verschiedenen Meßreihen erklären.
*N*-Desmethyl-Tamoxifen wurde ebenfalls in allen Ansätzen nachgewiesen, unabhängig von hCYP2D6 oder anderen Enzymen und auch mit Hitze-inaktiviertem Homogenat Schein-transfizierter Zellen. Im Gegensatz zu Tamoxifen-*N*-oxid war die Menge an *N*-Desmethyl-Tamoxifen jedoch sowohl von der Tamoxifen-Konzentration als auch dem Gesamtproteingehalt im Ansatz abhängig und variierte zwischen den verschiedenen Meßreihen lediglich um den Faktor 2. Eine exakte Quantifizierung war nicht möglich.

**Tabelle 10**

| **Vergleich der Beiträge unterschiedlicher Cytochrom P450-Isoformen zur Tamoxifen-4-Hydroxylierung in verschiedenen Expressionssystemen.** | | | | | | |
|---|---|---|---|---|---|---|
| **Isoform** | **V79MZhCYP- Homogenat** | **"Supersomen" rekombinanter Insektenzellen** | **Mikrosomen rekombinanter humaner B lymphoblastoider Zellen** | | | **humane Leber- mikrosomen** |
| | | | **(Dehal u. Kupfer, 1997)** | **(Styles *et al*., 1994)** | **(Crewe *et al*., 1997)** | **(Crewe *et al*., 1997)** |
| **hCYP2E1** | **-** | **n. u.** | **-** | **+** | **-** | **-** |
| **hCYP3A4** | **-** | **-** | **-** | **-** | **+** | **+** |
| **hCYP2C9*1** | **n. u.** | **+** | **-** | **-** | **+** | **+** |
| **hCYP2D6*1** | **+** | **n. u.** | **+** | **+** | **+** | **+** |
| **+: eindeutiger Beitrag zur Tamoxifen-4-Hydroxylierung; -: keine oder vernachlässigbare Tamoxifen-4- Hydroxylierung; n. u.: nicht untersucht** | | | | | | |

In Tabelle 10 werden die Resultate dieser Arbeit früheren Untersuchungen gegenübergestellt. Unter Berücksichtigung aller Ergebnisse, scheint hCYP2E1 nicht zur Tamoxifen-4-Hydroxylierung beizutragen. Ob wie von Dehal und Kupfer (1997) berichtet bei Inkubation mit V79MZh2E1-Homogenat Tamoxifen-*N*-Oxid und *N*-Desmethyl-Tamoxifen gebildet wurde, blieb wegen des hohen Hintergrundes dieser beiden Metaboliten unsicher.
Humanes Cytochrom P450 2D6 katalysierte in allen Expressionssystemen die Tamoxifen-4-Hydroxylierung.
Die Rolle von hCYP3A4 blieb hingegen unklar: Während bei Inhibitions-Studien an humanen Lebermikrosomen ein beträchtlicher Beitrag von hCYP3A4 an der Bildung von 4-Hydroxy-Tamoxifen gefunden wurde (Crewe *et al*., 1997), war die Tamoxifen-4-Hydroxylierung in allen anderen Expressions-Systemen praktisch vernachlässigbar. In Übereinstimmung mit Dehal und Kupfer (1997) und Styles *et al*. (1994) katalysierten sowohl V79MZh3A4-hOR-Homogenat als auch hCYP3A4-hOR-"Supersomen" die Tamoxifen-*N*-Desmethylierung.

Baculovirus-exprimiertes hCYP2C9*1 ("Supersomen") katalysierte die Tamoxifen-4-Hydroxylierung eindeutig. Die intrinsische Clearance war etwa halb so groß wie bei hCYP2D6 1, das in V79MZ-Zellen exprimiert wurde. Der Anteil des polymorphen hCYP2C9 am gesamten Cytochrom P450-Gehalt der Leber ist mit ca. 20 % allerdings etwa 10fach höher als derjenige von hCYP2D6. Auch wenn die Untersuchungen mit Mikrosomen rekombinanter humaner B lymphoblastoider Zellen zu widersprüchlichen Ergebnissen führten, kann daher von einem wesentlichen Beitrag von hCYP2C9 an der Tamoxifen-4-Hydroxylierung *in vivo* ausgegangen werden. Möglicherweise sind diese Widersprüche durch unterschiedliche Löungsmittel(konzentrationen) bei der Inkubation zu erklären.

### Tamoxifen-Metaboliten als hCYP2D6-Substrate

Um den Einfluß bestimmter Modifikationen am Substrat Tamoxifen, insbesondere am für die Substratbindung wichtigen Stickstoff, auf die hCYP2D6-katalysierte Hydroxylierung zu untersuchen, wurden Inkubationen mit verschiedenen Tamoxifen-Derivaten durchgeführt. Bei allen Versuchsreihen waren die relativen Umsätze der allelen Varianten etwa gleich. Dazu wurden bei den nicht standardisierten Metaboliten die relativen Peakflächen betrachtet.

### Konfigurationsisomere

Im Gegensatz zu Z-Tamoxifen wurde das Konfigurationsisomer E-Tamoxifen durch V79MZh2D6-Homogenat praktisch nicht umgesetzt.

### Z-4-Hydroxy-Tamoxifen

Der Hauptmetabolit des hCYP2D6-katalysierten Tamoxifen-Metabolismus, Z-4-Hydroxy-Tamoxifen, wurde durch V79MZh2D6-Homogenat metabolisiert. Die Metaboliten wurden anhand ihres Massenspektrums als Dihydroxy-Derivate identifiziert. Beispielsweise wurde die hCYP2D6-katalysierte *ortho*-Hydroxylierung von 4-Hydroxy-Tamoxifen zum Katechol beschrieben (Dehal und Kupfer, 1999). Eine genaue Identifizierung der Metaboliten war nicht möglich, weil keine geeigneten Standards zur Verfügung standen.
Obwohl diese Reaktion wegen der geringen *in vivo*-Konzentrationen von 4-Hydroxy-Tamoxifen quantitativ eine geringe Rolle spielen sollte, ist sie wegen der Bildung von Katecholen, die Protein-Addukte bilden können, toxikologisch interessant (Dehal und Kupfer, 1999).

### Modifikationen am Stickstoff von Tamoxifen

### Z-Tamoxifen-N-Oxid

Z-Tamoxifen-*N*-Oxid wurde zu Z-4-Hydroxy-Tamoxifen-*N*-oxid umgesetzt, allerdings nur in geringem Umfang. Ein entsprechender Standard wurde durch *N*-Oxidation von 4-Hydroxy-Tamoxifen mit Wasserstoffperoxid synthetisiert.

### Z-N-Desmethyl-Tamoxifen

Z-*N*-Desmethyl-Tamoxifen wurde gut umgesetzt. Der Hauptmetabolit besaß etwa dieselbe Retentionszeit wie Z-4-Hydroxy-Tamoxifen und das Chromatogramm sah ähnlich aus, wie nach Inkubation mit Z-Tamoxifen. Wahrscheinlich wurden Z-4-Hydroxy-*N*-Desmethyl-Tamoxifen und alle anderen Z-Tamoxifen-typischen Metaboliten in der Z-*N*-Desmethyl-Form gebildet.

### Z-N-Didesmethyl-Tamoxifen

Der Umsatz von Z-*N*-Didesmethyl-Tamoxifen war vernachlässigbar.

### Beispiel 11: Vergleich mit humanen Lebermikrosomen und gereinigtem nativen hCYP2D6

Die enzymkinetischen Eigenschaften des rekombinanten hCYP2D6 lagen im physiologischen Bereich, wie der Vergleich mit humanen Lebermikrosomen und gereinigtem *nativen* hCYP2D6 zeigt (Tabelle 11). Die (+)-Bufuralol-Hydroxylase-Aktivität des V79MZh2D6*1-Zellhomogenates von 171 ± 16 pmol/mg/min stimmt gut mit den publizierten Werten für humane Lebermikrosomen von 167 ± 43 (Kronbach *et al*., 1987) bzw. 199 ± 80 (Dayer *et al*., 1987) überein.

**Tabelle 11**

| **Vergleich der kinetischen Parameter der hCYP2D6 1-katalysierten Bufuralol-Hydroxylierung in verschiedenen Expressionssystemen. Bei Auswertungen unter Annahme einer biphasischen Michaelis-Menten-Kinetik wurden die Werte für die Isoform mit hoher Affinität und Stereoselektivität angegeben. Die Selektivität ist der Quotient aus den (-)-Bufuralol- und (+)-Bufuralol-Aktivitäten.** | | | | | | | |
|---|---|---|---|---|---|---|---|
| **Expressionssystem** (Referenz) | **Aufarbeitung** Elektronenquelle | **K**_{**M**} **µM** | **V**_{**max**} **(+) pmol/mg/min** | | **Umsatzrate 1/min** | | **Selek- tivität** |
| | | (+) | (+) | (+/-) | (+) | (+/-) | (-)/(+) |
| **V79MZh2D6*1** | **Homogenat** NADPH | **13,8±1,6** | **171±16** | **108±15** | **6,9±1,5** | **4,3±1,2** | **0,40±0,16** |
| **V79MZh2D6*1** | **Homogenat** | | | **153±26** | | | |
| (Bogni, 1999) | NADPH | | | | | | |
| **V79MZh2D6*1** | **in Kultur** | **7-8** | **170±10** | | | | |
| (Appel, 1999) | | | | | | | |
| **Humane Leber ***^{**1**} | **Mikrosomen** | **4,7±2,2** | **167±43** | | | | **0,56±0,17** |
| (Kronbach *et al*., 1987) | NADPH | | | | | | |
| **Humane Leber ***^{**2**} | **Mikrosomen** | **17,9±6,3** | **199±80** | | | | **0,49±0,09** |
| (Dayer *et al*., 1987) | NADPH | | | | | | |
| **Humane Leber***^{**2**} | **Mikrosomen** | | **715** | | | | **0,46** |
| (Zanger *et al*., 1988) | NADPH | | | | | | |
| **Humane Leber ***^{**1**} | **Mikrosomen** | | **50-2400** | | | | |
| (Gonzalez *et al*., 1988a) | NADPH | | | | | | |
| **Humane Leber***^{**2**} | **Mikrosomen** | **47,3±8,1** | | | **0,6±0,2** | | **0,48±0,15** |
| (Gut *et al*., 1986) | NADPH | | | | | | |

| **Expressionssystem** (Referenz) | **Aufarbeitung** Elektronenquelle | **K**_{**M**} **µM** | **V**_{**max**}**(+) pmol/mg/min** | | **Umsatzrate 1/min** | | **Selektivität** |
|---|---|---|---|---|---|---|---|
| | | (+) | (+) | (+/-) | (+) | (+/-) | (-)/(+) |
| **Humane Leber ***^{**1**} | **gereinigt** | **53,6±27,4** | | | **3,4±0,2** | | **0,15±0,02** |
| (Gut *et al*., 1986) | CYPOR/NADPH | | | | | | |
| **Humane Leber ***^{**1**} | **gereinigt** | **16,8** | **66333** | | **25,9** | | **0,17** |
| (Zanger *et al*., 1988) | CYPOR/NADPH | | | | | | |
| **Humane Leber ***^{**1**} | **gereinigt** | | | | **3,7-9,5** | **4,4-6,4** | **0,14-0,16** |
| (Distlerath *et al*., 1985) | CYPOR/NADPH | | | | | | |
| ***E. coli*** | **gereinigt** | **39±5 (+/-)** | | | | **1,2±0,1** | |
| (Gillam *et al*., 1995) | CYPOR/NADPH | | | | | | |
| **AHH-1 TK+/- (2D6Met/Hol)** | **Zell-Lysat** | | **68±0** | | | | |
| (Crespi *et al*., 1991) | NADPH | | | | | | |
| **AHH-1 TK+/- (h2D6Metv2)** | **Zell-Lysat** | | **126±1** | | | | |
| (Penman *et al*., 1993) | NADPH | | | | | | |
| **AHH-1 TK+1- (h2D6Metv2)** | **Mikrosomen** | **5,3** | **723±35** | | **4,5** | | **0,42** |
| (Penman *et al*., 1993) | NADPH | | | | | | |
| **AHH-1 TK+/- (h2D6Val/OR)** | **Mikrosomen** | **6,7±0,2** | | | **18,3±0,1** | | |
| (Crespi *et al*., 1995) | CYPOR/NADPH | | | | | | |
| **AHH-1 TK+/- (h2D6Val/OR)** | **Mikrosomen** | | | **550** | | **10,38** | |
| (Gentest, Produkt-Nr. M117r) | CYPOR/NADPH | | | | | | |
| **COS-1 Zellen** | **Homogenat** | | **20 - 50** | | | | |
| (Kagimoto *et al*., 1990) | NADPH | | | | | | |
| **COS-1 Zellen** | **Homogenat** | | **3,4** | | | | |
| (Johansson *et al*., 1994) | CYPOR/NADPH | | | | | | |
| **Hep G2** | **Zell-Lysat** | **4,0** | **34,1** | | **2,3** | | |
| (Tyndale *et al*., 1991a) | CuOOH | | | | | | |
| ***S*. *cerevisiae* W(R)** | **Mikrosomen** | **2** | **116** | | **10** | | |
| (Oscarson *et al*., 1997) | Yred/NADPH | | | | | | |
| **Sf9-Insektenzellen** | **Membranen** | **18,5±7,3** | **17500±2700** | | **26,2±0,4** | | **0,16±0,02** |
| (Evert *et al*., 1997) | CYPOR/NADPH | | | | | | |
| **Sf9-Insektenzelien** | **Membranen** | **55** | **500-1000** | | **0,96** | | |
| (Patten *et al*., 1996) | CYPOR/NADPH | | | | | | |
| **Sf9-Insektenzellen** | **Zell-Extrakt** | **4,7 (+/-)** | | **370** | | **12,23** | |
| (Paine *et al*., 1996) | CYPOR/NADPH | | | | | | |

| | | | | | | | |
|---|---|---|---|---|---|---|---|
| Abkürzungen:(+): (+)-Bufuralol; (-): (-)-Bufuralol; (+/-): razemisches Bufuralol; CuOOH: Cumen-Hydroperoxid; Yred: Hefe-Reduktase ^{*1} Keine Angabe über den hCYP2D6-Genotyp oder Phänotyp. | | | | | | | |
| ^{*2} Mit Spartein und/oder Debrisoquin *in vivo* als EM phänotypisiert. | | | | | | | |

In Validierungsstudien konnte eine gute Reproduzierbarkeit der Bufuralol-Hydroxylase-Aktivitäten für die polymorphen Zellinien gezeigt werden (Tabelle 11), obwohl nach unterschiedlichen Protokollen und sowohl in Kultur als auch mit Zellhomogenat gearbeitet wurde. Somit sind die Reproduzierbarkeit und Standardisierbarkeit der neuen Zellinien nachgewiesen, was von entscheidender Bedeutung für den zukünftigen Einsatz in der präklinischen Arzneimittel-Entwicklung sein wird.

Die Cytochrom P450-Gehalte der V79MZh2D6-Zellinien lagen mit bis zu etwa 25 pmol/mg Zellprotein im physiologischen Bereich von 8 - 115 pmol/mg in humanen Lebermikrosomen (Distlerath *et al.,* 1985). Ähnliche Werte werden mit anderen Säugerzellsystemen wie COS 1- Zellen (Clark und Waterman, 1991) oder humanen B lymphoblastoiden Zellen (Crespi, 1991) erreicht. Mit anderen Expressionssystemen wie Baculovirus-infizierten Insektenzellen werden wesentlich höhere Cytochrom P450-Gehalte von bis zu 800 pmol/mg Zellprotein erzielt (Evert *et al*., 1997). Die Höhe des Expressionsniveaus ist bei der Wahl des geeigneten Expressionssystems jedoch nur ein Kriterium unter vielen. Für die meisten Fragestellungen wesentlich bedeutsamer sind die experimentellen Möglichkeiten, die das Expressionssystem aufgrund seiner Biologie erlaubt.

### Literaturliste

Aklillu, E.; Persson, I.; Bertilsson, L.; Johansson, I.; Rodrigues, F.; Ingelman-Sundberg, M. (1996) Frequent distribution of ultrarapid metabolizers of debrisoquine in an Ethiopian population carrying duplicated and multiduplicated functional *CYP2D6* alleles. *J*. *Pharmacol. Exp*. *Ther*. **278**: 441-446.

Alvan, G. (1991) Clinical consequences of polymorphic drug oxidation. *Fundam*. *Clin. Pharmacol.* **5**: 209-228.

Alvan, G.; Bechtel, P.; Iselius, L.; Gundert-Remy, U. (1990) Hydroxylation polymorphisms of debrisoquine and mephenytoin in European populations. *Eur*. *J*. *Clin*. *Pharmacol*. **39**: 533-537.

Alvan, G.; Grind, M.; Graffner, C.; Sjoqvist, F. (1984) Relationship of N-demethylation of amiflamine and its metabolites to debrisoquine hydroxylation polymorphism. *Clin*. *Pharmacol. Ther.* **36**: 515-519.

Aoyama, T.; Yamano, S.; Guzelian, P. S.; Gelboin, H. V.; Gonzalez, F. J. (1990) Five of 12 forms of vaccinia virus-expressed human hepatic cytochrome P450 metabolically activate aflatoxin B1. *Proc. Natl. Acad*. *Sci*. *USA* **87**: 4790-4793.

Appel, K. (1999) PHARMBIODYN GmbH, Denzlingen. *persönliche Mitteilung.*

Armstrong, M.; Fairbrother, K.; Idle, J. R.; Daly, A. K. (1994) The cytochrome P450 CYP2D6 allelic variant CYP2D6J and related polymorphisms in a European population. *Pharmacogenetics* **4**: 73-81.

Asseffa, A.; Smith, S. J.; Nagata, K.; Gillette, J.; Gelboin, H. V.; Gonzalez, F. J. (1989) Novel exogenous heme-dependent expression of mammalian cytochrome P450 using baculovirus. *Arch. Biochem. Biophys.* **274**: 481-490.

Bames, H. J.; Jenkins, C. M.; Waterman, M. R. (1994) Baculovirus expression of bovine cytochrome P450c17 in Sf9 cells and comparison with expression in yeast, mammalian cells, and *E. coli. Arch. Biochem. Biophys.* **315**: 489-494.

Becker, N. und Wahrendorf, J. (1981-1990). Brust. *Krebsatlas der Bundesrepublik Deutschland.* 3. Ausgabe. Heidelberg: Springer Verlag, 366-371.

Bertilsson, L. (1995) Geographical/interracial differences in polymorphic drug oxidation. Current state of knowledge of cytochromes P450 (CYP) 2D6 and 2C19. *Clin*. *Pharmacokin*. **29**: 192-209.

Bertilsson, L.; Dahl, M. L.; Sjoqvist, F.; Aberg-Wistedt, A.; Humble, M.; Johansson, I.; Lundqvist, E.; Ingelman-Sundberg, M. (1993) Molecular basis for rational megaprescribing in ultrarapid hydroxylators of debrisoquine. *The Lancet* **341**: 363.

Bertilsson, L.; Lou, Y. Q.; Du, Y. L.; Liu, Y.; Kuang, T. Y.; Liao, X. M.; Wang, K. Y.; Reviriego, J.; Iselius, L.; Sjoqvist, F. (1992) Pronounced differences between native Chinese and Swedish populations in the polymorphic hydroxylations of debrisoquin and S-mephenytoin. *Clin*. *Pharmacol*. *Ther*. **51**: 388-397.

Bichara, N.; Ching, M. S.; Blake, C. L.; Ghabrial, H.; Smallwood, R. A. (1996) Propranolol hydroxylation and *N*-desisopropylation by cytochrome P4502D6. *Drug Metab. Dispos.* **24**: 112-118.

Blech, J. (1999) Die ganz private Pille. *Die Zeit* **22**: 42.

Bloomer, J. C.; Woods, F. R.; Haddock, R. E.; Lennard, M. S.; Tucker, G. T. (1992) The role of cytochrome P4502D6 in the metabolism of paroxetine by human liver microsomes. *Clin*. *Pharmacol.* **33**: 521-523.

Bogni, A. (1999) European Centre for the Validation of Alternative Methods (ECVAM), Ispra, Italien. *persönliche Mitteilung*.

Boobis, A. R.; Murray, S.; Hampden, C. E.; Davies, D. S. (1985) Genetic polymorphism in drug oxidation: in vitro studies of human debrisoquine 4-hydroxylase and bufuralol 1'hydroxylase activities. *Biochem. Pharmacol*. **34**: 65-71.
Borgna, J.-L. and Rochefort, H. (1981) Hydroxylated metabolites of tamoxifene are formed *in vivo* and bound to estrogen receptor target tissues. *J*. *Biol*. *Chem.* **256**: 859-868.

Bradley, M. O.; Bhuyan, B.; Francis, M. C.; Langenbach, R.; Peterson, A.; Huberman, E. (1981) Mutagenesis by chemical agents in V79 Chinese hamster cells. *Mutat. Res.* **87**: 81-142.

Bradwell, L. (1989) The mutagenic and carcinogenic effects of gene transfer. *Mutagenesis* **4**: 245-253.

Broly, F.; Marez, D.; Lo Guidice, J. M.; Sabbagh, N.; Legrand, M.; Boone, P.; Meyer, U. A. (1995a) A nonsense mutation in the cytochrome P450 CYP2D6 gene identified in a Caucasian with an enzyme deficiency. *Hum. Genet.* **96**: 601-603.

Broly, F.; Marez, D.; Sabbagh, N.; Legrand, M.; Millecamps, S.; Lo Guidice, J.-M.; Boone, P.; Meyer, U. A. (1995b) An efficient strategy for detection of known and new mutations of the *CYP2D6* gene using single strand conformation polymorphism analysis. *Pharmacogenetics* **5**: 373-384.

Broly, F. and Meyer, U. A. (1993) Debrisoquine oxidation polymorphism: phenotypic consequences of a 3-base-paar deletion in exon 5 of the *CYP2D6* gene. *Pharmacogenetics* **3**: 123-130.

Brosen, K. and Gram, L. F. (1989a) Clinical significance of the sparteine/debrisoquine oxidation polymorphism. *Eur*. *J*. *Clin. Pharmacol*. **36**: 537-547.

Brosen, K. and Gram, L. F. (1989b) Clinical significance of the sparteine/debrisoquine oxidation polymorphism. *Eur*. *J*. *Clin*. *Pharmacol.* **36**: 537-547.

Brosen, K.; Otton, V.; Gram, L. F. (1986) Imipramine demethylation and hydroxylation: Impact ofthe sparteine oxidation phenotype. *Clin*. *Pharmacol*. *Ther*. **40**: 543-549.

Buckley, M. T. and Goa, K. L. (1989) Tamoxifen: a reappraisal of its pharmacodynamic and pharmacokinetic properties, and therapeutic use. *Clin*. *Pharmacol*. **37**: 451-490.

Burnette, W. N. (1981) Western-Blotting. *Analyt. Biochem.* **112**: 195-203.

Busby, W. F. J.; Ackermann, J. M.; Crespi, C. L. (1999) Effect of methanol, ethanol, dimethyl sulfoxide, and acetonitrile on in vitro activities of cDNA-expressed human cytochromes P-450. *Drug Metab*. *Dispos.* **27**: 246-249.

Buters, J. T. M.; Korzekwa, K. R.; Kunze, K. L.; Omata, Y.; Hardwick, J. P.; Gonzalez, F. J. (1994) cDNA-directed expression of human cytochrome P450 CYP3A4 using baculovirus. *Drug Metab. Dispos.* **22**(5): 688-692.

Butner, K. A. and Lo, C. W. (1986) High frequency rearrangements associated with mouse centromeric satellite DNA. *J*. *Mol. Biol.* **187**: 547-556.

Calos, M. P.; Lebkowski, J. S.; Botchan, M. R. (1983) High mutation frequency in DNA transfected into mammalian cells. *Proc. Natl*. *Acad*. *Sci*. *USA* **80**: 3015-3019.

Carcillo, J. A.; Parise, R. A.; Adedoyin, A.; Frye, R.; Branch, R. A.; Romkes, M. (1996) CYP2D6 mRNA expression in circulating peripheral blood mononuclear cells correlates with in vivo debrisoquine hydroxylase activity in extensive metabolizers. *Res. Commun. Mol*. *Pathol*. *Pharmacol.* **91**: 149-159.

Chance, B. (1957). Techniques for the Assay of the Respiratory Enzymes. In: *Methods in Enzymology*, Colowick, S. P. and Kaplan, N. O. (eds.). New York: Academic Press, **IV**: 273-329.

Chauret, N.; Gauthier, A.; Nicoll-Griffith, D. A. (1998) Effect of common solvents on in vitro cytochrome P450-mediated metabolic activities in human liver microsomes. *Drug Metab*. *Dispos*. **26**: 1-4.

Chu, E. H. Y. and Malling, H. V. (1968) Mammalian cell genetics II.Chemical induction of specific locus mutations in Chinese hamster cells in vitro. *Proc*. *Natl. Acad*. *Sci*. **61**: 1306-1312.

Clark, B. J. and Waterman, M. R. (1991). Heterologous expression of mammalian P450 in COS cells. In: *Methods in Enzymology*, Waterman, M. R. and Johnson, E. F., (eds.). New York: Academic Press, **206**: 100-108.
Cooper, R. G.; Evans, D. A. P.; Price, A. H. (1987) Studies on the metabolism of perhexiline in man. *Eur. J*. *Clin. Pharmacol.* **32**: 569-576.

Crespi, C. L. (1991). Expression of cytochrome P450 cDNAs in human B lymphoblastoid cells: applications to toxicology and metabolite analysis. In: *Methods in Enzymology,* Waterman, M. R. and Johnson, E. F., (eds.). New York: Academic Press, **206**: 123-129.

Crespi, C. L.; Penman, B. W.; Gelboin, H. V.; Gonzalez, F. J. (1991) A tobacco smokederived nitrosamine, 4-(methylnitrosamino)-1-(3-pyridyl)-1-butanone, is activated by multiple human cytochrome P450s including the polymorphic human cytochrome P4502D6. *Carcinogenesis* **12**: 1197-1201.

Crespi, C. L.; Steimel, D. T.; Penman, B. W.; Korzekwa, K. R.; Fernandezsalguero, P.; Buters, J. T. M.; Gelboin, H. V.; Gonzalez, F. J.; Idle, J. R.; Daly, A. K. (1995) Comparison of substrate metabolism by wild type CYP2D6 protein and a variant containing methionine, not valine, at position 374. *Pharmacogenetics* **5**: 234-243.

Crewe, H. K.; Ellis, S. W.; Lennard, M. S.; Tucker, G. T. (1997) Variable contribution of cytochrome P450 2D6, 2C9 and 3A4 to the 4-hydroxylation of tamoxifen by human liver microsomes. *Biochem. Pharmacol.* **53**: 171-178.

Dahl, M.; Johansson, I.; Bertilsson, L.; Ingelman-Sundberg, M.; Sjöqvist, F. (1995a) Ultrarapid hydroxylation of debrisoquine in a Swedish population. Analysis ofthe molecular genetic basis. *J*. *Pharmacol*. *Exp. Ther.* **274**: 516-520.

Dahl, M. L. and Bertilsson, L. (1993) Genetically variable metabolism of antidepressants and neuroleptic drugs in man. *Pharmacogenetics* **3**: 61-70.

Dahl, M. L.; Yue, Q. Y.; Roh, H. K.; Johansson, I.; Sawe, J.; Sjoqvist, F.; Bertilsson, L. (1995b) Genetic analysis of the *CYP2D* locus in relation to debrisoquine hydroxylation capacity in Korean, Japanese and Chinese subjects. *Pharmacogenetics* **5**: 159-164.

Daly, A. K. (1995) Molecular basis of polymorphic drug metabolism. *J*. *Mol. Med*. **173**: 539-553.

Daly, A. K.; Brockmöller, J.; Broly, F.; Eichelbaum, M.; Evans, W. E.; Gonzalez, F. J.; Huang, J.-D.; Idle, J. R.; Ingelman-Sundberg, M.; Ishizaki, T.; Jacqz-Aigrain, E.; Meyer, U. A.; Nebert, D. W.; Steen, V. M.; Wolf, C. R.; Zanger, U. M. (1996a) Nomenclature for human *CYP2D6* alleles. *Pharmacogenetics* **6**: 193-201.

Daly, A. K.; Fairbrother, K. S.; Andreasson, O. A.; London, S. J.; Idle, J. R.; Steen, V. M. (1996b) Characterization and PCR-based detection oftwo different hybrid *CYP2D7*/*CYP2D6* alleles associated with the poor metabolizer phenotype. *Pharmacogenetics* **6**: 231-240.

Daly, A. K.; Leathart, J. B. S.; London, S. J.; Idle, J. R. (1995) An inactive cytochrome P450 *CYP2D6* allele containing a deletion and a base substitution. *Human Genet.* **95**: 337-341.

Dayer, P.; Kronbach, T.; Eichelbaum, M.; Meyer, U. A. (1987) Enzymatic basis of the debrisoquine/sparteine-type genetic polymorphism of drug oxidation: characterization of bufuralol 1'-hydroxylation in liver microsomes of *in vivo* phenotyped carriers of the genetic deficiency. *Biochem. Pharmacol.* **260**: 4145-4152.

De Groene, E. M.; Hassing, I. G. M.; Blom, M. J.; Seinen, W.; Fink-Gremmels, J.; Horbach, G. J. (1996) Development of human cytochrome P450-expressing cell lines: application in mutagenicity of ochratoxin A. *Cancer Res*. **56**: 299-304.

De Groot, M. J. and Vermeulen, N. P. E. (1997) Modeling the active sites of cytochrome P450s and glutathione S-transferases, two of the most important biotransformation enzymes. *Drug Metab. Rev.* **29**: 747-799.

De Matteis, F.; White, I. N. H.; Smith, L. L. (1998) Species differences in the metabolic activation of tamoxifen into genotoxic derivatives: risk assessment in woman. *Eur. J*. *Drug Metab. Pharmacokin.* **23**: 425-428.

Dehal, S. S. and Kupfer, D. (1996) Evidence that the catechol 3,4-dihydroxy tamoxifen is a proximate intermediate to the reactive species binding covalently to proteins. *Cancer Res.* **56**: 1283-1290.

Dehal, S. S. and Kupfer, D. (1997) CYP2D6 catalyzes tamoxifen-4-hydroxylation in human liver. *Cancer Res.* **57**: 3402-3406.

Dehal, S. S. and Kupfer, D. (1999) Cytochrome P-450 3A and 2D6 catalyze *ortho* hydroxylation of 4-hydroxytamoxifen and 3-hydroxytamoxifen (droloxifen) yielding tamoxifen catechol:. involvement of catechols in covalent binding to hepatic proteins. *Drug Metab*. *Dispos*. **27**: 681-688.

Distlerath, L. M.; Reilly, P. E. B.; Martin, M. V.; Davis, G. G.; Wilkinson, G. R.; Guengerich, F. P. (1985) Purification and characterization ofthe human liver cytochromes P-450 involved in debrisoquine 4-hydroxylation and phenacetin O-deethylation, two prototypes for genetic polymorphisms in oxidative drug metabolism. *J*. *Biol. Chem.* **260**: 9057-9067.

Doehmer, J. (1993) V79 Chinese hamster cells genetically engineered for cytochrome P450 and their use in mutagenicity and metabolism studies. *Toxicology* **82**: 105-118.

Doehmer, J.; Dogra, S.; Friedberg, T.; Monier, S.; Adesnik, M.; Glatt, H.; Oesch, F. (1988) Stable expression of rat cytochrome P-450IIB1 cDNA in Chinese hamster cells (V79) and metaholic activation of aflatoxin B₁. *Proc*. *Natl*. *Acad*. *Sci*. *USA* **85**: 5769-5773.

Doepker, C. L.; Livingston, G. K.; Schumann, B. L.; Srivastava, A. K. (1998) Structural and numerical chromosomal aberrations in a metabolically competent human lymphoblast cell line (MCL-5). *Mutagenesis* **13**(3): 275-280.

Dogra, S.; Doehmer, J.; Glatt, H.; Mölders, H.; Siegert, P.; Friedberg, T.; Seidel, A; Oesch, F. (1990) Stable expression of rat cytochrome P-450IA1 cDNA in V79 Chinese hamster cells and their use in mutagenicity testing. *Mol*. *Pharmacol*. **37**: 608-613.

Droll, K.; Bruce-Mensah, K.; Otton, S. V.; Gaedigk, A.; Sellers, E. M.; Tyndale, R. F. (1998) Comparison of three CYP2D6 probe substrates and genotype in Ghanians, Chinese and Caucasians. *Pharmacogenetics* **8**: 325-333.

Eichelbaum, M.; Baur, M. P.; Dengler, H. J.; Osikowska-Evers, B. O.; Tieves, G.; Zekorn, C.; Rittner, C. (1987) Chromosomal assignment of human cytochrome P-450 (debrisoquine/sparteine type) to chromosome 22. *Br*. *J*. *Clin. Pharmacol*. **23**: 455-458.

Eichelbaum, M.; Bertilsson, L.; Sawe, J.; Zekorn, C. (1982) Polymorphic oxidation of sparteine and debrisoquine: related pharmacogenetic entities. *Clin. Pharmacol*. *Ther*. **31**: 184-186.

Eichelbaum, M. and Gross, A. S. (1990) The genetic polymorphism of debrisoquine/sparteine metabolism - clinical aspects. *Pharmac. Ther*. **46**: 377-394.

Eichelbaum, M. and Gross, A. S. (1992). In: *Pharmacogenetics of drug metabolism,* Kalow, W., (ed.). New York: Pergamon Press, Inc..

Eichelbaum, M.; Spannbrucker, N.; Dengler, H. J. (1979a) Influence of the defective metabolism of sparteine on its pharmacokinetics. *Eur*. *J*. *Clin. Pharmacol*. **16**: 189-194.

Eichelbaum, M.; Spannbrucker, N.; Steincke, B.; Dengler, H. J. (1979b) Defective N-oxidation of sparteine in man: a new pharmacogenetic defect. *Eur*. *J*. *Clin. Pharmacol.* **16**: 183-187.

Eichelbaum, M. and Woolhouse, N. M. (1985) Interethnic differences in sparteine oxidation among Ghanaians and Germans. *Eur. J. Clin. Pharmacol*. **28**: 79-83.

Ellis, S. W.; Ching, M. S.; Watson, P. F.; Hendersson, C. J.; Simula, A. P.; Lennard, M. S.; Tucker, G. T.; Woods, H. F. (1992) Catalytic activities of human debrisoquine 4-hydroxylase cytochrome P450 (CYP2D6) expressed in yeast. *Biochem. Pharmacol.* **44**: 617-620.

Ellis, S. W.; Hayhurst, G. P.; Smith, G.; Lightfoot, T.; Wong, M. M. S.; Simula, A. P.; Ackland, M. J.; Sternberg, M. J. E.; Lennard, M. S.; Tucker, G. T.; Wolf, C. R. (1995) Evidence that aspartic acid 301 is a critical substrate-contact residue in the active site of cytochrome P450 2D6. *J*. *Biol. Chem*. **270**: 29055-29058.

Estabrook, R. W.; Peterson, J.; Baron, J.; Hildebrandt, A. (1972). The spectrophotometric measurement of turbid suspensions of cytochromes associated with drug metabolism. In: *Methods in Pharmacology,* Chigell, C. F., (ed.). New York (u.a.): Plenum Press, **2**: 303-350.

Evans, D. A.; Mahgoub, A.; Sloan, T. P.; Idle, J. R.; Smith, R. L. (1980) A family and population study of the genetic polymorphism of debrisoquine oxidation in a white British population. *J*. *Med*. *Genet*. **17**: 102-105.

Evans, W. E.; Relling, M. V.; Rahman, A.; McLeod, H. L.; Scott, E. P.; Lin, J. S. (1993) Genetic basis for a lower prevalence of deficient CYP2D6 oxidative drug metabolism phenotypes in black Americans. *J*. *Clin*. *Invest*. **91**: 2150-2154.

Evert, B.; Eichelbaum, M.; Haubruck, H.; Zanger, U. M. (1997) Functional properties of CYP2D6 1 (wildtype) and CYP2D6 7 (His324Pro) expressed by recombinant baculovirus in insect cells. *Naunyn-Schmiedeberg's Arch*. *Pharmacol.* **355**: 309-318.

Evert, B.; Griese, E.-U.; Eichelbaum, M. (1994a) Cloning and sequencing of a new nonfunctional *CYP2D6* allele: deletion of T1795 in exon 3 generates a premature stop codon. *Pharmacogenetics* **4**: 271-274.

Evert, B.; Griese, E.-U.; Eichelbaum, M. (1994b) A missense mutation in exon 6 of the *CYP2D6* gene leading to a histidine to proline exchange is associated with the poor metabolizer phaenotype of sparteine. *Naunyn-Schmiedeberg's Arch. Pharmacol.* **350**: 434-439.

Fiers, W.; Contreras, R. R.; Haegeman, G.; Rogiers, R.; De Voorde, A.; van Heuverswyn, H.; Van Herreweghe, J.; G., V.; M., Y. (1978) Complete nucleotide sequence of SV40 DNA. *Nature* **273**: 113-120.

Fischer, V.; Vogels, B.; Maurer, G.; Tynes, R. E. (1992) The antipsychotic clozapine is metabolized by the polymorphic human microsomal and recombinant cytochrome P450 2D6. *J*. *Pharmacol*. *Exp*. *Ther*. **260**: 1355-1360.

Fonne-Fister, R.; Bargetzi, M. J.; Meyer, U. A. (1987) MPTP, the neurotoxin inducing Parkinson's disease, is a potent competitive inhibitor of human and rat cytochrome P450 isozymes (P450bufI, P450db1) catalyzing debrisoquine 4-hydroxylation. *Biochem. Biophys*. *Res.* **148**: 1144-1150.

Ford, D. K. and Yerganian, G. (1958) Observations on the chromosomes of Chinese hamster cells in tissue culture. *J*. *Natl. Cancer Inst.* **21**: 393-425.

Furr, B. J. A. and Jordan, V. C. (1984) The pharmacology and clinical uses of tamoxifen. *Pharmac. Ther*. **25**: 127-205.

Gaedigk, A.; Blum, M.; Gaedigk, R.; Eichelbaum, M.; Meyer, U. A. (1991) Deletion of the entire cytochrome P450 gene as a cause of impaired drug metabolism on poor metabolizers of the debrisoquine/sparteine polymorphism. *Am. J*. *Hum. Genet*. **48**: 943-950.

Garfinkel, D. (1958) Isolation and properties of cytochrome b5 from pig liver. *Arch. Biochem. Biophys.* **77**: 111-120.

Gillam, E. M. J.; Guo, Z.; Martin, M. V. M.; Jenkins, C. M.; Guengerich, F. P. (1995) Expression of cytochrome P450 2D6 in *Escherichia coli,* purification, and spectral and catalytic characterization. *Arch. Biochem. Biophys.* **319**: 540-550.

Glatt, H.; Davis, W.; Meinl, W.; Hermersdorfer, H.; Venitt, S.; Phillips, D. H. (1998) Rat, but not human, sulfotransferase activates a tamoxifen metabolite to produce DNA adducts and gene mutations in bacteria and mammalian cells in culture. *Carcinogenesis* **19**: 1709-1713.

Glatt, H.; Gemperlein, I.; Turchi, G.; Heinritz, H.; Doehmer, J.; Oesch, F. (1987) Search for cell culture systems with diverse xenobiotic-metabolizing activities and their use in toxicological studies. *Mol. Toxicol*. **1**: 313-334.

Glatt, H. R.; Gemperlein, I.; Setiabudi, F.; Platt, K. L.; Oesch, F. (1990) Expression of xenobiotic metabolizing enzymes in propagable cell cultures and induction of micronuclei by 13 compounds. *Mutagenesis* **5**: 241-249.

Gonzalez, F. J. and Korzekwa, K. R. (1995) Cytochromes P450 expression systems. *Annu. Rev*. *Pharmocol. Toxicol.* **35**: 369-390.

Gonzalez, F. J.; Matsunaga, T.; Nagata, K.; Meyer, U.; Nebert, D. W.; Pastewka, J.; Kozak, C. A.; Gillette, J.; Gelboin, H. V.; Hardwick, J. P. (1987) Debrisoquine 4-hydroxylase: characterization of a new P450 gene subfamily, regulation, chromosomal mapping, and molecular analysis in the DA rat polymorphism. *DNA* **6**: 149-161.

Gonzalez, F. J.; Skoda, R. C.; Kimura, S.; Umeno, M.; Zanger, U. M.; Nebert, D. W.; Gelboin, H. V.; Hardwick, J. P.; Meyer, U. A. (1988a) Characterization of the common genetic defect in humans deficient in debrisoquine metabolism. *Nature* **331**: 442-446.

Gonzalez, F. J.; Skoda, R. C.; Kimura, S.; Umeno, M.; Zanger, U. M.; Nebert, D. W.; Gelboin, H. V.; Hardwick, J. P.; Meyer, U. A. (1990) Molecular characterization of the common human deficiency in metabolism of debrisoquine and other drugs. *Nature* **331**: 442-446.

Gonzalez, F. J.; Vilbois, F.; Hardwick, J. P.; McBride, O. W.; Nebert, D. W.; Gelboin, H. V.; Meyer, U. A. (1988b) Human debrisoquine 4-hydroxylase (P450IID6): cDNA and deduced amino acid sequence and assignment of the CYP2D6 locus to chromosome 22. *Genomics* **2**: 174-179.

Gotoh, O. (1991) Substrate recognition sites in cytochrome P450 family 2 (CYP2). Proteins inferred from comparative analysis of amino acid and encoding nucleotide sequences. *J*. *Biol*. *Chem*. **267**: 83-90.

Gough, A. C.; Miles, J. S.; Spurr, N. K.; Moss, J. E.; Gaedigk, A.; Eichelbaum, M.; Wolf, C. R. (1990) Identification of the primary gene defect at the cytochrome P450 *CYP2D6* locus. *Nature* **347**: 773-776.

Gough, A. C.; Smith, C. A.; Howell, S. M.; Wolf, C. R.; Bryant, S. P.; Spurr, N. K. (1993) Localization of the CYP2D gene locus to human chromosome 22q13.1 by polymerase chain reaction, *in situ* hybridization, and linkage analysis. *Genomics* **15**: 430-432.

Graham, F. L. and Van der Eb, A. J. (1973) A new technique for the assay of infectivity of human adenovirus 5 DNA. *Virology* **52**: 456-467.

Greim, H. und Deml, E. H. (Hrsg.) (1996). *Toxikologie. Eine Einführung für Naturwissenschaftler und Mediziner*. Weinheim: VCH.

Griese, E.-U.; Zanger, U. M.; Brudermanns, U.; Gaedigk, A.; Mikus, G.; Morike, K.; Stuven, T.; Eichelbaum, M. (1998) Assessment of the predictive power of genotypes for the *in-vivo* catalytic function of CYP2D6 in a German population. *Pharmacogenetics* **8**: 15-26.

Gross, A. S.; Phillips, A. C.; Rieutord, A.; Shenfield, G. M. (1996) The influence of the sparteine/debrisoquine genetic polymorphism on the disposition of dexfenfluramine. *Br*. *J*. *Clin*. *Pharmacol*. **41**: 311-317.

Guidice, J. M.; Marez, D.; Sabbagh, N.; Legrand-Andreoletti, M.; Spire, C.; Alcaide, E.; Lifitte, J. J.; Broly, F. (1997) Evidence for CYP2D6 expression in human lung. *Biochem. Biophys. Res.* **241**: 79-85.

Gut, J.; Catin, T.; Dayer, P.; Kronbach, T.; Zanger, U.; Meyer, U. A. (1986) Debrisoquine/sparteine-type polymorphism of drug oxidation: purification and characterization of two functionally different human liver cytochrome P-450 isoenzymes involved in impaired hydroxylation of the prototype substrate bufuralol. *J*. *Biol*. *Chem.* **261**: 11734-11743.

Halliday, R. C.; Jones, B. C.; Park, B. K.; Smith, D. A. (1997) Synthetic strategies to lower affinity for CYP2D6. *Eur. J*. *Drug Metab. Pharmacokin.* **22**: 291-294.

Hickman, D.; Wang, J.-P.; Wang, Y.; Unadkat, J. D. (1998) Evaluation of the selectivity of *in vitro* probes and suitability of organic solvents for the measurement of human cytochrome P450 monooxygenase activities. *Drug Metab*. *Dispos.* **26**: 207-215.

Higashi, Y.; Hiromasa, T.; Tanae, A.; Miki, T.; Nakura, J.; Kondo, T.; Ohura, T.; Ogawa, E.; Nakayama, K.; Fujii-Kuriyama, Y. (1991) Effects of individual mutations in the P-450(C21) pseudogene on the P-450(C21) activity and their distribution in the patient genomes of congenital steroid 21-hydroxylase deficiency. *J*. *Biochem. (Tokyo)* **109**: 638-644.

Hiroi, T.; Imaoka, S.; Funae, Y. (1998) Dopamine formation from tyramine by CYP2D6. *Biochem. Biophys. Res*. *Commun.* **249**: 838-843.

Hogstedt, S.; Lindberg, B.; Rane, A. (1983) Increased oral clearance of metoprolol in pregnancy. *Eur. J*. *Clin*. *Pharmacol*. **24**: 217-220.

Horai, Y.; Nakano, M.; Ishizaki, T.; Ishikawa, K.; Zhou, H. H.; Zhou, B. I.; Liao, C. L.; Zhang, L. M. (1989) Metoprolol and mephenytoin oxidation polymorphisms in Far Eastem Oriental subjects: Japanese versus mainland Chinese. *Clin*. *Pharmacol. Ther*. **46**: 198-207.

Huang, Z.; Fasco, M. J.; Figge, H. L.; Keyomarsi, K.; Kaminsky, L. S. (1996) Expression of cytochromes P450 in human breast tissue and tumors. *Drug Metab*. *Dispos.* **24**: 899-905.

Huang, Z.; Fasco, M. J.; Kaminsky, L. S. (1997) Alternative splicing of CYP2D mRNA in human breast tissue. *Arch. Biochem. Biophys.* **343**: 101-108.

IARC (1996). Some pharmaceutical drugs: anti-estrogenic compounds. Tamoxifen. *IARC monographs on the evaluation of carcinogenic risks to humans.* WHO, **66**: 253-365.

Ingelman-Sundberg, M.; Oscarson, M.; Persson, I.; Masimirembwa, C. M.; Dahl, M.-L.; Bertilsson, L.; Sjöqvist, F.; Johansson, I. (1995). Genetic polymorphism of human drug metabolizing enzymes. Recent aspects on polymorphic forms of cytochromes P450. *European conference on specificity and variability in drug metabolism,* Luxembourg, EU.

Jaenisch, R. and Jahner, D. (1984) Methylation, expression and chromosomal position of genes in mammals. *Biochem. Biophys. Acta* **782**: 179-189.

Johansson, I.; Lundqvist, E.; Bertilsson, L.; Dahl, M.-L.; Sjöqvist, F.; Ingelman-Sundberg, M. (1993) Inherited amplification of an active gene in the cytochrome P450 *CYP2D* locus as a cause of ultrarapid metabolism of debrisoquine. *Proc*. *Natl. Acad*. *Sci*. *USA* **90**: 11825-11829.

Johansson, I.; Oscarson, M.; Yue, Q.-Y.; Bertilsson, L.; Sjöqvist, F.; Ingelman-Sundberg, M. (1994) Genetic analysis of the Chinese cytochrome P4502D locus: characterization of variant *CYP2D6* genes present in subjects with diminished capacity for debrisoquine hydroxylation. *Mol*. *Pharmacol.* **46**: 452-459.

Jordan, V. C. (1997) Tamoxifen: the herald of a new era of preventive therapeutics. *J*. *Nat*. *Cancer Inst*. **89**: 747-749.

Jordan, V. C. (1998) Designer estrogens. *Scientific American* **Okt.**: 60-67.

Jordan, V. C. and Allen, K. E. (1980) Evaluation of the antitumor activity of the nonsteroidal anti-estrogen monohydroxytamoxifen in the DMBA-induced rat carcinoma model. *Eur*. *J*. *Cancer Clin*. *Oncol*. **16**: 239-251.

Jordan, V. C. and Chem, C. (1982) Metabolites of tamoxifen in animals and man: identification, pharmacology, and significance. *Breast Cancer Res. and Treatment* **2**: 123-138.

Jordan, V. C. and Robinson, S. P. (1987) Species-specific pharmacology of antiestrogens: role of metabolism. *Federation proceedings* **46**: 1870-1874.

Kagimoto, M.; Heim, M.; Kagimoto, K.; Zeugin, T.; Meyer, U. A. (1990) Multiple mutations ofthe human cytochrome IID6 gene (*CYP2D6*) in poor metabolisers of debrisoquine. *J*. *Biol. Chem*. **265**: 17209-17214.

Kempf, A. C.; Zanger, U. M.; Meyer, U. A. (1995) Truncated human P450 2D6: expression in *Escherichia coli*, Ni(2+)-chelate affinity purification, and characterization of solubility and aggregation. *Arch. Biochem. Biophys.* **321**: 277-288.

Kiefer, F. and Wiebel, F. J. (1989) V79 Chinese hamster cells express cytochrome P-450 activity after simultaneous exposure to polycyclic aromatic hydrocarbons and aminophylline. *Toxicol*. *Letters* **48**: 265-273.

Kimura, S.; Umeno, M.; Skoda, R. C.; Meyer, U. A.; Gonzalez, F. J. (1989) The human debrisoquine 4-hydroxylase (CYP2D) locus: sequence and identification of the polymorphic CYP2D6 gene, a related gene, and a pseudogene. *Am*. *J*. *Hum. Genet.* **45**: 889-904.

Kivistö, K. T.; Griese, E.-U.; Stüven, T.; Fritz, P.; Friedel, G.; Kroemer, H. K.; Zanger, U. M. (1997) Analysis of CYP2D6 expression in human lung: implications for the association between CYP2D6 activity and susceptibility to lung cancer. *Pharmacogenetics* **7**: 295-302. Klingenberg, M. (1958) Pigments of rat liver microsomes. *Arch. Biochem. Biophys.* **75**: 376-386.

Kozak, M. (1987) At least six nucleotides preceding the AUG initiator codon enhance translation in mammalian cells. *J*. *Mol*. *Biol*. **196**: 947-950.

Kozak, M. (1990) Downstream secondary structure facilitates recognition of initiator codons by eukaryotic ribosomes. *Proc*. *Natl*. *Acad* .*Sci*. *USA*(87): 8301-8305.

Kroemer, H. K. and Eichelbaum, M. (1995) "It's the genes, stupid". Molecular bases and clinical consequences of genetic cytochrome P450 2D6 polymorphism. *Life Sci*. **56**: 2285-2298.

Kronbach, T. (1991) Bufuralol, dextromethorphan, and debrisoquine as prototype substrates for human P450IID6. *Methods in Enzymology* **206**: 509-517.

Kronbach, T.; Mathys, D.; Gut, J.; Catin, T.; Meyer, U. A. (1987) High-performance liquid chromatographic assay fot bufuralol 1'-hydroxylase, debrisoquine 4-hydroxylase, and dextromethorphan O-demethylation in microsomes and purified cytochrome P-450 isozymes of human liver. *Analytical Biochemistry* **162**: 24-32.

Krynetski, E. Y.; Drutsa, V. L.; Kovaleva, I. E.; Luzikov, V. N. (1995) High yield expression of functionally active human liver CYP2D6 in yeast cells. *Pharmacogenetics* **5**: 103-109.

Krynetski, E. Y.; Drutsa, V. L.; Kovaleva, I. E.; Luzikov, V. N.; Uvarov, V. Y. (1993) Effects of amino-terminus truncation in human cytochrome P450IID6 on its insertion into the endoplasmatic reticulum membrane of *Saccharomyces cerevisiae. FEBSLett.* **336**: 87-89.

Kubota, S.; Yoshida, Y.; Kumaoka, H.; Furumichi, A. (1977) Studies on the microsomal electron-transport system of anaerobically grown yeast. *J*. *Biochem*. **81**: 197-205.

Laemmli, U. K. (1970) Cleavage of structural proteins during the assembly of the head of bacteriophage T4. *Nature* **227**: 680-685.

Lazarou, J.; Pomeranz, B. H.; Corey, P. N. (1998) Incidence of adverse drug reactions in hospitalized patients. *JAMA* **279**: 1200-1205.

Lewis, D. F. V. (1998) The CYP2 family: models, mutants and interactions. *Xenobiotica* **28**: 617-661.

Lim, C. K.; Yuan, Z.-X.; Lamb, J. H.; White, I. N. H.; De Matteis, F.; Smith, L. L. (1994) A comparative study of tamoxifen metabolism in female rat, mouse and human liver microsomes. *Carcinogenesis* **15**: 589-593.

Lodwick, R.; McConkey, B.; Brown, A. M.; Beeley, L. (1987) Life threatening interaction between tamoxifen and warfarin. *Br*. *Med*. *J*. **295**: 1141.

Logan, W. P. D. (1975) *World Health Organisation Statistics Report.* WHO, **28**: 232.

Lowry, O. H.; Rosebrough, A. L.; Farr, A. L.; Randell, R. J. (1951) Protein measurement with the folin phenol reagent. *J*. *Biol. Chem.* **214**: 741.

Lundqvist, E.; Johansson, I.; Ingelman-Sundberg, M. (1999) Genetic mechanisms for duplication and multiplication of the human CYP2D6 gene and methods for detection of duplicated CYP2D6 genes. *Gene* **226**: 327-338.

MacGregor, J. and Jordan, V. C. (1998) Basic guide to the mechanisms of antiestrogen action. *Pharmacol*. *Rev*. **50**: 151-196.

Mahgoub, A.; Idle, J. R.; Dring, L. G.; Lancaster, R.; Smith, R. L. (1977) Polymorphic hydroxylation of debrisoquine in man. *Lancet* **2**: 584-586.

Marez, D.; Legrand, M.; Sabbagh, N.; Lo Guidice, J. M.; Boone, P.; Broly, F. (1996) An additional allelic variant of the *CYP2D6* gene causing impaired metabolism of sparteine. *Human Genetics* **97**: 668-670.

Marez, D.; Sabbagh, N.; Legrand, M.; Lo Guidice, J. M.; Boone, P.; Broly, F. (1995) A novel *CYP2D6* allele with an abolished splice recognition site associated with the poor metabolizer phenotype. *Pharmacogenetics* **5**: 305-311.
Marquardt, H. und Schäfer, S. G. (Hrsg.) (1997). *Lehrbuch der Toxikologie.* 1. Ausgabe. Heidelberg, Berlin: Spektrum Akademischer Verlag.

Masimirembwa, C.; Hasler, J.; Bertilssons, L.; Johansson, I.; Ekberg, O.; Ingelman-Sundberg, M. (1996a) Phenotype and genotype analysis of debrisoquine hydroxylase (CYP2D6) in a black Zimbabwean polulation. Reduced enzyme activity and evaluation of metabolic correlation of CYP2D6 probe drugs. *Eur. J*. *Clin. Pharmacol.* **51**: 117-122.

Masimirembwa, C.; Persson, I.; Bertilsson, L.; Hasler, J.; Ingelman-Sundberg, M. (1996b) A novel mutant variant of the *CYP2D6* gene (*CYP2D6*17*) common in black African population: association with diminished debrisoquine hydroxylase activity. *Br*. *J*. *Clin*. *Pharmacol.* **42**: 713-719.

Matsunaga, E.; Umeno, M.; Gonzalez, F. J. (1990) The rat P450 IID subfamily: complete sequences of four closely linked genes and evidence that gene conversions maintained sequence homogeneity at the heme-binding region of the cytochrome P450 active site. *J*. *Mol. Evol.* **30**: 155-169.

Meyer, U. A. (1991) Genotype or phenotype: the definition of a pharmacogenetic polymorphism. *Pharmacogenetics* **1**: 66-67.

Meyer, U. A. and Zanger, U. M. (1997) Molecular mechanisms of genetic polymorphisms of drug metabolising enzymes. *Annu. Rev*. *Pharmacol*. *Toxicol*. **37**: 269-296.

Monier, S.; Van Luc, P.; Adesnik, M. (1988) Signals for the incorporation and orientation of cytochrome P450 in the endoplasmatic reticulum membrane. *J*. *Cell Biol*. **107**: 457-570.

Moorthy, B.; Sriram, P.; Pathak, D. N.; Bodell, W. J.; Randerath, K. (1996) Tamoxifen metabolic activation: comparison of DNA adducts formed by microsomal and chemical activation of tamoxifen and 4-hydroxy-tamoxifen with DNA adducts formed *in vivo. Cancer Res.* **56**: 53-57.

Mortimer, O.; Persson, K.; Ladona, M. G.; Spalding, D.; Zanger, U. M.; Meyer, U. A.; Rane, A. (1990) Polymorphic formation of morphine from codeine in poor and extensive metabolizers of dextromethorphan: relationship to the presence of immunoidentified cytochrome P-450IID1. *Clin*. *Pharmacol. Ther*. **47**: 27-35.

Mulligan, R. C. and Berg, P. (1981) Selection for animal cells that express the *Escherichia coli* gene coding for xanthine-guanine phosphoribosyltransferase. *Proc. Natl*. *Acad Sci*. *USA* **78**: 2972-2976.

Nayfield, S. G. (1995) Tamoxifen's role in chemoprevention of breast cancer: an update. *J*. *Cell. Biol*. **Suppl. 22**: 42-50.

Nebert, D. W. (1997) Polymorphisms in drug-metabolizing enzymes: what is their clinical significance and why do they exist? *Am. J*. *Hum. Genet.* **60**: 265-271.

Nebert, D. W. and McKinnon, R. A. (1994) Cytochrome P450: evolution and functional diversity. *Prog. Liv. Dis*. **12**: 63-97.

Nelson, D. R.; Koymans, L.; Kamataki, T.; Stegeman, J. J.; Feyereisen, R.; Waxman, D. J.; Gotoh, O.; Coon, M. J.; Estabrook, R. W.; Gunsalus, I. C.; Nebert, D. W. (1996) P450 superfamily: update on new sequences, gene mapping, accession numbers and nomenclature. *Pharmacogenetics* **6**: 1-42.

Nelson, D. R. and Stobel, H. W. (1988) On the membrane topolgy ofvertebrate cytochrome P-450 proteins. *J*. *Biol. Chem.* **263**: 6038-6050.

Oates, N. S.; Shah, R. R.; Idle, J. R.; Smith, R. L. (1982) Genetic polymorphism of phenformin 4-hydroxy-lation. *Clin. Pharmacol. Ther*. **32**: 81-89.

Ohgiya, S.; Komori, M.; Ohi, H.; Shiramatsu, K.; Shinriki, N.; Kamataki, T. (1992) Six-base deletion occurring in messages of human cytochrome P-450 in the CYP2C subfamily results in reduction of tolbutamide hydroxylase activity. *Biochemistry International* **27**: 1073-1081.

Omura, T. and Sato, R. (1964a) The carbon monoxide-binding pigment of liver microsomes. I. Evidence for its hemoprotein nature. *J*. *Biol*. *Chem.* **239**: 2370-2385.

Omura, T. and Sato, R. (1964b) The carbon monoxide-binding pigment of liver microsomes. II. Solubilization, purification, and properties. *J*. *Biol. Chem.* **239**: 2379-2385.

Onderwater, R. C. A.; Goeptar, A. R.; Levering, P. R.; Vos, R. M. E.; Konings, P. N. M.; Doehmer, J.; Commandeur, J. N. M.; Vermeulen, N. P. E. (1996) The use of macroporous microcarriers for the large-scale growth of V79 cells genetically designed to express single human cytochrome P450 isoenzymes and for the characterization of the expressed cytochrome P450. *Prot*. *Expr*. *Pur*. **8**: 439-446.

Osborne, C. K. (1998) Tamoxifen in the treatment of breast cancer. *Drug Therapy* **339**: 1609-1618.

Osborne, M. R.; Davis, W. D.; Hewer, A. J.; Hardcastle, I. R.; Phillips, D. H. (1999) 4-Hydroxytamoxifen gives DNA adducts by chemical activation, but not in rat liver cells. *Chem. Res. Toxicol.* **12**: 151-158.

Oscarson, M.; Hidestrand, M.; Johansson, I.; Ingelman-Sundberg, M. (1997) A combination of mutations in the *CYP2D6*17* (*CYP2D6Z*) allele causes alterations in enzyme function. *Mol*. *Pharmacol.* **52**: 1034-1040.

Paine, M. J. I.; Gilham, D.; Roberts, G. C. K.; Wolf, C. R. (1996) Functional high level expression of cytochrome P450 CYP2D6 using baculovirus expression systems. *Arch. Biochem. Biophys.* **328**: 143-150.

Panserat, S.; Mura, C.; Gerard, N.; Vincent-Viry, M.; Galteau, M. M.; Jacqz-Aigrain, E.; Krishnamorthy, R. (1994) DNA haplotype-dependent differences in the amino acid sequence of debrisoquine 4-hydroxylase (CYPZD6): evidence for two major allozymes in extensive metabolisers. *Hum. Genet.* **94**: 401-406.

Panserat, S.; Mura, C.; Gerard, N.; Vincent-Viry, M.; Galteau, M. M.; Jacqz-Aigrain, E.; Krishnamorthy, R. (1995) An unequal cross-over event within the *CYP2D6* gene cluster generates a chimeric *CYP2D7*/*CYP2D6* gene which is associated with the poor metabolizer phenotype. *Br*. *J*. *Clin*. *Pharmacol.* **40**: 361-367.

Parker, B. A. and Stark, F. R. (1979) Regulation of simian virus 40 transcription. *J*. *Virol*. **31**: 360.

Pathak, D. N.; Pongracz, K.; Bodell, W. J. (1995) Microsomal and peroxidase activation of 4-hydroxy-tamoxifen to form DNA adducts: comparison with DNA adducts formed in Sprague-Dawley rats treated with tamoxifen. *Carcinogenesis (Lond.)* **16**: 11-15.

Patten, C. J.; Smith, T. J.; Murphy, S. E.; Wang, M.-H.; Lee, J.; Tynes, R. E.; Koch, P.; Yang, C. S. (1996) Kinetic analysis of the activation of 4-(Methylnitroso)-1-(3-pyridyl)-1-butanone by heterologously expressed human P450 enzymes and the effect of P450-specific chemical inhibitors on this activation in human liver microsomes. *Arch. Biochem. Biophys.* **333**: 127-138.

Penman, B. W.; Reece, J.; Smith, T.; Yang, C. S.; Gelboin, H. V.; Gonzalez, F. J.; Crespi, C. L. (1993) Characterization of a human cell line expressing high levels of cDNA-derived CYP2D6. *Pharmacogenetics* **3**: 28-39.

Pierce, D. M.; Smith, S. E.; Franklin, R. A. (1987) The pharmacogenetics of indoramin in poor and excessive hydroxylators of debrisoquine. *Eur*. *J*. *Clin. Pharmacol.* **33**: 59-65.

Poon, G. K.; Chui, Y. C.; McCague, R.; Lonning, P. E.; Feng, R.; Rowlands, M. G.; Jarman, M. (1993) Analysis of phase I and phase II metabolites of tamoxifen in breast cancer patients. *Drug Metab*. *Dispos*. **21**: 1119-1124.

Poulos, T. L.; Finzel, B. C.; Howard, A. J. (1987) High-resolution crystal structure of cytochrome P450cam. *J*. *Mol*. *Biol*. **195**: 687-700.

Price-Evans, D. A. P. (1993). *Genetic factors in drug therapy*. *Clinical and molecular pharmacogenetics*. Cambridge: Cambridge University Press.

Pritchard, M. P.; Glancey, M. J.; Blake, J. A. R.; Gilham, D. E.; Burchell, B.; Wolf, C. R.; Friedberg, T. (1998) Functional co-expression of CYP2D6 and human NADPH-cytochrome P450 reductase in *Escherichia coli. Pharmacogenetics* **8**: 33-42.

Prueksaritanont, T.; Dwyer, L. M.; Cribb, A. E. (1995) (+)-Bufuralol 1'-hydroxylation activity in human and rhesus monkey intestine and liver. *Biochem. Pharmacol*. **50**: 1521-1525.

Randerath, K.; Moorthy, B.; Mabon, N.; Sriram, P. (1994) Tamoxifen: evidence by ³²Ppostlabeling and use of metabolic inhibitors for two distinct pathways leading to mouse hepatic DNA adduct formation and identification of 4-hydroxytamoxifen as a proximate metabolite. *Carcinogenesis (Lond.)* **15**: 2987-2094.

Rauschenbach, R.; Gieschen, H.; Salomon, B.; Kraus, C.; Kühne, G.; Hildebrand, M. (1997) Development of a V79 cell line expressing human cytochrome P450 2D6 and its application as a metabolic screening tool. *Environm. Toxicol*. *Pharmacol*. **3**: 31-39.

Rettie, A. E.; Korzekwa, K. R.; Kunze, K. L.; Laurence, R. F.; Eddy, A. C.; Ayoma, T.; Gelboin, H. V.; Gonzalez, F. J.; Trager, W. F. (1992) Hydroxylation of warfarin by human cDNA expressed cytochrome P450: a role for P4502C9 in the etiology of (S)-warfarin drug interactions. *Chem*. *Res. Toxicol*. **5**: 54-59.

Roh, H. K.; Dahl, M. L.; Johansson, I.; Ingelman-Sundberg, M.; Cha, Y. N.; Bertilsson, L. (1996) Debrisoquine and S-mephenytoin hydroxylation phenotypes and genotypes in a Korean population. *Pharmacogenetics* **6**: 441-447.

Romkes-Sparks, M.; Mnuskin, A.; Chern, H. D.; Persad, R.; Fleming, C.; Sibley, G. N.; Smith, P.; Wilkinson, G. R.; Branch, R. A. (1994) Correlation of polymorphic expression of CYP2D6 mRNA in bladder mucosa and tumor tissue to *in vivo* debrisoquine hydroxylase activity. *Cacinogenesis* **15**: 1955-1961.

Roy, S. D.; Hawes, E. M.; McKay, G.; Korchinski, E. D.; Midha, K. K. (1985) Metabolism of methoxyphenamine in extensive and poor metabolisers of debrisoquine. *Clin. Pharmacol. Ther*. **38**: 128-133.

Sabbagh, N.; Brice, A.; Marez, D.; Durr, A.; Legrand, M.; Lo Guidice, J.-M.; Korzekwa, K. R.; Destee, A.; Agid, Y.; Broly, F. (1997) *CYP2D6* polymorphism in familial and sporadic Parkinson's disease. *unveröffentlicht*.

Sabbagh, N.; Brice, A.; Marez, D.; Durr, A.; Legrand, M.; Lo Guidice, J.-M.; Korzekwa, K. R.; Destee, A.; Agid, Y.; Broly, F. (1999) CYP2D6 polymorphism and Parkinson's disease susceptibility. *Mov. Disord*. **14**: 230-236.

Sachse, C.; Brockmöller, J.; Bauer, S.; Reum, T.; Roots, I. (1996) A rare insertion of T226 in exon 1 of *CYP2D6* causes a ftameshift and is associated with the poor metabolizer phenotype: *CYP2D6*15*. *Pharmacogenetics* **6**: 269-272.

Sachse, C.; Brockmöller, J.; Bauer, S.; Roots, I. (1997) Cytochrome P450 2D6 variants in a Caucasian population: allele frequencies and phenotypic consequences. *Am*. *J. Hum. Genet.* **60**: 284-95.

Saiki, R. K.; Gelfand, D. H.; Stoffel, S.; Scharf, S. J.; Higuchi, R.; Horn, G. T.; Mullis, K. B.; Erlich, H. A. (1988) Primer-directed enzymatic amplification of DNA with a thermostable DNA polymerase. *Science* **239**: 487-491.

Sambrook, J.; Fritsch, E. F.; Maniatis, T. (1989). *Molecular Cloning*. 2nd Edition. Cold Spring Harbor, New York: Cold Spring Harbor Laboratory Press.

Sawada, M. and Kamataki, T. (1998) Genetically engineered cells stably expressing cytochrome P450 and their application to mutagen assays. *Mutation Research* **411**: 19-43.

Saxena, R.; Shaw, G. L.; Relling, M. V.; Frame, J. N.; Moir, D. T.; Evans, W. E.; Caporaso, N.; Weiffenbach, B. (1994) Identification of a new variant *CYP2D6* single base pair deletion in exon 3 and its association with the poor metabolizer phenotype. *Hum. Mol*. *Genet.* **3**: 923-926.

Schenkman, J. B. und Greim, H. (1993). Cytochrome P450, **105**. Berlin: Springer.

Schenkman, J. B.; Tamburini, P. P.; Jansson, I.; Epstein, P. M. (1989). Functional aspects of protein-protein interactions of cytochrome b₅, cytochrome P-450 reductase and cytochrome P-450. In: *Book of abstracts and lecture notes. NATO Advanced Study Institute on Molecular Aspects of Monooxygenases and Bioactivation of Toxic Compounds.*

Schmalix, W. A.; Barrenscheen, M.; Landsiedel, R.; Janzowski, C.; Eisenbrand, G.; Gonzalez, F.; Eliasson, E.; Ingelman-Sundberg, M.; Perchermeier, M.; Greim, H.; Doehmer, J. (1995) Stable expression of human cytochrome P450 2E1 in V79 Chinese hamster cells. *Eur*. *J. Pharmacol.* **293**: 123-131.
Schmalix, W. A.; Maeser, H.; Kiefer, F.; Reen, R.; Wiebel, F. J.; Gonzalez, F. J.; Seidel, A.; Glatt, H. R.; Doehmer, J. (1993) Stable expression of human cytochrome P450 1A1 cDNA in V79 Chinese hamster cells and metabolic activation of benzo[*a*]pyrene. *Eur. J*. *Pharmacol.* **248**: 251-261.

Schneider, A.; Schmalix, W. A.; Siruguri, V.; de Groene, E. M.; Horbach, G. J.; Kleingeist, B.; Lang, D.; Böcker, R.; Belloc, C.; Beaune, P.; Greim, H.; Doehmer, J. (1996) Stable expression of human cytochrome P450 3A4 in conjunction with human NADPH-cytochrome P450 oxidoreductase in V79 hamster cells. *Arch. Biochem. Biophys.* **332**: 295-304.

Schulz, M.; Freisem-Rabien, U.; Jessberger, R.; Doerfler, W. (1987) Transcriptional activities of mammalian genomes at active sites of recombination with foreign DNA. *J*. *Virol*. **61**: 344-353.

Schulz-Utermoehl, T.; Bennett, A.; Ellis, S.; Tucker, G. B. A.; Edwards, R. (1999) Polymorphic debrisoquine 4-hydroxylase activity in the rat is due to differences in CYP2D2 expression. *Pharmacogenetics* **in press.**

Sengstag, C.; Eugster, H. P.; Wurgler, F. E. (1994) High promutagen activating capacity of yeast microsomes containing human cytochrome P-450 1A and human NADPH-cytochrome P-450 reductase. *Carcinogenesis* **15**: 837-843.

Sharp, P. A.; Sugden, B.; Sambrook, J. (1973) Detection of two restriction endonuclease activities in *Haemophilus parainfluenzae* using analytical agarose-ethidium bromide electrophoresis. *Biochemistry* **12**: 3055-3063.

Shimada, T.; Yamazaki, H.; Mimura, M.; Inui, Y.; Guengerich, F. P. (1994) Interindividual variations in human liver cytochrome P-450 enzymes involved in the oxidation of drugs, carcinogens and toxic chemicals: studies with liver microsomes of 30 Japanese and 30 Caucasians. *J*. *Pharmacol. Exp*. *Ther*. **270**: 414-423.

Simi, S.; Xiao, Y.; Campagna, M.; Doehmer, J.; Darroudi, F. (1999) Dual-colour FISH analysis to characterize a marker chromosome in cytochrome P450 2B1 recombinant V79 Chinese hamster cells. *Mutagenesis* **14**: 101-105.

Smith, D. A.; Abel, S. M.; Hyland, R.; Jones, B. C. (1998) Human cytochrome P450s: selectivity and measurement *in vivo. Xenobiotica* **28**: 1095-1128.

Smith, L. L. and White, I. N. H. (1998) Antiestrogen therapy: uncertainties and risk assessment. *Oncology* **12 Suppl.** 5: 14-22.

Stearns, V. and Gelmann, E. P. (1998) Does Tamoxifen cause cancer in humans? *J*. *Clin*. *Oncol*. **16**: 779-792.

Steen, V. M.; Molven, A.; Aarskog, N. K.; Gulbrandsen, A.-K. (1995) Homologous unequal cross-over involving a 2.8 kb direct repeat as a mechanism for the generation of allelic variants of the human cytochrome P450 *CYP2D6* gene. *Hum. Mol. Genet.* **4**: 2251-2257.

Steiner, E.; Iselius, L.; Alvan, G.; Lindsten, J.; Sjoqvist, F. (1985) A family study of genetic and environmental factors determining polymorphic hydroxylation of debrisoquin. *Clin*. *Pharmacol. Ther*. **38**: 394-401.

Strain, A. J. and Wylie, A. H. (1984) Uptake and stability of SV40 DNA after calcium phosphate transfection of CV-1 cells. *Biochem. J*. **218**: 475-482.

Styles, J. A.; Davies, A.; Lim, C. K.; De Matteis, F.; Stanley, L. A.; White, I. N. H.; Yuan, Z.-X.; Smith, L. L. (1994) Genotoxicity of tamoxifen, tamoxifen epoxide and toremifene in human lymphoblastoid cells containing human cytochrome P450s. *Carcinogenesis* **15**: 5-9.

Swierenga, S. H.; Heddle, J. A.; Sigal, E. A.; Gilman, J. P.; Brillinger, R. L.; Douglas, G. R.; Nestmann, E. R. (1991) Recommended protocols based on a survey of current practice in genotoxicity testing laboratories, IV. Chromosome aberration and sister-chromatid exchange in Chinese hamster ovary, V79 Chinese hamster lung and human lymphocyte cultures. *Mutat*. *Res*. **246**: 301-322.

Szczesna-Skorupa, E.; Straub, P.; Kemper, B. (1993) Deletion of a conserved tetrapeptide, PPGP, in P450 2C2 results in loss of enzymatic activity without a change in its cellular location. *Arch. Biochem. Biophys.* **304**: 170-175.
Tenni, P.; Lalaich, D. L.; Byrne, M. J. (1989) Life threatening interaction between tamoxifen and warfarin. *Br. Med*. *J*. **298**: 93.

Tucker, G. T. (1998). Clinical aspects of polymorphic drug metabolism. In: *Drug metabolism: towards the next millenium,* Gooderman, N. (ed.). Amsterdam, Berlin, Oxford, Tokyo, Washington, DC: IOS Press, 109-118.
Tucker, G. T.; Lennard, M. S.; Ellis, S. W.; Woods, H. F.; Cho, A. K.; Lin, L. Y.; Hiratsuka, A.; Schmitz, D. A.; Chu, T. Y. (1994) The demethylenation of methylenedioxymethamphetamine ("ecstasy") by debrisoquine hydroxylase (CYP2D6). *Biochem. Pharmacol.* **47**: 1151-1156.

Tyndale, R.; Aoyama, T.; Broly, F.; Matsunaga, T.; Inaba, T.; Kalow, W.; Gelboin, H. V.; Meyer, U. A.; Gonzalez, F. J. (1991a) Identification of a new *CYP2D6* allele lacking the codon encoding Lys-281: possible association with the poor metabolizer phenotype. *Pharmacogenetics* **1**: 26-32.

Tyndale, R. F.; Kalow, W.; Inaba, T. (1991b) Oxidation of reduced haloperidol to haloperidol: involvement of human P450IID6 (sparteine/debrisoquine monooxygenase). *Br*. *J*. *Clin*. *Pharmacol.* **31**: 655-660.

Vorhees, C. V.; Reed, T. M.; Schilling, M. A.; Fisher, J. E.; Moran, M. S.; Cappon, G. D.; Nebert, D. W. (1998) *CYP2D1* polymorphism in methamphetamine-treated rats: genetic differences in neonatal mortality and effects on spatial learning and acoustic startle. *Neurotoxicology and Teratology* **20**: 263-273.

Wadelius, M.; Darj, E.; Frenne, G.; Rane, A. (1997) Induction of CYP2D6 in pregnancy. *Clin*. *Pharmacol*. *Ther.* **62**: 400-407.

Wahl, G. M.; de Saint Vincent, B. R.; DeRose, M. L. (1984) Effect of chromosomal position on amplification oftransfected genes in animal cells. *Nature* **307**: 516-520.

Wang, S. (1992). Phenotypes and genotypes of debrisoquine hydroxylation polymorphism in Chinese. *Master's thesis*. National Cheng Kung University, Tainan, Taiwan.

Wilking, N.; Isaksson, E.; von Schoultz, A. (1997) Tamoxifen and secondary tumors. *Drug Safety* **16**: 104-117.

Wolfel, C.; Platt, K. L.; Dogra, S.; Glatt, H.; Wachter, F.; Doehmer, J. (1991) Stable expression of rat cytochrome P450IA2 cDNA and hydroxylation of 17 beta-estradiol and 2-aminofluorene in V79 Chinese hamster cells. *Mol*. *Carcinog*. **4**: 489-498.

Yamamoto, Y.; Tasaki, T.; Nakamura, A.; Iwata, K.; Kazusaka, A.; Gonzalez, F. J.; Fujita, S. (1998) Molecular basis of the Dark Agouti rat drug oxidation polymorphism: importance of CYP2D1 and CYP2D2. *Pharmacogenetics* **8**: 73-82.

Yokota, H.; Tamura, S.; Furuya, H.; Kimura, S.; Watanabe, M.; Kanazawa, I.; Kondo, I.; Gonzalez, F. J. (1993) Evidence for a new variant allele *CYP2D6J* in a Japanese population associated with lower *in vivo* rates of sparteine metabolism. *Pharmacogenetics* **3**: 256-263.

Zanger, U. M.; Vilbois, F.; Hardwick, J. P.; Meyer, U. A. (1988) Absence of hepatic cytochrome P450bufI causes genetically deficient debrisoquine oxidation in man. *Biochemistry* **27**: 5447-5454.

## Patentansprüche

1. Zelluläres Testsystem zur Untersuchung der metabolischen Aktivität aktiver Formen des Cytochrom P4502D6, bestehend aus Zellen, die ein *Cytochrom P450 2D6 (hCYP2D6)*-Allel heterolog exprimieren, **dadurch gekennzeichnet, daß** die Allele *hCYP2D6*1, *2, *9, *10* und **17* exprimiert werden.

2. Testsystem gemäß einem der Ansprüche 1 bis 2, **dadurch gekennzeichnet, daß** die Zellen Lungenfibroblasten des Chinesischen Hamsters oder davon abgeleitete Zellen sind.

3. Testsystem gemäß Anspruch 2, **dadurch gekennzeichnet, daß** die Zellen V79-Zellen sind.

4. Testsystem gemäß Anspruch 3, **dadurch gekennzeichnet, daß** die Zellen die am 15.02.2000 bei der DSMZ-Deutsche Sammlung von Mikroorganismen und Zellkulturen GmbH unter den Zugangsnummern DSM ACC2446, DSM ACC2447, DSM ACC2448, DSM ACC2449 und DSM ACC2450 hinterlegten Zellinien V79MZh2D6*1, V79MZh2D6*2, V79MZh2D6*9, V79MZh2D6*10 und V79MZh2D6*17 sind.

5. Testsystem gemäß einem der Ansprüche 1 bis 4, **dadurch gekennzeichnet, daß** die Zellen cDNA exprimieren.

6. Kit, umfassend das Zelluläre Testsystem gemäß einem der Ansprüche 1 bis 5 und Wachstumsmedien sowie Kontrollsubstanzen.

7. Verwendung des Zellulären Testsystems gemäß einem der Ansprüche 1 bis 5 zur Untersuchung einer genetisch bedingten Toxizität von Metaboliten.

8. Verwendung gemäß Anspruch 7, wobei die Metaboliten Arzneistoffe sind.

9. Verwendung des Zellulären Testsystems gemäß einem der Ansprüche 1 bis 5 zur Bestimmung einer toxischen, mutagenen oder kanzerogenen Wirkung von Verbindungen.

10. Verwendung gemäß einem der Ansprüche 7 bis 9, wobei die humanes Cytochrom P450 2D6 exprimierenden Zellen mit der zu untersuchenden Substanz kontaktiert werden.

11. Verfähren zum Screening von Substanzen in Bezug auf Metabolisierung durch humanes Cytochrom P450 2D6, wobei die Zellen des Testsystems gemäß einem der Ansprüche 1 bis 5 mit einer Substanz kontaktiert werden und ein metabolisches Produkt gemessen wird.

12. Verfahren zum Nachweis neuer *P450 2D6* -Allele, umfassend eine heterologe Expression des in Frage stehenden Alleles in einer Zelle, Testen der das in Frage stehende Allel exprimierenden Zellen in Hinbück auf den Cytochrom P450 2D6-abhängigen Metabolismus einer oder mehrer Verbindungen und Vergleich des Metabolismus der Zellen mit dem Metabolismus von Zellen des Testsystem gemäß einem der Ansprüche 1 bis 5.

## Claims

1. Cellular test system for examination of the metabolic activity of active forms of cytochrome P450 2D6 consisting of cells expressing a *cytochrome P450 2D6 (hCYP2D6)* allele in a heterologous manner **characterized in that** the alleles *hCYP2D6*1, *2, *9, *10* and **17* are expressed.

2. Test system according to any one of claims 1 to 2 **characterized in that** said cells are Chinese hamster lung fibroblasts or cells derived therefrom.

3. Test system according to claim 2 **characterized in that** said cells are V79 cells.

4. Test system according to claim 3 **characterized in that** said cells are the cell lines V79MZh2D6*1, V79MZh2D6*2, V79MZh2D6*9, V79MZh2D6*10 and V79MZh2D6*17 deposited on February, 15, 2000, at the DSMZ-Deutsche Sammlung von Mikroorganismen und Zellkulturen GmbH under the accession numbers DSM ACC2446, DSM ACC2447, DSM ACC2448, DSM ACC2449 and DSM ACC2450.

5. Test system according to any one of the claims 1 to 4 **characterized in that** said cells express cDNA.

6. Kit comprising the cellular test system according to any of the claims 1 to 5 and growth media and control substances.

7. Use of the cellular test system according to any of the claims 1 to 5 for the study of the gene-dependent toxicity of metabolites.

8. Use according to claim 7 wherein said metabolites are drugs.

9. Use of the cellular test system according to any of the claims 1 to 5 for determining a toxic, mutagenic or cancerogenous effect of compounds.

10. Use according to any one of claims 7 to 9 wherein the cells expressing human cytochrome P450 2D6 are contacted with the substance to be tested.

11. Method for screening of substances with respect to their metabolization by human cytochrome P450 2D6 wherein the cells of the test system according to any one of the claims 1 to 5 are contacted with a substance and the metabolic product is measured.

12. Method for the detection of novel *P450 2D6* alleles wherein said method comprises the heterologous expression of the allele in question in a cell, testing the cells expressing the allele in question with respect to the cytochrome P450 2D6-dependent metabolism of one or more compounds and comparison of the metabolism of the cells to the metabolism of cells of the test system according to any one of the claims 1 to 5.

## Revendications

1. Système de test cellulaire pour la détermination de l'activité métabolique de formes actives du cytochrome P450 2D6 consistant en des cellules qui expriment de manière hétérologue un allèle de *Cytochrome P450 2D6 (hCYP2D6),* **caractérisé en ce que** les allèles *hCYP2D6*1, *2, *9, *10* et **17* sont exprimés.

2. Système de test selon l'une des revendications 1 à 2, **caractérisé en ce que** les cellules sont des fibroblastes pulmonaires du hamster chinois ou des cellules qui en dérivent.

3. Système de test selon la revendication 2, **caractérisé en ce que** les cellules sont des cellules V79.

4. Système de test selon la revendication 3, **caractérisé en ce que** les cellules sont les lignées cellulaires V79MZh2D6*1, V79MZh2D6*2, V79MZh2D6*9, V79MZh2D6*10 et V79MZh2D6*17 déposées le 15.02.2000 auprès de la DSMZ-Deutsche Sammlung von Mikroorganismen und Zellkulturen GmbH sous les numéros d'accès DSM ACC2446, DSM ACC2447, DSM ACC2448, DSM ACC2449 et DSM ACC2450.

5. Système de test selon l'une des revendications 1 à 4, **caractérisé en ce que** les cellules expriment l'ADNc.

6. Kit, comprenant le système de test cellulaire selon l'une des revendications 1 à 5 et des milieux de croissance, ainsi que des substances de régulation.

7. Utilisation du système de test cellulaire selon l'une des revendications 1 à 5 pour la détermination d'une toxicité conditionnée génétiquement de métabolites.

8. Utilisation selon la revendication 7, dans laquelle les métabolites sont des substances médicamenteuses.

9. Utilisation du système de test cellulaire selon l'une des revendications 1 à 5 pour la détermination d'une activité toxique, mutagène ou cancérigène de composés.

10. Utilisation selon l'une des revendications 7 à 9, dans laquelle les cellules exprimant le cytochrome humain *P450 2D6* sont mises en contact avec la substance à examiner.

11. Procédé pour le criblage de substances en relation avec la métabolisation par le cytochrome humain *P450 2D6*, dans lequel les cellules du système de test selon l'une des revendications 1 à 5 sont mises en contact avec une substance et qu'un produit métabolique est mesuré.

12. Procédé pour la détection de nouveaux allèles de *P450 2D6,* comportant une expression hétérologue de l'allèle en question dans une cellule, des tests des cellules exprimant l'allèle en question en ce qui concerne le métabolisme dépendant du *Cytochrome P450 2D6* d'un ou plusieurs composés et la comparaison du métabolisme des cellules avec le métabolisme de cellules du système de test selon l'une des revendications 1 à 5.
